# EUROPEAN PATENT APPLICATION

(11) **EP 1 498 482 A2**
(43) Date of publication of application: **19.01.2005**
(21) Application number: 04015106.0
(22) Date of filing: 28.06.2004
(51) Int. Cl.: C12N 9/90, C30B 7/00, G01N 23/20, G06F 17/50, G01F 19/00

(54) **Crystal structure of oxidosqualene cyclase**

(30) Priority: 07.07.2003 EP 03014558
(71) Applicant: F. HOFFMANN-LA ROCHE AG, 4070 Basel (CH)
(72) Inventor: Ruf, Armin, 79104 Freiburg (DE); Schulz-Gasch, Tanja, 4410 Liestal (CH); Thoma, Ralf, 79639 Grenzach-Wyhlen (DE)

(57) **Abstract**

The present invention relates to crystal forms of mammmalian OSC and the crystal structure information obtained from them, to methods of preparing such crystal forms, and to their use for the identification and/or design of inhibitors of OSC activity. A further subject matter of the invention are methods for the identification and/or design of inhibitor compounds of OSC activity, the inhibitor compounds of OSC activity identified by these methods and their use in pharmaceutical compositions for the treatment and/or prevention of diseases which are associated with OSC comprising hypercholesterolemia, hyperlipemia, arteriosclerosis, vascular diseases, mycoses, parasite infections and gallstones, and/or treatment and/or prophylaxis of impaired glucose tolerance, diabetes, tumors and/or hyperproliferative disorders, preferably for the treatment and/or prophylaxis of hypercholesterolemia and/or hyperlipemia.

## Description

The present invention relates to methods for recombinant expression of sufficient amounts of mammalian oxidosqualene synthase (OSC), to crystal forms of mammalian OSC and the three-dimensional X-ray crystal structure derived thereof, the modeling of new structures, the binding mode of a OSC ligand, as well as methods for rational drug design based on the use of such data.

2,3-Oxidosqualene:lanosterol cyclase (OSC, E.C.5.4.99.7) is a monotopic integral membrane protein associated with the endoplasmaic reticulum in eucaryotic cells. It catalyzes the key step in the conversion of the linear triterpene (3S)-2,3-oxidosqualene into fused ring compounds such as lanosterol in mammals and fungi or cycloartenol in plants. Lanosterol is the precursor of cholesterol and numerous steriodal hormones. High stereoselectivity and precision of the skeletal rearrangements has aroused the interest of researchers for nearly half a century, and valuable data on studying mechanistic details in the complex enzyme-catalyzed cyclization cascade has been collected (Viola, F., et al. (2000), *Rationally designed inhibitors as tools for comparing the mechanism of squalene-hopene cyclase with oxidosqualene cyclase,* Lipids *35*, 297-303). Today, interest in cyclases is still unbroken, since OSCs became targets for the development of antifungal and hypocholesterolemic drugs (Morand, O. H., et al. (1997), *Ro 48-8.071, a new 2,3-oxidosqualene:lanosterol cyclase inhibitor lowering plasma cholesterol in hamsters, squirrel monkeys, and minipigs: comparison to simvastatin,* J Lipid Res *38*, 373-90). OSC is downstream of farnesyl-pyrophosphate, beyond the synthesis of isoprenoids and coenzyme Q. In hamsters, pharmacologically active doses of an OSC inhibitor showed no adverse side-effects, in contrast to a statin which reduced food-intake and body weight, and increased plasma bilirubin, liver weight and liver triglyceride content (Morand et al., J. Lipid Res. *38,* 1997, 373-390). OSC inhibition does not trigger the overexpression of HMGR because of an indirect, negative feed-back regulatory mechanism involving the production of 24(S),25-epoxycholesterol (Peffley et al., Biochem. Pharmacol. 56, 1998, 439-449; Nelson et al., J. Biol. Chem. 256, 1981, 1067-1068; Spencer et al., J. Biol. Chem. *260,* 1985, 13391-13394; Panini et al., J. Lipid Res. *27*, 1986, 1190-1204; Ness et al., Arch. Biochem. Biophys. *308,* 1994, 420-425). This negative feed-back regulatory mechanism is fundamental to the concept of OSC inhibition because (i) it potentiates synergistically the primary inhibitory effect with an indirect down-regulation of HMGR, and (ii) it prevents the massive accumulation of the precursor monooxidosqualene in the liver. In addition, 24(S),25-epoxycholesterol was found to be one of the most potent agonists of the nuclear receptor LXR (Janowski et al., Proc. Natl. Acad. Sci. USA 96, 1999, 266-271). Considering that 24(S),25-epoxycholesterol is a by-product of inhibition of OSC it is hypothesized that the inhibition of OSC could also indirectly activate LXR-dependent pathways such as (i) cholesterol-7alpha-hydroxylase to increase the consumption of cholesterol via the bile acid route, (ii) expression of ABC proteins with the potential to stimulate reverse cholesterol transport and increase plasma HDL-C levels (Venkateswaran et al., J. Biol. Chem. *275,* 2000, 14700-14707; Costet et al., J. Biol. Chem. June 2000, in press; Ordovas, Nutr Rev *58,* 2000, 76-79; Schmitz and Kaminsky, Front Biosci 6, 2001, D505-D514), and/or inhibit intestinal cholesterol absorption (Mangelsdorf, XIIth International Symposium on Atherosclerosis, Stockholm, June 2000). In addition, possible cross talks between fatty acid and cholesterol metabolism mediated by liver LXR have been hypothesized (Tobin et al., Mol. Endocrinol. *14*, 2000, 741-752). Compounds inhibiting OSC therefore also inhibit the biosynthesis of cholesterol, ergosterol and other sterols, and reduce the plasma cholesterol levels. They can therefore be used in the therapy and prophylaxis of hypercholesterolemia, hyperlipemia, arteriosclerosis and vascular diseases in general. Furthermore, they can be used in the therapy and/or prevention of mycoses, parasite infections, gallstones, cholestatic liver disorders, tumors and hyperproliferative disorders, e.g. hyperproliferative skin and vascular disorders. In addition, these compounds can also be of therapeutical use to improve glucose tolerance in order to treat and/or prevent related diseases such as diabetes.

The structure of the bacterial squalene hopene cydase has been solved in complex with an inhibitor (Lenhart, A., Weihofen, W.A., Pleschke, A. E. and Schulz, G.E., Chemistry & Biology, 2002, *Vol*. *9*, 639-645). However, in contrast to mammalian OSC the bacterial squalene hopene cyclase (SHC) is dimeric (Wendt, K.U., Poralla, K., Schulz, G.E., (1997) *Structure and function of a squalene cyclase,* Science, *277,* 1811-1815) and is catalyzing a reaction with different educts and products as compared to OSC. Therefore, there are marked differences between mammalian OSC and SHC in the active site region and the substrate binding pocket that need to be considered in the design of OSC ligands.

Studies on membrane proteins like the triterpene synthases are often hampered by the generally low amount of protein in cells, difficulties in establishing overexpression system, their limited solubility and crystallizability. Also in the case of OSC only limited amounts of pure protein were available (Cattel, L. & Ceruti M. (1998) *Inhibitors of 2,3-oxidosqualene cyclase as tools for studying the mechanism and function of the enzyme.* Crit. Rev. Biochem. Mol. Biol. 33, 353-373). In most cases the protein was purified from natural sources as it was done for rat OSC (Kusano, M., Abe, I., Sankawa, U. & Ebizuka, Y. (1991) *Purification and some properties of squalene-2,3-epoxide: lanosterol cyclase from rat liver.* Chem Pharm Bull (Tokyo) *39,* 239-241). Heterologous expression of rat OSC in yeast yielded only small amounts of recombinant protein (Abe, I. & Prestwich, G.D. (1995). *Molecular cloning, characterization, and functional expression of rat oxidosqualene cyclase cDNA*. Proc. Natl. Acad. Sci. USA *92*, 9274-9278).

Therefore, the present invention provides a solution to the problem of identifying and/or designing inhibitors of OSC activity by providing methods for recombinant expression of sufficient amounts of OSC, crystal forms of OSC and their crystal structure information, methods of preparing such crystals, and methods of identifying and/or designing inhibitors of OSC with these crystals by structure based drug design.

The present invention relates to crystal forms of mammmalian OSC and the crystal structure information obtained from them, to methods for recombinant expression of sufficient amounts of OSC and to methods of preparing such crystal forms, and to their use for the identification and/or design of inhibitors of OSC activity. A further subject matter of the invention are methods for the identification and/or design of inhibitor compounds of OSC activity, the inhibitor compounds of OSC activity identified by these methods and their use in pharmaceutical compositions for the treatment and/or prevention of diseases which are associated with OSC comprising hypercholesterolemia, hyperlipemia, arteriosclerosis, vascular diseases, mycoses, parasite infections and gallstones, and/or treatment and/or prophylaxis of impaired glucose tolerance, diabetes, tumors and/or hyperproliferative disorders, preferably for the treatment and/or prophylaxis of hypercholesterolemia and/or hyperlipemia.

The present invention provides methods for expressing, purifying and crystallizing mammalian OSC to form a crystal which diffracts x-rays with sufficiently high resolution to allow determination of the three-dimensional structure of the OSC, or a portion or subdomain thereof. The three-dimensional structure is useful for rational design of ligands of OSC. The crystals may be used for determining the binding mode of potential ligands (e.g., inhibitors) by soaking the compounds into the crystals and then determining the structure of the complex.

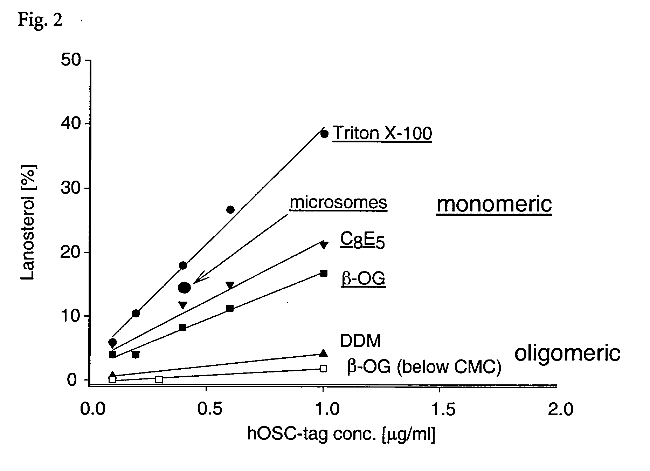

In one aspect the present invention provides a crystal form of mammalian OSC, preferably having the space group symmetry C222₁ (space group No. 20) and one monomer of OSC in the asymmetric unit. Preferably, the crystal includes a unit cell having dimensions a, b, and c; wherein a is from 186Å to 194Å, b is from 198 Å to 206 Å, and c is from 58 Å to 66 Å; and α = β = γ = 90°. Preferably, the crystal includes atoms arranged in a spatial relationship represented by the atomic structure coordinates as listed in Table 3 and any three-dimensional structure thereof having an overall fold as illustrated in Figure 2 and is of a protein containing at least about 80% of the amino acids in SEQ ID NO: 5. The atomic coordinates of OSC, or a fragment, analog, or variant thereof may be used to model a OSC protein, or a OSC -related protein. A model may also be developed based on a binding site identified herein. Preferably, the crystal includes OSC comprising the amino acid sequence of the native protein of SEQ ID NO: 5 as well as shorter variants thereof comprising all amino acids necessary for forming the active site. The crystal of the invention may also be a crystal of mammalian OSC with a C-terminal addition of amino acids, e.g., a C-terminal tag, as a crystal of mammalian OSC with a His-tag comprising the amino acid sequence of SEQ ID NO: 6.

The crystals of the invention include apo crystals and co-crystals. The apo crystals of the invention refer to crystals of mammalian OSC formed without a bound active site or allosteric ligand but could include bound detergents and/or lipids. The co-crystals generally comprise OSC with a ligand bound to the active site or to an allosteric site. The "active site" refers in general to the site where the enzymatic reaction catalyzed by the enzyme takes place. An active site ligand refers to any compound, which specifically binds to the active site of a mammalian OSC. Some data also give hints to allosteric sites of OSC by showing non-competitive inhibition (Abe, I., Zheng, Y.F. & Prestwich, G.D. (1998) *Photoaffinity labeling of oxidosqualene cyclase and squalene cyclase by a benzophenone-containing inhibitor.* Biochemistry *37*, 5779-5784).

Preferably, the co-crystal form of the present invention is characterized as having a space group of C222₁ (space group No. 20) and one monomer of OSC in the asymmetric unit.

More preferably, the co-crystal form has unit cell dimensions of a is from 186Å to 194Å, b is from 198 Å to 206 Å, and c is from 58 Å to 66 Å; and α = β = γ = 90° and a C222₁ symmetry.

The co-crystal forms of the invention generally comprise a crystalline OSC polypeptide in association with one or more compounds at an active or allosteric binding site of the polypeptide. The association may be covalent or non-covalent.

The OSC (2,3-Oxidosqualene:lanosterol cyclase (E.C.5.4.99.7)) of the present invention may be a mammalian OSC. Preferably, the OSC of the present invention is a human OSC (hOSC). Preferably, OSC comprises the amino acid sequence as set forth in SEQ. ID NO: 5 as well as shorter variants thereof comprising all amino acids necessary for forming the active site or longer variants comprising C-terminal amino acid additions as set forth in SEQ ID NO: 6.

It is to be understood that the crystal forms of OSC of the invention are not limited to crystal forms of naturally occurring or native OSC including recombinantly produced OSC. Indeed, the crystals of the invention include mutants of the native OSC. Mutants of native OSC are obtained by replacing at least one amino acid residue in the native OSC with a different amino acid residue, or by adding or deleting amino acid residues within the native polypeptide or at the N- or C- terminus of the native polypeptide, and have substantially the same three-dimensional structure as the native OSC from which the mutant is derived.

By having substantially the same three-dimensional structure is meant having a set of atomic structure coordinates from an apo- or co-crystal that have a root mean square deviation of less than or equal to about 1.5 Å when superimposed with the atomic structure coordinates of the native OSC when at least 50% of the alpha carbon atoms of OSC are included in the superposition.

In some instances, it may be particularly advantageous or convenient to substitute, delete and/or add amino acid residues to native OSC in order to provide convenient cloning sites in cDNA encoding the polypeptide, to aid in purification of the polypeptide, etc. Such substitutions, deletions and/or additions which do not substantially alter the three dimensional structure of the native OSC will be apparent to those having skills in the art.

It should be noted that the mutants contemplated herein need not exhibit OSC activity. Indeed, amino acid substitutions, additions or deletions that interfere with the enzymatic activity of the OSC but which do not significantly alter the three-dimensional structure of the domain are specifically contemplated by the invention. Such crystalline polypeptides, or the atomic structure coordinates obtained therefrom, can be used to identify compounds that bind to the native enzyme. These compounds may affect the activity of the native enzyme.

The native and mutated OSC polypeptides described herein may be isolated from natural sources or produced by methods well known to those skilled in the art of molecular biology. Expression vectors to be used may contain a native or mutated OSC polypeptide coding sequence and appropriate transcriptional and/or translational control signals. These methods include *in vitro* recombinant DNA techniques, synthetic techniques and *in vivo* recombination/genetic recombination. See, for example, the techniques described in Maniatis et al. (1989) *Molecular Cloning: A Laboratory Manual,* Cold Spring Harbor Laboratory, NY and Ausubel et al. (1989) *Current Protocols in Molecular Biology,* Greene Publishing Associates and Wiley Interscience, NY.

A variety of host-expression vector systems may be utilized to express the OSC coding sequence. These include but are not limited to microorganisms such as bacteria transformed with recombinant bacteriophage DNA, plasmid DNA or cosmid DNA expression vectors containing the OSC coding sequence; yeast transformed with recombinant yeast expression vectors containing the OSC coding sequence; insect cell systems infected with recombinant virus expression vectors (e.g. baculovirus) containing the OSC coding sequence; plant cell systems infected with recombinant virus expression vectors (e.g., cauliflower mosaic virus, CaMV; tobacco mosiac virus, TMV) or transformed with recombinant plasmid expression vectors (e.g., Ti plasmid) containing the OSC coding sequence; or animal cell systems. The expression elements of these systems vary in their strength and specificities. Depending on the host/vector system utilized, any of a number of suitable transcription and translation elements, including constitutive and inducible promoters such as pL of bacteriophage µ, plac, ptrp, ptac (ptrp-lac hybrid promoter) and the like may be used; when cloning in insect cell systems, promoters such as the baculovirus polyhedrin promoter may be used; when cloning in plant cell systems, promoters derived from the genome of plant cells (e.g., heat shock promoters; the promoter for the small subunit of RUBISCO; the promoter for the chlorophyll a/b binding protein) or from plant viruses (e.g., the 35 S RNA promoter of CaMV; the coat protein promoter of TMV) may be used; when cloning in mammalian cell systems, promoters derived from the genome of mammalian cells (e.g., metallothionein promoter) or from mammalian viruses (e.g., the adenovirus late promoter; the vaccinia virus 7.5K promoter) may be used; when generating cell lines that contain multiple copies of the OSC coding sequence, SV40-, BPV- and EBV-based vectors may be used with an appropriate selectable marker.

In a preferred embodiment of the present invention, an isolated nucleic acid sequence encoding hOSC comprising the nucleotide sequence of SEQ ID NO: 4 is provided.

Additionally, an expression vector containing an isolated nucleic acid sequence encoding hOSC comprising the nucleotide sequence of SEQ ID NO: 4 is provided. Preferably, the expression vector is an expression vector for the expression of proteins in *Pichia pastoris*. Furthermore, a host cell transformed with the said expression vector is provided. Preferably, the host cell is *Pichia pastoris*.

A further aspect of the present invention relates to a method of producing the hOSC comprising culturing the host cell with the said expression vector under conditions permitting the expression of hOSC by the host cell. Preferably, the host cell is P. pastoris. The present invention also provides hOSC produced by this method. Preferably, hOSC is produced in amounts sufficient for obtaining crystal forms of OSC in a quality appropriate for high-resolution structure determination.

The apo-, derivative and co-crystals of the invention can be obtained by techniques well-known in the art of protein crystallography, including batch, liquid bridge, free interface diffusion, dialysis, vapor diffusion and hanging drop methods (see e.g. McPherson (1982), *Preparation and Analysis of Protein Crystals,* John Wiley, NY; McPherson (1990), Eur. J. Biochem. *189*:1-23; Webber (1991) Adv. Protein Chem. *41*:1-*36; Crystallization of Nucleic Acids and Proteins,* Edited by Arnaud Ducruix and Richard Giege, Oxford University Press; *Protein Crystallization Techniques, Strategies, and Tips,* Edited by Terese Bergfors, International University Line, 1999). Generally, the apo- or co-crystals of the invention are grown by placing a soluble and substantially pure OSC polypeptide in an aqueous buffer containing a precipitant at a concentration just below that necessary to precipitate the protein. Water is then removed from the solution by controlled evaporation to produce crystallizing conditions, which are maintained until crystal growth ceases.

Preferably, the crystal forms of OSC are produced by a method for crystallizing mammalian OSC, the method comprising providing a buffered, aqueous solution of pH 7.5 to 9.5 with a concentration of 2 mg/ml to 40 mg/ml of monodisperse mammalian OSC. More preferably, mammalian OSC is provided in micelles of a detergent selected from the group comprising nonionic detergents comprising alkyl glucosides, e.g., n-octyl-β-D-glucopyranoside (β-OG), alkyl maltosides, e.g., DDM (dodecyl maltoside), alkyl oligooxyethylenes, e.g., C₈E₅, and polydisperse polyoxyethylenes, e.g. Triton, and zwitterionic detergents (amphotheric detergents) comprising sulfobetaines, e.g., zwittergent, and betaines, and cationic detergents comprising alkyldimethylamine oxides, e.g., lauryldimethylamine oxide (LDAO), and the CHAPS series, e.g., CHAPS. Preferably, the detergent is a non-ionic detergent or a cationic detergent. More preferably, the detergent is an alkyl glucoside, e.g., β-OG present in a concentration between 0.3% w/v to 3% w/v. Most preferably, the crystal forms of OSC are produced by a method for crystallizing mammalian OSC comprising (a) providing a buffered, aqueous solution of pH 7.5 to 9.5 with a concentration of 2 mg/ml to 40 mg/ml of mammalian OSC in β-OG micelles and (b) growing crystal forms using a buffered precipitant solution comprising between 2% and 25% of a small organic amphiphilic molecule, and between 10% and 35% of PEG (polyethylene glycol). Preferably, the small organic amphiphilic molecule is selected from the group comprising glycerol, ethylen glycol, butyl ether, benzamidine, dioxane, ethanol, isopropanol, butanol, pentanol, methyl pentanediol, pentanediol, hexanediol, heptanetriol and sucrose. Preferably, mammalian OSC is produced in the yeast *Pichia pastoris (P. pastoris).*

Preferably, co-crystals are produced by a method for co-crystallizing mammalian OSC and an active site ligand, the method comprising providing a buffered, aqueous solution of pH 7.5 to 9.5 with a concentration of 2 mg/ml to 40 mg/ml of monodisperse mammalian OSC and adding the active site ligand to the aqueous solution of mammalian OSC. More preferably, mammalian OSC is provided in micelles of a detergent selected from the group comprising nonionic detergents comprising alkyl glucosides, e.g., n-octyl-β-D-glucopyranoside (β-OG), alkyl maltosides, e.g., DDM (dodecyl maltoside), alkyl oligooxyethylenes, e.g., C₈E₅, and polydisperse polyoxyethylenes, e.g. Triton, and zwitterionic detergents (amphotheric detergents) comprising sulfobetaines, e.g., zwittergent, and betaines, and cationic detergents comprising alkyldimethylamine oxides, e.g., lauryldimethylamine oxide (LDAO), and the CHAPS series, e.g., CHAPS. Preferably, the detergent is a non-ionic detergent or a cationic detergent. More preferably, the detergent is an alkyl glucoside, e.g., β-OG present in a concentration of 0.3% w/v to 3% w/v. Most preferably, the crystal forms of OSC are produced by a method for crystallizing mammalian OSC comprising (a) providing a buffered, aqueous solution of pH 7.5 to 9.5 with a concentration of 2 mg/ml to 40 mg/ml of mammalian OSC in β-OG micelles, (b) adding the active site ligand to the aqueous solution of mammalian OSC, and (c) growing crystal forms using a buffered precipitant solution comprising between 2% and 25% of a small organic amphiphilic molecule, and between 10% and 35% of PEG (polyethylene glycol). Preferably, the small organic amphiphilic molecule is selected from the group comprising glycerol, ethylen glycol, butyl ether, benzamidine, dioxane, ethanol, isopropanol, butanol, pentanol, methyl pentanediol, pentanediol, hexanediol, heptanetriol and sucrose. Preferably, mammalian OSC is produced in the yeast *Pichia pastoris (P. pastoris).* Preferably, the active site ligand is added in a molar excess.

A further aspect, the present invention relates to a crystal or co-crystal form of OSC produced by the methods for crystallizing or co-crystallizing OSC of the present invention.

Furthermore, the present invention provides a crystal form of OSC or a co-crystal form of OSC with a bound ligand for the determination of the three-dimensional structure of mammalian OSC.

Another aspect of the present invention is the use of the crystal form and active site to identify potential OSC ligands. The atomic coordinate/x-ray diffraction data may be used to create a physical model which can then be used to design molecular models of compounds that may interact with the determined active sites, binding sites or other structural or functional domains or subdomains of OSC. Alternatively, the atomic coordinate/x-ray diffraction data of the complex may be represented as atomic model output data on computer readable media which can be used in a computer modeling system to calculate different molecules expected to interact with the determined active sites, binding sites, or other structural or functional domains or subdomains of the OSC. For example, computer analysis of the data allows one to calculate the three-dimensional interaction of the OSC and the ligand to confirm that the ligand binds to, or changes the conformation of, particular domain(s) or subdomain(s) of OSC. Ligands identified from the analysis of the physical or computer model can then be synthesized and tested for biological activity with an appropriate assay.

The atomic coordinate/x-ray diffraction data of the present invention are generally provided on computer readable media. A skilled artisan can then access the data and analyze it for structure determination and/or rational ligand (e.g., inhibitor) design using a computer-based system. A typical computer system includes hardware means, software means, and data storage means. The hardware means typically includes a central processing unit (CPU), input means, output means and data storage means. One skilled in the art will readily appreciate which of the currently available computer-based systems are suitable for use in the practice of the present invention.

A variety of commercially available software programs are available for conducting the analysis and comparison of data in the computer-based system. One skilled in the art will readily recognize which of the available algorithms or implementing software packages for conducting computer analyses can be utilized or adapted for use in the computer-based system. A target structural motif or target motif refers to any rationally selected sequence or combination of sequences in which the sequence(s) are chosen based on a three-dimensional configuration or electron density map which is formed upon the folding of the target motif. There are a variety of target motifs known in the art. Protein target motifs include, but are not limited to, enzymatic active sites, structural subdomains, epitopes, functional domains and signal sequences. A variety of structural formats for the input and output means can be used to input and output the information in the computer-based systems of the present invention.

A variety of comparing means can be used to compare a target sequence or target motif with the data storage means to identify structural motifs or interpret electron density maps derived in part from the atomic coordinate/x-ray diffraction data. One skilled in the art can readily recognize any one of the publicly available computer modeling programs that can be used.

Suitable software that can be used to view, analyze, design, and/or model a protein comprise MOLOC (Roche, 1985), FRED, MAIN, FlexX, Gold, XtalView, Alchemy TM , LabVision TM , Sybyl TM , Molcadd TM , Leapfrog TM , Matchmaker TM , Genefold TM and Sitel TM (available from Tripos Inc., St. Louis, MO.); Quanta TM , Cerius2 TM , X-Plor TM, CNS TM , Catalyst TM , Modeller TM , ChemX TM , Ludi TM , Insight TM , Discover TM , Cameleon TM and Iditis TM (available from Accelrys Inc., Princeton NJ); Rasmol TM (available from Glaxo Research and Development, Greenford, Middlesex, U.K.); MOE TM (available from Chemical Computing Group, Montreal, Quebec, Canada); Maestro TM (available from Shrdinger Inc.,); Midas/MidasPlus TM (available from UCSF, San Francisco, CA); VRML (webviewer- freeware on the internet); Chime (MDL - freeware on the intemet); MOIL (available from University of Illinois, Urbana-Champaign, IL); MacroModel TM and GRASP TM (available from Columbia University, New York, NY); Ribbon TM (available from University of Alabama, Tuscaloosa, Alabama); NAOMI TM (available from Oxford University, Oxford, UK); Explorer Eyechem TM (available from Silicon Graphics Inc., Mountain View, CA.); Univision TM (available from Cray Research Inc., Seattle WA); Molscript TM and O (available from Uppsala University, Uppsala, Sweden); Chem 3D TM and Protein Expert TM (available from Cambridge Scientific); and upgraded versions thereof.

Crystals may be frozen prior to data collection.

The mosaic spread of the frozen crystals could sometimes be reduced by annealing, wherein the stream of cold nitrogen gas is briefly blocked, allowing the frozen crystal to thaw momentarily before re-freezing in the nitrogen gas stream.

Diffraction data typically extending to 2.2 A was collected from the frozen crystals at the synchrotron beam line PX6A at the Swiss light source (SLS, Villigen, Switzerland). Under optimum conditions, data extending to 1.8 Å was recorded. Preferably, data is collected to a resolution of 3.5 Å or better. More preferably, data is collected to a resolution of 2.7 Å or better.

Derivative crystals of the invention can be obtained by soaking apo or co-crystals in mother liquor containing salts of heavy metal atoms, according to procedures known to those of skill in the art of X-ray crystallography.

Co-crystals of the invention can be obtained by soaking an apo crystal in mother liquor containing a ligand that binds to the active site, or can be obtained by co-crystallizing the OSC polypeptide in the presence of one or more ligands that bind to the active site or to an allosteric site. Preferably, co-crystals are formed with an active site OSC ligand, which is not converted by the enzyme. One example for such an active site ligand is [4-[6-(allyl-methyl-amino)-hexyloxy]-2-fluoro-phenyl]-(4-bromo-phenyl)-methanone fumarate.

In a further embodiment of the present invention a method for determining the three-dimensional structure of a crystal form of mammalian OSC to a resolution of 3.5 Å or better is provided, the method comprising
(a) crystallizing mammalian OSC; and
(b) analyzing the crystal form of mammalian OSC by X-ray diffraction to determine the three-dimensional structure of the crystal form of mammalian OSC, whereby the three-dimensional structure of a crystal form of mammalian OSC is determined to a resolution of 3.5 Å or better.

The present invention further relates to a machine-readable data storage medium comprising a data storage material encoded with machine readable data which, when using a machine programmed with instructions for using said data, displays a graphical three-dimensional representation of a molecule or molecular complex comprising at least a portion of mammalian OSC comprising the amino acids of SEQ ID NO:5 comprising the ligand binding active site being defined by a set of points having a root mean square deviation of less than about 1.5Å from points representing the backbone alpha carbon atoms of said amino acids as represented by structure coordinates as listed in Table 3.

The crystals of the invention, and particularly the atomic structure coordinates obtained therefrom, have a wide variety of uses. For example, the crystals and structure coordinates described herein are particularly useful for identifying compounds that interact with OSC as an approach towards developing new therapeutic agents. Pharmaceutical compositions of said compounds can be developed, and said compounds can be used for the manufacture of a medicament comprising said compound for the treatment and/or prevention of diseases which are associated with OSC comprising hypercholesterolemia, hyperlipemia, arteriosclerosis, vascular diseases, mycoses, parasite infections and gallstones, and/or treatment and/or prophylaxis of impaired glucose tolerance, diabetes, tumors and/or hyperproliferative disorders, preferably for the treatment and/or prophylaxis of hypercholesterolemia and/or hyperlipemia. Hyperproliferative skin and vascular disorders particularly come into consideration as hyperproliferative disorders.

Therefore, the present invention also relates to the use of a crystal form or a co-crystal form of the invention for the identification and/or design of inhibitors of OSC activity.

Moreover, the present invention relates to a method for identifying a compound that interacts with OSC, comprising the steps of
(a) generating a three-dimensional model of OSC using the structure coordinates as listed in Table 3, a root mean square deviation from the backbone alpha carbon atoms of said amino acids of less than 1.5Å; and
(b) employing said three-dimensional model to design or select a compound that interacts with OSC.
   In another aspect, the method further comprises the steps of
(c) obtaining the identified compound; and
(d) contacting the obtained compound with OSC in order to determine the effect the compound has on OSC activity.

The compound in these methods may be a compound that interacts with the active site of OSC or may be a compound that interacts with an allosteric site of OSC. Preferred are compounds, which interact with the active site of OSC. Even more preferred are compounds, which show an inhibitory effect on OSC activity in step (d) of the methods of the present invention.

In a further aspect of the present invention the method for identifying a compound that interacts with OSC is a computer-assisted method. Preferably, determining whether the compound is expected to bind to or interfere with the molecule or molecular complex includes performing a fitting operation between the compound and a binding site or substrate binding surface of the molecule or molecular complex, followed by computationally analyzing the results of the fitting operation to quantify the association between, or the interference with, the compound and the binding site. Optionally, the method further includes screening a library of compounds. Optionally, the method further includes supplying or synthesizing the compound, then assaying the compound to determine whether it interacts with and has an effect on mammalian OSC activity.

The present invention also relates to the compounds identified by the said methods for identifying a compound that interacts with OSC.

The structure coordinates described herein can be used as phasing models in determining the crystal structures of additional native or mutated OSC, as well as the structures of co-crystals of such OSC with active site inhibitors or activators bound. The structure coordinates, as well as models of the three-dimensional structures obtained therefrom, can also be used to aid the elucidation of solution-based structures of native or mutated OSCs, such as those obtained via NMR. Thus, the crystals and atomic structure coordinates of the invention provide a convenient means for elucidating the structures and functions of OSC or other cyclases.

For purposes of clarity and discussion, the crystal forms of the invention will be described by reference to specific OSC exemplary apo crystals and co-crystals. Those skilled in the art will appreciate that the principles described herein are generally applicable to crystal forms of any mammalian OSC, including, but not limited to hOSC.

Therefore, in a further aspect of the present invention a pharmaceutical composition comprising the compound identified by the methods of the present invention as having an effect on OSC activity, or pharmaceutically acceptable salts thereof, and a pharmaceutically acceptable carrier is provided.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, benzenesulfonic, toluenesulfonic, methanesulfonic, ethane disulfonic, oxalic, isethionic, and the like.

The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418, the disclosure of which is hereby incorporated by reference.

"Stable compound" and "stable structure" are meant to indicate a compound that is sufficiently robust to survive isolation to a useful degree of purity from a reaction mixture, and formulation into an efficacious therapeutic agent.

Furthermore, a compound identified by the methods of the present invention as having an effect on OSC activity for use as a therapeutic active substance, in particular for the treatment and /or prevention of diseases which are associated with OSC comprising hypercholesterolemia, hyperlipemia, arteriosclerosis, vascular diseases, mycoses, parasite infections and gallstones, and/or treatment and/or prophylaxis of impaired glucose tolerance, diabetes, tumors and/or hyperproliferative disorders, preferably for the treatment and/or prophylaxis of hypercholesterolemia and/or hyperlipemia, is provided. Hyperproliferative skin and vascular disorders particularly come into consideration as hyperproliferative disorders.

A further aspect of the present invention relates to the use of a compound identified by the methods of the present invention as having an effect on OSC activity for the manufacture of a medicament for the treatment and/or prevention of diseases which are associated with OSC comprising hypercholesterolemia, hyperlipemia, arteriosclerosis, vascular diseases, mycoses, parasite infections and gallstones, and/or treatment and/or prophylaxis of impaired glucose tolerance, diabetes, tumors and/or hyperproliferative disorders, preferably for the treatment and/or prophylaxis of hypercholesterolemia and/or hyperlipemia. Hyperproliferative skin and vascular disorders particularly come into consideration as hyperproliferative disorders.

Having now generally described this invention, the same will become better understood by reference to the specific examples, which are included herein for purpose of illustration only and are not intended to be limiting unless otherwise specified, in connection with the following figures.

### Figures

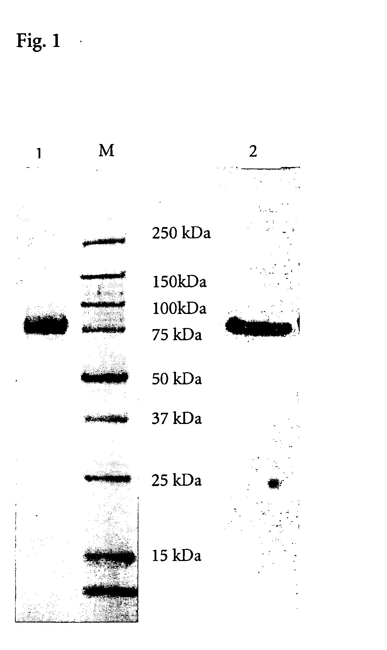

Figure 1: SDS-PAGE of hOSC after purification. Lane 1: purified hOSC-tag after gel filtration step; Lane M: standard proteins with molecular weights given in kDa; Lane 2: corresponding Western blot of the same protein sample with antibody directed against the myc-epitope.

Figure 2: Influence of the detergent on the activity. The amount of MOS converted to Lanosterol in one hour was determined by a radioactive assay as described. The percentage of lanosterol was plotted against the protein concentration of hOSC-tag (0.1-1 µg/ml) used in the assay. The influence of different detergents was tested by supplementing the assay buffer (50mM sodium phosphate buffer (pH 7.4), 1mM EDTA, 1mM dithiothreitol) with the appropriate detergent (0.2% TritonX-100 (λ), 0.6% C8E5 (τ), 0.8% β-OG (ν), 0.2% β-OG (o) and 0.02% DDM (σ). Detergents at the certain concentration giving monodispers protein in the analytical ultracentrifugation experiments (Table 1) were underlined.

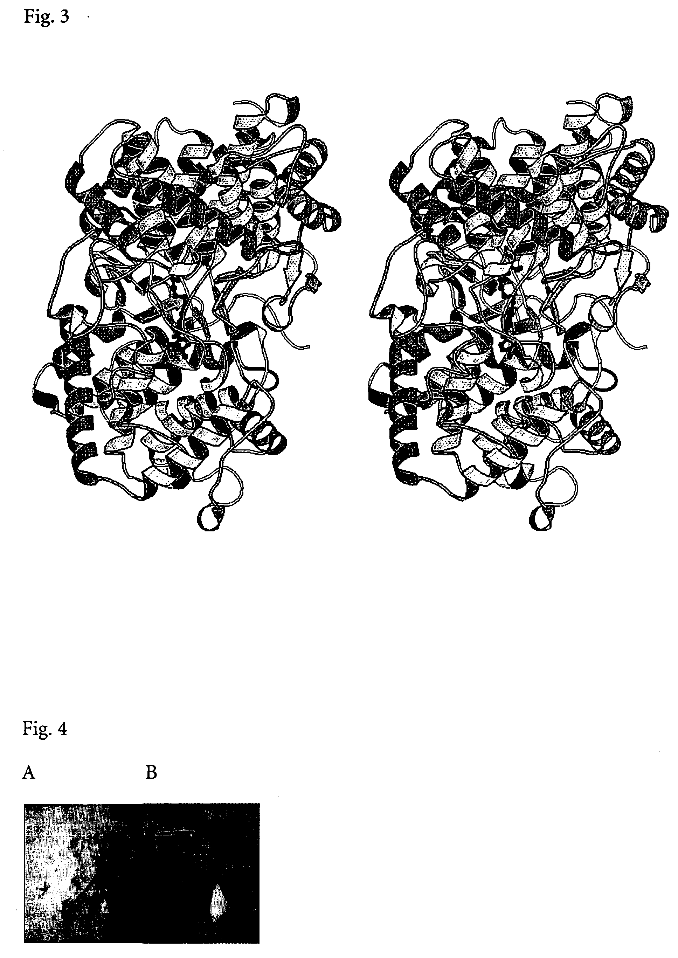

Figure 3: Stereo ribbon diagram of hOSC. The 2 (α/α) barrel domains (grey = outer barrel helices, light grey = inner barrel helices) and the C- and N-termini are labeled. Helix 8 (white) carries part of the non-polar plateau that inserts into the membrane. The bound inhibitor (black) indicates the location of the central active site cavity.

Figure 4: A) Crystals found in the crystallization screen (condition 22, Crystal Screen 1, Hampton Research). Phase separation and small detergent crystals are visible. B) Optimized crystals grown in hanging drops with 25% PEG 3350, 0.4M sodium acetate, 0.1M Tris, pH8.5, 10% (w/v) glycerol in the reservoir. No phase separation occurred, although a protein solution saturated with β-OG was used for crystallization.

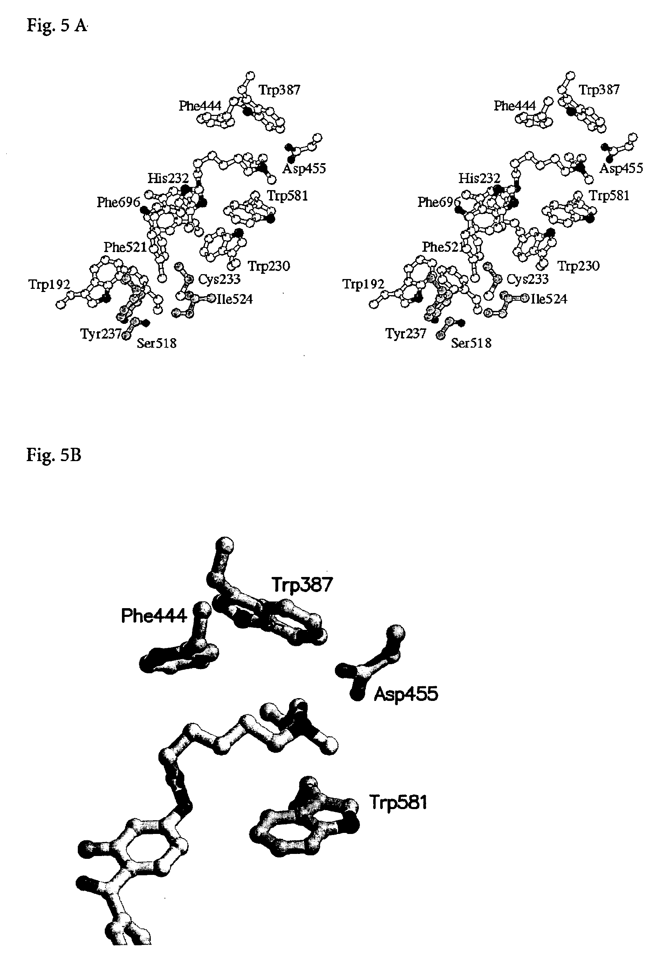

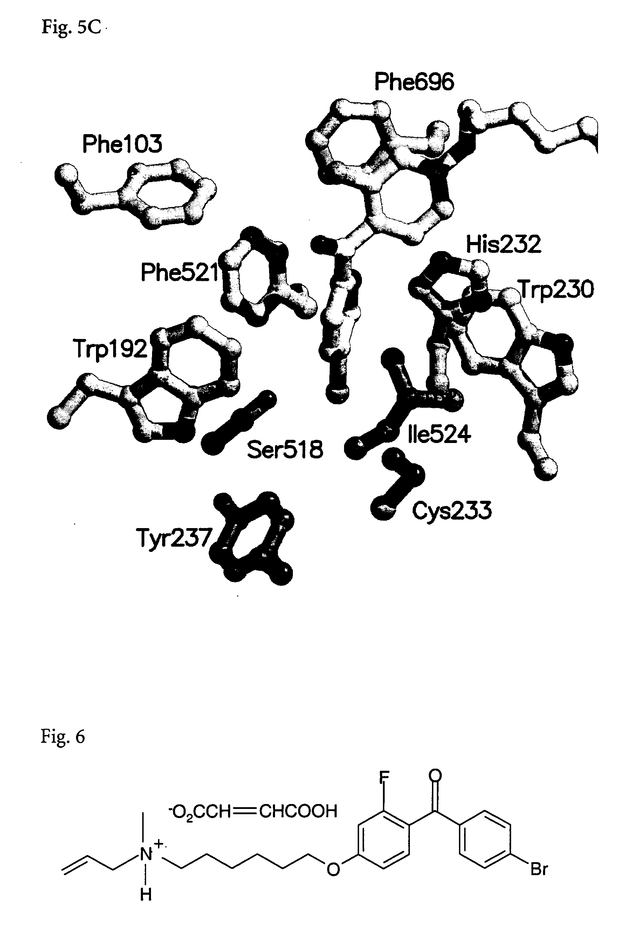

Figure 5: A) Stereo view of OSC bound inhibitor [4-[6-(allyl-methyl-amino)-hexyloxy]-2-fluoro-phenyl]-(4-bromo-phenyl)-methanone fumarate together with selected interacting side chains. The grey side chains form the constriction site that separates the active site pocket from the non-polar substrate uptake channel. B) OSC residues that interact with the positively charged amino group of the inhibitor [4-[6-(allyl-methyl-amino)-hexyloxy]-2-fluoro-phenyl]-(4-bromo-phenyl)-methanone fumarate. Similar interactions will stabilize the positively charged early high-energy intermediates in the cyclization reaction. The tryptophanes Trp387 and Trp581 can stabilize a positive charge at the position of the amino N-atom by cation-π interactions. The inhibitor amino group is hydrogen bonded to the catalytic asartic acid Asp455. C) Interactions between the inhibitor benzophenone group and the protein. The terminal bromo phenyl group has edge-to-face on interactions with Trp192 (3.6Å) and Trp230 (3.7Å) and a face-to-face interaction with Phe521 (4.6Å). The fluoro phenylgroup is stacked between the Phe696 (3.7Å) and the His232 imidazole ring (3.1Å). A conformational change of the Tyr237 side chain in the construction site (grey) can connect the active site cavity with the substrate entrance channel.

Figure 6: Structural formula of the inhibitor [4-[6-(allyl-methyl-amino)-hexyloxy]-2-fluoro-phenyl]-(4-bromo-phenyl)-methanone fumarate

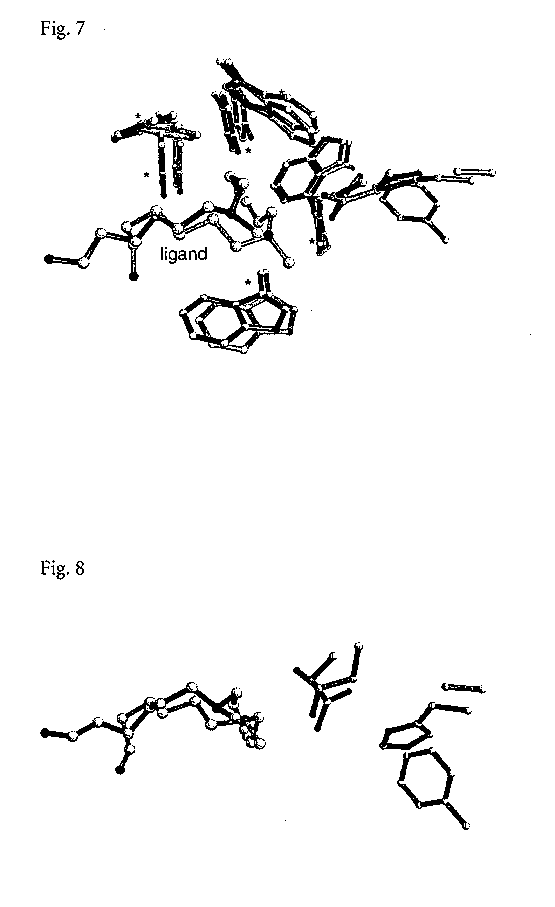

Figure 7: Superposition of the catalytic residue region: OSC - grey, SHC - black. Aromatic residues at the top of the hydrophobic tunnel (indicated by an asterisk in the Figure) are highly conserved between members of the cyclase family to which OSC and SHC are belonging. The side chains of these residues are supposed to be important for stabilization of cations via π-cation-interactions in the cyclization cascade of squalene (SHC) and 2,3-oxidosqualene (OSC). The high conservation of these residues in the top of the hydrophobic tunnel is in good accordance to the analogy of postulated cyclization mechanisms for A-ring formation. Differences occur however in the cyclase-catalyzed initiation of the process: SHC needs to initiate the cyclization cascade by protonation of a C=C double bond of squalene (olefin protonation), whereas OSC catalyzed cyclization is initiated by epoxide protonation of 2,3-oxidosqualene.

Figure 8: Differences in side chain orientation of the catalytic ASP (OSC - grey, SHC - black)

### Examples

Commercially available reagents referred to in the examples were used according to manufacturer's instructions unless otherwise indicated.

Abbreviations used comprise β-OG (n-Octyl-β-D-glucopyranoside); DDM (Dodecyl maltopyranoside); C₈E₅ (n-Octyl-pentaoxyethylene); LDAO (Lauryldimethylamine oxide).

### Example 1: Heterologous expression and purification of hOSC

### DNA manipulation and sequence analysis

Preparation of DNA probes, digestion with restriction endonucleases, DNA ligation and transformation of *E*.*coli* strains were performed as described by Sambrook (Sambrook, J., et al. (1989), *Molecular Cloning: A Laboratory Manual* (Cold Spring Harbor, NY, Cold Spring Harbor Laboratory Press, 1989). For DNA sequencing, the ABI PRISM BigDye Terminator Cycle Sequencing Ready Reaction Kit and ABI PRISM 310 Genetic analyzer was used. PCR were performed in the T3 Thermocycler (Whatman Biometra), using the *Pfu* polymerase (Stratagene).

Human OSC (hOSC) was amplified by PCR using a cDNA as a template. The oligonucleotides used for PCR were 5'-GTACCTTTCGAAACAATGACGGAGGGCACGTGTCTGCGG-3' (SEQ ID NO: 1) with a *Sfu*I site (in bold) and 5'-GCCTCTAGATCAGGGGTGGCCAGCAAGGG-3' (SEQ ID NO: 2) for hOSC with His-tag or 5'-GGCTCTAGATCAGGGGTGGCCAGCAAGGG-3' (SEQ ID NO: 3) for wildtype hOSC (SEQ ID NO: 4) both with a *Xba*I site (in bold). Using the two new restriction sites, both amplified DNA fragment were cloned into pPICZB vector (Invitrogen) leading to a fusion with the myc-epitope and the 6xHis-tag at the C-terminus for the construct with tag. The sequence was confirmed by sequencing. pPICZB-hOSC was linearized with *Pme*I, transformed by electroporation in *P. pastoris* strain GS115 and the phenotype of the colonies obtained was checked as recommended by the distributor Invitrogen.

The monotopic membrane protein hOSC was cloned into the pPICZB vector resulting in a construct with and without a tag. For the variant with tag due to the cloning the last amino acid, a proline, was changed to a leucine followed by the myc-epitope and the 6xHis-tag (EQKLISEEDLNSAVDHHHHHH). Sequencing of the whole gene shows one mutation L642V in comparison to the reported sequence (Sung, C.K., et al. (1995), *Molecular cloning of cDNA encoding human lanosterol synthase,* Biol Pharm Bull *18*, 1459-61 and Young, M., et al., (1996), *The human lanosterol synthase gene maps to chromosome 21q22.3*, Hum Genet *97*, 620-4). This conservative mutation is described in the literature as single nucleotide polymorphism (SNPs) and seems to be very common (Roessler, J., et al. (1989). *Molecular Cloning: A Laboratory Manual* (Cold Spring Harbor, NY, Cold Spring Harbor Laboratory Press 1999).

### Expression of recombinant human OSC in P. pastoris

Transformation into GS115 with *Pme*I linearized hOSCtag-pPICZB and hOSC-pPICZB results for both constructs in numerous colonies on the selection plate with 100 µg/ml Zeocin. For each construct eight clones with phenotype MutS plus a control GS115 with pPICZB were tested for hOSC expression. For this small scale expression tests colonies were grown at 30°C in YPD medium (1% yeast extract, 2% peptone, 2% glucose) with zeocin (100 µg/ml) to an OD₆₀₀ of 10-12. Cells were collected by centrifugation and resuspended in YP medium supplemented with 2% methanol. The same amount of methanol was added every 24 h. After 48 h cells were harvested, disrupted with glass bead lysis. The supernatant was analyzed by Western blot with a monoclonal antibody against the myc-epitope (Invitrogen) or a polyclonal antibody raised by immunization of rabbits against a synthetic peptide (C₄₇₁-P-H-V-T-E-H-I-P-R₄₈₀; SEQ ID NO: 7) was used. Supernatant of the glass beads lysis of *P*. *pastoris* cells were separated by SDS-PAGE and immobilized on a PDVF-membrane (Invitrogen) by transfer electrophoresis at 25V for 120min using as transfer buffer12 mM Tris base, 96 mM Glycine and 10% methanol. For blocking and the detection the Lumi-Light^{plus}-Kit from Roche Diagnostics was used following the manufacturer's recommendations.

For both constructs only one clone could be identified with a significant signal in the Western blot with the polyclonal hOSC antibody.

For the large scale expression a one liter culture (2x 2L shake flasks with 500 ml medium) of the transformed cell line with the strongest signal in the Western blot has been carried out with the same protocol as described above. The production level was modest with approximately 1-2 mg and 0.5 mg per one gram cell mass for the variant with and without tag, respectively.

### Purification of hOSC with 6xHis-tag

Cells expressing hOSC 6xHis-tag (SEQ ID NO: 6) harvested from one liter culture (40g) were resuspended in 50 mM Tris-HCl pH 7.5, 5% glycerol, 2 mM TCEP and 2mM Mg₂Cl₂ (buffer A) supplemented with 1mM DNAse (Roche Diagnostics) and two tablets Complete protease inhibitor (Roche Diagnostics) per 50 ml. The cell suspension was disrupted with 1.9 kbar in the Basic Z Model Cell homogenizer (Constant Systems LTD), sonicated for 3 minutes (50%, level 5 and 0°C) and was passed a second round through the cell homogenizer. Triton X-100 (Fluka) was added to the crude extract with a final concentration of 0.2% and incubated for one hour at 4°C. After centrifugation with 31000xg (JA20, Beckman) for half an hour the supernatant was loaded on a Ni-NTA-agarose column (10 x 2.5cm) equilibrated with buffer A plus 0.2% Triton X-100. The column was washed with 1 column volumes (CV) of the same buffer and 5 CV of buffer A plus 0.2% N-Octyl-beta-D-Glucopyranoside (β-OG, Applichem). The His-tag bound protein was then eluted with a linear gradient to 0.5M imidazole in buffer A. Collected fractions were screened on SDS-PAGE. Fractions containing hOSC were pooled and concentrated with a millipore filter device. The concentrated protein solution was loaded on a Fractogel EMD Biosec size exclusion column (1.6 cm x 60 cm, Merck), that was equilibrated with buffer A plus 0.8% β-OG. Fractions were analyzed by SDS-PAGE, showing a purity around 95%. For storage the pooled fractions were shock-frozen in liquid nitrogen and stored at -80°C.

For the solubilization two different detergents, β-OG (0.8%) and Triton X-100 (0.2%), were tried. Effectiveness was estimated by the yield obtained after Ni-NTA-chelate column. Triton X-100 gave the best results in yield. Also the incubation time was very critical, incubation longer than 1 hour resulted in one major contamination binding to the affinity column. After centrifugation at 31400xg (JA20, Beckman) the supernatant was applied to Ni-NTA-chelate column. Different combinations of β-OG and Triton X-100 both together, alone, above and below the CMC for β-OG were tried for the wash and elution steps. The best results were obtained with 0.2% β-OG in the wash and elution buffer, which is below the CMC (0.6%). The purification with the Ni-chelating column gave two peaks with hOSC one directly after the detergent exchange from Triton X-100 to β-OG and a second one in the normal Imidazol gradient. Two separate pools were made. The largest amount of protein (100 mg) was found in the detergent exchange pool, whereas only a quarter eluted in the imidazol gradient. N-terminal sequencing showed that in both pools the protein lost its N-terminal methionine. Additionally, mass spectroscopy analysis showed clearly a difference in mass, which corresponds to one histidine. Thus, the OSC eluted directly in the detergent exchange has only five histidines at the C-terminus and may bind less tightly as the one with six histidines. The solubilized hOSC was stable enough for one or two days with a detergent concentration under the CMC (0.2% for β-OG). The two pools were worked off separately. After size exclusion chromatography for both pools the protein had a purity to 95% as judged by SDS-PAGE (Fig. 1). The protein was stable in the gelfiltration buffer supplemented with 10% Glycerol and could be stored at -80°C without any significant loss of activity for at least half a year. The total amount of protein obtained for both pools together was 105 mg, which corresponds to 2 mg hOSC per gram biomass.

### Purification of hOSC wild-type

Cells expressing hOSC wildtype (SEQ ID NO: 5) harvested from one liter culture (45g) were resuspended in buffer A and disrupted as described for the variant with 6xHis-tag. After centrifugation at 31000xg (JA20, Beckman) for 30 min, the supernatant was further centrifuged at 40000xrpm for 60 min in the ultracentrifuge (Centrikon T-2070, Kontron) to isolate the membrane fraction. The pellet was resuspended in buffer A supplemented with 0.2% Triton X-100 and centrifuged again at 40000xrpm for 60 min. The supernatant was loaded on TMAE-Fractogel (1.6 cm x 13 cm, Merck) equilibrated with the resuspension buffer. The column was washed with six column volumes (CV) of buffer A supplemented with 0.2% β-OG and eluted with a linear gradient to 0.5 M sodium chloride in the same buffer. Collected fractions were screened on SDS-PAGE. Fractions containing hOSC were pooled. As a last step a gel filtration run was performed as described for the variant with His-tag.

In the case of hOSC without tag the membrane fraction was isolated containing hOSC as a major component as a first enrichment step. For the selective solubilization process three different detergents were tried β-OG (0.8%), Triton X-100 (0.2%) and Octyl-POE (0.6 %). Amount and purity were estimated by SDS-PAGE. The best results in yield and purity were obtained again with Triton X-100. The solubilized protein was bound classically to an anion exchanger, the detergent exchanged to β-OG by washing the column with 5 column volumes and eluted in a single peak at approximately 100 mM sodium chloride. The protein lacked again the N-terminal methionine and had a purity to 90% as judged by SDS-PAGE. In respect to stability and storage conditions it behaved identical to the variant with tag.

### Example 2: Characterization of expressed hOSC

### Analytical methods

Purification of hOSC was followed by electrophoresis on 10-20% Tricine SDS polyacrylamide gradient gels. Protein concentrations were determined according to Bradford (Bradford, M. M. (1976). A *rapid and sensitive method for the quantitation of microgram quantities of protein utilizing the principle of protein-dye binding,* Anal Biochem 72, 248-2541976) or for pure protein by absorption spectroscopy (Pace, C.N., Vajdos, F., Fee, L., Grimsley, G. & Gray, T. (1995). How to measure and predict the molar absorption coefficient of a protein. Protein Sci. 4, 2411-2423.) using the calculated molecular extinction coefficient at 280 nm of 181240 M⁻¹cm⁻¹.

### Activity of OSC

Activity assay and IC₅₀ determination was carried out as described for liver microsomal preparations (Lenhart, A., et al. (2003), *Binding structures and potencies of oxidosqualene cyclase inhibitors with the homologous squalene-hopene cyclase,* J Med Chem 46, 2083-92, 2003). Only the reaction buffer was modified by adding a detergent. The final conditions for IC₅₀ measurements were 30 µM of [¹⁴C] R,S-MOS in reaction buffer (50mM sodium phosphate buffer (pH 7.4), 1mM EDTA, 1mM dithiothreitol and 0.8% β-OG), containing 0.5% albumin, and ethanol <2%, in a total volume of 80 µl. The protein concentration of recombinant hOSC was 0.4 µg/ml. For testing the influence of different detergents on the activity the protein concentration was varied between 0.1 and 10 µg/ml in the presence of 0.2% β-OG, 0.6% C₈E₅, 0.1% Triton X-100 or 0.016% DDM. As positive control human liver microsomes were used at a final concentration of 0.4 mg/ml without detergent (Lenhart, A., et al. (2003), *Binding structures and potencies of oxidosqualene cyclase inhibitors with the homologous squalene-hopene cyclase*, J Med Chem 46, 2083-92 2003).

The activity of the recombinant enzyme was tested with different detergents in assay buffer (DDM, C₈E₅, β-OG or Triton X-100; all above the CMC). The highest activity was observed with Triton X-100, followed by C₈E₅ and β-OG (Figure 2). Although the concentration of DDM was two times the CMC a similar low activity was observed as with β-OG below the CMC. To compare the OSC from human liver microsomes and the recombinant enzyme more precisely the IC₅₀-value for RO-488071 a well known inhibitor of OSC (Morand, O.H., et al., (1997). *Ro 48-8.071, a new 2,3-oxidosqualene:lanosterol cyclase inhibitor lowering plasma cholesterol in hamsters, squirrel monkeys, and minipigs: comparison to simvastatin,* J Lipid Res *38,* 373-90) was determined in the presence of 0.8% β-OG. The recombinant human OSC shows an IC₅₀-value of 8.0 nM in comparison to an IC₅₀-value of 6.5 nM for OSC from liver microsomes.

### Oligomerisation state of OSC

Sedimentation equilibrium (SE) runs were performed at various speeds at 20°C with sample volume of 100 µl using an Optima XL-I analytical ultracentrifuge (Beckman-Coulter, Palo Alto). The initial protein concentrations were 0.2 - 0.6 mg/ml in 20 mM Tris-HCl pH 7.5, 5% glycerol, 1 mM TCEP, 0.8% β-OG and 1 mM MgCl₂ (buffer B). The equilibrium absorbance profiles were recorded after 20 hours at 280 nm. A further record taken 2 hours later proved the equilibrium was reached.

Analysis of the profiles was performed with the Beckman software package and the Schuck program DISCREEQ (Schuck P. (1994). Simultaneous radial and wavelength analysis with the Optima XL-A analytical centrifuge, *Progr.Colloid Polym.Sci.* 94, 1-13). All solvent densities matched to the detergent densities by addition of sucrose, or not matched, were measured with a PAAR density meter, type DMA4500 (Anton Paar, Graz, Austria). They are given in Table 2. The detergent densities were assessed in the buffer without protein from separate SE runs where the interference detection optics was used for the non absorbing detergents. The partial specific volume computed from the amino acids sequence according to Edsall (Edsall JT, in Cohn EJ, Edsall JT (eds) (1943) Protein, amino acids and peptides as ions and dipolar ions. Reinhold, New York, 370-381) is 0.729 cm³/g. Because of the sucrose addition the hydration state of the protein has to be considered. An estimated value for vbar can be given by the average between a fully hydrated protein vbar = 0.787 cm³/g (Rickwood D. and Chambers J.A.A. (1984) in *Centrifugation*, *a practical approach,* 2^{nd} Edition, Edited by D. Rickwood, IRL Press, Oxford) and the non-hydrated protein as indicated above. Best fits however were obtained for vbar = 0.766 cm³/g. Thus, this value was used. In the case of LDAO the density of 0.914 g/cm³ was not compensated. The profiles were analysed by a model with various numbers of bound micelles. Best fits were obtained with 1 bound micelle. Thus, the analysis was carried out under this assumption providing a vbar value of 0.791 cm³/g. Similarly, the non-density-matched sample with β-OG under the cmc was analysed with vbar = 0746 cm³/g. This value corresponds to 10 bound β-OG.

The purified OSC on SDS-PAGE showed a protein band, corresponding to a molecular mass of 80kDa, which is a little bit lower than the predicted molecular mass of 85'690 Da. Mass spectral analysis of the protein resulted in a mass of 85'700 Da. This result was nearly identical to the theoretical predicted mass for hOSC-tag without the N-terminal methionin indicating that the protein is not glycosylated or posttranlational modified.

The purified hOSC-tag was characterized by a sedimentation equilibrium experiment with the analytical ultracentrifuge. The solvent density was adjusted to the density of the detergent according to the approach established by Lustig et al. (Lustig, A., Engel, A., Tsiotis, G., Landau, E.M. & Baschong, W. (2000). *Molecular weight determination of membrane proteins by sedimentation equilibrium at the sucrose or nycodenz-adjusted density of the hydrated detergent micelle.* Biochim Biophys Acta 1464, 199-206). At a β-OG concentration of 0.8% the protein was monomeric with a moelcular weight of 86 kDA. The same oligomerisation state was found for the wild-type hOSC. At a concentration of 0.2% large losses for aggregates with molecular mass higher than 400 kDa and a broad distribution between dimers, trimers and tetramers (Table 2) were observed. Since analytical ultracentrifugation allows such a detailed picture on the oligomerisation state this method was used also to test the influence of other detergents on the oligomerisation state. In the presence of 0.2% Triton X-100 in the buffer the protein is also monomeric and monodisperse. The same was found at a C₈E₅ concentration of 0.6% (CMC 0.2%), but 50% of the protein was lost as large aggregates (>400 kDa). In detergents like LDAO and DDM the protein was polydisperse with a mixture of monomers, dimers and tetramers and more than 50% of the protein was lost as large aggregates. This effect was more pronounced for DDM. The correlation with the activity measurements showed that the detergents with monomeric hOSC have the highest activity (Fig. 2).

### Example 3: Crystallization

For crystallization hOSC was concentrated to approximately 10 mg/ml in a buffer containing 0.3% (w/v) β-OG. Detergent was added prior to crystallization to concentrations above the CMC. For inhibitor complex co-crystals the protein was incubated with 5mM [4-[6-(allyl-methyl-amino)-hexyloxy]-2-fluoro-phenyl]-(4-bromo-phenyl)-methanone fumarate (Fig. 6) over night prior to the crystallization. Crystals were grown at room temperature in hanging drops by mixing equal volumes of protein solution with the reservoir solution consisting of 25%PEG 3350, 0.4M ammonium acetate, 0.1M Tris, pH8.5, 10% (w/v) ethylene glycol. Once the initial crystallization conditions were established crystals were grown using seeding methods.

Initial hOSC crystals were obtained at room temperature by mixing equal volumes of a reservoir solution containing 30% (w/v) PEG 4000, 0.2M sodium acetate in 0.1M Tris pH 8.5 with the protein solution containing 2.4% (w/v) β-OG. The resulting β-OG concentration is well above the saturation concentration of β-OG at the crystallization conditions and crystals of β-OG grew in the crystallization setups together with the protein crystals (Figure 4A).

Crystallization of hOSC can be achieved at numerous conditions, as long as the pH is around pH 8.5 and the precipitant is PEG. hOSC was crystallized at PEG concentrations varying from 20% to 30% (w/v) PEG 3350 and PEG 4000. The addition of 100mM to 400mM of a salt, preferably ammonium or sodium acetate, helped in obtaining crystals. But hOSC could be crystallized in the absence of salt and in the presence of all of the following salts: ammonium acetate, sodium acetate, sodium and potassium tartrate, sodium malonate, sodium succinate and sodium citrate, magnesium chloride, ammonium sulfate, sodium chloride, lithium sulfate. Big and untwinned hOSC crystals suitable for structure determination could be grown in the presence of the small amphiphilic organic molecules glycerol, ethylene glycol or sucrose at concentrations ranging from 5% to 15%(w/v). These molecules have an amphiphilic characteristic that influences the phase behavior of β-OG, avoiding phase separation in the crystallization drops. These molecules also increase viscosity of the solution and therefore slow down crystal growth. Moreover they act as cryo protectants.

Well diffracting crystals of membrane proteins often grow at crystallization conditions near the cloud point, when no phase separation is observed (Rosenow, M., et al. (2001), *Amphiphiles modify the properties of detergent solutions used in crystallization of membrane proteins,* Acta Crystallogr D Biol Crystallogr *57*, 925-7 2001). The phase behavior of β-OG was modulated by the variation of acetate concentration and the addition of small amphiphiles in order to avoid phase separation in the crystallization drop. Dynamic light scattering is used to predict the crystallization properties of soluble proteins by assessing the aggregation state. Monodispers proteins are more likely to crystallize. (D'Arcy, A. (1994), *Crystallizing Proteins - a Rational Approach?,* Acta Cryst *D50,* 469-471). In the case of the membrane protein hOSC the assessment of protein aggregation by analytical ultra centrifugation proved to be a tool for the prediction of the crystallizability. In agreement with the results on aggregation obtained by analytical ultra centrifugation, it was only possible to crystallize hOSC that was monodispersely solubilized with the detergent β-OG at concentrations above the CMC.

### Example 4: X-ray Data Collection and Analysis

Crystals were mounted in a loop and fast cooled in liquid nitrogen. Diffraction images in several crystal orientations were collected with CuKα radiation at the home source in order to detect crystal disorder that was not readily visible under the microscope alone. Untwinned crystals were selected for the collection of full data sets with synchrotron radiation at the beamline PX6A at the Swiss Light Source (Villigen, Switzerland) at 120K. The crystals diffracted at the beam line X06A up to 1.8 Å resolution. The crystals belong to space group C222₁ with cell dimensions of about 190 Å, 202 Å and 62 Å. Data was processed with DENZO (Otwinowski, Z. & Minor, W.M. *Processing of X-ray diffraction data collected in oscillation mode.* Methods Enzymol. *276,* 307-326 ( 1997)) and XDS (Kabsch, W. (1993) *Automatic processing of rotation diffraction data from crystals of initially unknown symmetry and cell constants* J. appl. Cryst. *26*, 795-800).

### Structure determination and refinement

The structure was solved by molecular replacement with the CCP4 (Collaborative Computational Project, Number 4, *The CCP4 Suite: Programs for Protein Crystallography* Acta Cryst. *D* 50, 760-763(1994)) program MOLREP (A.Vagin,A.Teplyakov, *MOLREP: an automated program for molecular replacement., J.* Appl. Cryst. *30*, 1022-1025. (1997)) using squalene hopene cyclase (Wendt, K.U., Lenhart A., Schulz, G.E. *The structure of the membrane protein squalene hopene cyclase at 2.0 Å resolution*, J.Mol. Biol. *286,* 175-187, (1999), pdb-entry 2sqc ) as search model and was automatically rebuilt and refined using the program ARP/wARP (Morris, Perrakis & Lamzin, Acta Cryst. D58:968-975,2002). After adding the ligands to the model and minor manual rebuilding of the protein with the molecular graphics program MOLOC (Gerber P.R., Müller K., The *MOLOC program with the MAB force field,* J. Comput. Aided Mol. Design *9*, 251-268 (1995)) it was refined with REFMAC (Murshudow, G.N., Vagin, A.A. & Dodson, E.J. *Refinement of macromolecular structures by the maximum-likelyhood method.* Acta Crystallogr. *D*53, 240-255 (1997)).

### Monomeric structure of hOSC

As a typical membrane protein hOSC is not soluble in the absence of detergents and detergents had to be added to obtain stable and active enzyme. Crystals of hOSC were grown in the presence of β-OG micelles and the structure was solved by molecular replacement. In agreement with our gel filtartion results and the analytical ultra centrifuge data on detergent solubilized hOSC (Table 2) hOSC is monomeric in the crystal. This is in contradiction to the dimeric behavior of OSC from rat liver preparations observed in gelfiltration experiments (Kusano et al. (1991) Chem Pharm Bull *39*, 239-241). The pairs of hOSC molecules that are created by applying the crystallographic twofold axes of space group C222₁ have membrane portions that point into different directions. They can therefore not be biological dimers. OSC consists of two (α/α) barrel domains that are connected by loops and four smaller β-structures. Domain 1 is a (α/α)₆ barrel whereas domain 2, that is inserted into domain 1, contains an (α/α) barrel that appears to be an evolved form of the (α/α)₆ barrel of domain 1 with some helices missing (Wendt, K.U., Poralla, K., Schulz, G.E., *Structure and function of a squalene cyclase,* Science *277*, 1811-1815(1997)). The amino ends of all inner helices of both barrel domains point to the center of the molecule. The loops connecting the helices and the N-terminal region with their β-structures fill the space between the barrel domains and enclose the large active site cavity that is located in the center of the hOSC molecule (Figure 3). Domain 2 carries an additional helix that forms part of the hydrophobic plateau, which is the point of membrane insertion of the monotopic membrane protein hOSC.

The five QW-sequence motifs, that are located C-terminal of some helices of the outer barrel ring (Poralla et al. (1994) Trends Biochem. Sci. *19*, 157) assume a conformation with stacked side chains of Q and W that was also observed in the structure of the archeal homologue squalene hopen cyclase. They connect the outer helices with the inner helices by hydrogen bonds and are thought to contribute to thermodynamic stability of cyclases (Wendt, K.U., Poralla, K., Schulz, G.E. (1997), *Structure and function of a squalene cyclase,* Science *277*, 1811-1815).

### Substrate binding pocket

The large active site cavity is located between the two (α/α) barrel domains at the center of the OSC molecule. It is enclosed by loops and several β-sheets . It is lined mostly by hydrophobic and aromatic residues. The cavity extends from the catalytic Asp455 at the "top" down towards a channel that reaches the enzyme surface at the hydrophobic plateau. This channel is separated from the cavity by a constriction that is created by the side chains of residues Trp192, Cys233, Tyr237, Ile524 and M525 (Figure 5). The channel blocking side chain of Tyr237 in this constriction can permit the passage of a substrate to the active site cavity by adopting another conformation. This channel is thought to be the substrate uptake channel, allowing the hydrophobic substrate to reach the catalytic residues of hOSC in a non-polar environment (Wendt & Schulz, Science 1998). There is another channel leading from the hydrophilic surface of domain 1 towards the active site cavity. This channel is separated from the active site cavity by the side chains of residues Phe391, Phe591 and Glu459.

### Inhibitor binding

The co-crystallized inhibitor was completely defined in the initial 2.2 Å (Fo-Fc)-electron density map at the 3σ contour level. It binds in the active site cavity. The allyl-amino group of the inhibitor is hydrogen bound to the catalytic aspartate Asp455 that is responsible for initiating the cyclization reaction by protonating oxido-squalene. There is a charged hydrogen bond between the protonated amino nitrogen and the Asp455 carboxylate group at 2.9Å distance. The amino group also interacts with several aromatic residues that are thought to initiate the cyclization reaction by stabilizing the positively charged early high-energy intermediates (Figure 5B). The amino N has cation-π interactions with Trp387 and Trp581 at distances of 4.7Å to 5.4Å. The distance to the hydroxyl. groups of Tyr98 and Tyr704 (7.7Å and 6.4A) are too big for dielectric stabilization of the positive charge.

The alkyl spacer between the allyl-amino and the benzphenone groups is not fully extended showing a distance of only 6.3Å from the amino-N-atom to the ether-O-atom. The distance between amine nitrogen and the benzophenone carbonyl has a influence on the potency of the inhibitor, with lowest IC50 values for a distance of 10.7A (Jolidon, S., Polak-Wyss, A., Hartmann, P.G., and Guerry, P. (1993). *2,3-Oxidosqualene-lanosterol cyclase: an attractive target for antifungal drug design.* In: Recent Advances in the Chemistry of Anti-infective Agents, P.H. Bentley, ed. (Cambridge, U.K.: Royal Soc. Chemistry), pp.223-233). In the hOSC complex of inhibitor [4-[6-(allyl-methyl-amino)-hexyloxy]-2-fluoro-phenyl]-(4-bromo-phenyl)-methanone fumarate this distance is 12.0Å, which compares well to the distance between the epoxide oxygen and the carbocation position of the protosterol cation.

The phenyl groups of the benzophenone portion of the inhibitor make several aromatic interactions to the protein. The terminal bromo phenyl group has edge-to-face on interactions with Trp192 (3.6Å) and Trp230 (3.7Å) and a face-to-face interaction with Phe521 (4.6Å). The fluoro phenylgroup is stacked between the Phe696 (3.7Å) and the His232 imidazole ring (3.1Å) (Figure 5C). His232 is not within hydrogen bond distance to the benzophenone carbonyl group. The benzophenone carbonyl is hydrogen bonded to a water molecule which in turn is bound to the amid N of Ile338. This water mediated interaction is in good agreement with the observation that the IC50 of compounds containing aryl-X-aryl groups shows no dependence on the hydrogen bond acceptor properties of the linker X (Brown et al., 1999, J.Med.Chem 42, pp1306-1311). The terminal bromo group of the inhibitor points towards the substrate uptake channel, interacting with the residues Ile524, Tyr237, Ser518 and Ser518 of the channel constriction site. hOSC does not move upon inhibitor binding as shown by the rmsd-Cα of 0.32Å between inhibitor-liganded and free hOSC. The inhibitor adapts to the geometry of the cavity assuming a conformation different from that in the complex with the homologue SHC (Lenhart, A.; Weihofen, W.A.; Pleschke, A. E. and Schulz, G.E.; Chemistry & Biology, 2002, Vol. 9, 639-645, 2002).

In contrast to hOSC the bacterial squalene hopene cyclase (SHC) is dimeric (Wendt, K.U., Poralla, K., Schulz, G.E. (1997), *Structure and function of a squalene cyclase,* Science, *277*, 1811-1815). OSC and SHC have a common cyclase chain fold. The superposition of the structures yields 550 pairs of α-carbon atoms with a rmsd of 1.76Å when a 4.5Å cutoff was applied (pdb entry 2sqc). The rmsd-Cα is 0.85Å, when only the 94 residues are superimposed that are conserved spatially and in sequence.

When compared to SHC, OSC has 70 additional N-terminal amino acid residues that form a small structural motif that folds into the region between the two (α/α) barrel domains opposite to the membrane attachment site. Together with the extended loop 427-431 this N-terminal structural motif could solidify the loops between the domains that form most of the ligand binding pocket. The relative orientation of the two (α/α) barrel domains in hOSC differs slightly from that in SHC.

The (α/α) barrel in domain 1 of hOSC is wider than that in SHC, with the Cα distance across the inner ring of helices increased by 1.2Å to 15.8Å (distance between A536 Cα to W714 Cα). This barrel widening might be a consequence of the more bulky OSC side chains that are pointing into the barrel center at the C-terminal side of the barrel (eg. F391, W590 and W714).

### Example 5: Identification of new OSC inhibitors by docking to hOSC crystal structure

### Method (Docking)

Docking calculations were performed with the docking software FRED (OpenEye Scientific Software). The first stage in the docking procedure is a shape fitting routine (docking algorithm), which takes a set of ligand conformers as input and tests them against a "bump map" (a Boolean grid with true values where ligand atoms can potentially be place). Orientations that clash with the protein or are distant from the active site are rejected. The crude docking solutions are further tested against a specified pharmacophore feature, and any poses that do not satisfy the pharmacophore are rejected. Poses surviving the shape fitting routine are then passed through a scoring function filter. In the docking procedure for OSC, docking was performed twice with two different scoring function filters, ChemScore and PLP. 5000 poses were returned by each of the two docking runs, and a consensus list of common compounds (∼500) from both runs was generated. Flags for optimization of hydroxyl group rotamers, rigid body optimization and torsion optimization were set to 'true' values in the scoring process. The number of compounds in the consensus list was further reduced by filtering out unwanted substructures (e.g. benzamidine) and by removing highly similar compounds (maximization of structural diversity). Finally 138 compounds (0.03% from virtually screened Roche compound library) were sent for screening.

### Receptor preparation

Water molecules were removed from the OSC X-ray structure and polar hydrogen atoms were added (Gerber P.R., Müller K. (1995), *The MOLOC program with the MAB force field,* J. Comput. Aid ed Mol. Design *9*, 251-268) to the structure.

### Binding site definition

The OSC inhibitor trans-Methyl-{4-[5-(methyl-propyl-amino)-pent-1-ynyl]-cyclohexyl}-carbamic acid 4-chloro-phenyl ester was used for the binding site definition in FRED. FRED builds a rectangular box around a given (x-ray, manually modeled) ligand and allows to increase the box size by the '-addbox' flag. The box around trans-Methyl-{4-[5-(methyl-propyl-amino)-pent-1-ynyl]-cyclohexyl}-carbamic acid 4-chloro-phenyl ester was increased by 0.5 Angstrom.

### Compound library preparation

For compounds from the Roche compound library multiconformer libraries in a binary format were generated by OMEGA (OpenEye Scientific Software). Modifications applied to the default settings of OMEGA were (i) rejection of conformers with an energy difference to the global minimum of >5.0 kcal/mol (GP_ENERGY_WINDOW), (ii) maximum number of output conformers 400 (GP_NUM_OUTPUT_CONFS) and (iii) low energy selection (no random selection) of conformers from the final ensemble (GP_SELECT_RANDOM false).

### Results

20 compounds showed reasonable inhibition at 1 µM in the activity assay with liver microsomal preparations as described in Example 2 and were retested at 50 µM. 7 compounds showed >20% inhibition at 50 µM. 2 compounds showed good inhibition of 68% and 75% at 50 µM. The compounds represent a structurally new class of OSC inhibitors and disproved the assumption that a basic nitrogen atom is necessary for OSC inhibition. Further compounds showed 35%, 38%, and 57% inhibition at 50 µM.

### OSC for drug-design (active site differences to SHC)

Aromatic residues (indicated by an asterisk in Fig. 7) at the top of the hydrophobic tunnel are highly conserved between members of the cyclase family to which OSC and SHC are belonging. The aromatic side chains are supposed to be important for stabilization of cations via π-cation-interactions in the cyclization cascade of squalene (SHC) and 2,3-oxidosqualene (OSC). The high conservation of the aromatic residues in the top of the hydrophobic tunnel is in good accordance to the analogy of postulated cyclization mechanisms for A-ring formation by both cyclases. Differences occur however in the initiation of the cyclase-catalyzed process, SHC needs to initiate the cyclization cascade by protonation of a C=C double bond of squalene (olefin protonation) whereas OSC catalysis is initiated by epoxide protonation of 2,3-oxidosqualene.

For olefin protonation in SHC catalysis a strong Brønsted acid is required. Based on crystallographic data it was proposed that acidity of catalytic Asp is increased by hydrogen bonding to a protonated His (Fig. 8) and additionally through an ordered water molecule. The acidity of the pair Asp:His seems to be sufficient to protonate squalene. In contrast, for OSC an adequate activating residue in the position of His in SHC is lacking (His in SHC can be superimposed with Ala in OSC). Because epoxides are more readily protonated than olefins, it was assumed that an isolated aspartic acid in OSC's may suffice for initiation. Experiments showed however that 2,3-oxidosqualene is completely stable in neutral media and in glacial acetic acid at room temperature for about 1 day. The crystallographic data for OSC now revealed close Cysteines that are conserved in the OSC family to be important for activation of catalytic Asp. Differences in the activation of the two aspartic acids result in completely different side-chain orientations for the catalytic residue (Fig. 8). The different side-chain orientations provide important information for structure-based drug design. The example shown in Fig. 7 and 8 (only fragment of [4-[6-(allyl-methyl-amino)-hexyloxy]-2-fluoro-phenyl]-(4-bromo-phenyl)-methanone fumarate is shown) shows, that the side-chain orientation of catalytic Asp in SHC is not appropriate to form a hydrogen bond to the protonated amine of the ligand, the positive charge in the [4-[6-(allyl-methyl-amino)-hexyloxy]-2-fluoro-phenyl]-(4-bromo-phenyl)-methanone fumarate -SHC complex is stabilized by π-cation-interactions of a Trp side-chain at the bottom of the hydrophobic tunnel. The side-chain of catalytic Asp in contrast directs both oxygen atoms toward the hydrophobic tunnel. Thus, forming efficient hydrogen bonding interactions to [4-[6-(allyl-methyl-amino)-hexyloxy]-2-fluoro-phenyl]-(4-bromo-phenyl)-methanone fumarate. As a result for structure-guided drug design, inhibitors are shifted backwards in the binding site (position of nitrogen atoms differ by 1.9 Å) and effect the length of inhibitors to optimally fill the hydrophobic tunnel. Further, the possibility to attach to catalytic Asp via direct hydrogen bonding offers possibilities for the structure-based design of new structural classes to efficiently inhibit OSC and to avoid having a basic amine function in the inhibitors that often come along with metabolic instability.

At the back of the hydrophobic tunnel the number of aromatic residues for both cyclases decreases, whereas sequence and structural differences between the cyclases increase. Comparison of the two complex structures with the inhibitor [4-[6-(allyl-methyl-amino)-hexyloxy]-2-fluoro-phenyl]-(4-bromo-phenyl)-methanone fumarate shows, that the fluorinated aromatic ring is located exactly between two aromatic side chains for SHC. Since residues for similar π-interactions are lacking in OSC and because of the shift in the binding pocket caused by the side chain orientation of catalytic Asp the fluorinated aromatic ring of the inhibitor in OSC is located closer to the middle of the hydrophobic tunnel to pick up π-interactions with a close Phe side chain there. As a consequence for structure-based drug design, chain length and location of the π-system of the inhibitor can not be optimized for OSC with structural information of SHC.

More differences between the active sites of the two cyclases are obvious at the end of the hydrophobic tunnel as well as in residues participating in the formation of the entrance to the hydrophobic tunnel. The binding site of OSC in the back part is shorter and accommodates water molecules. Thus, to avoid steric conflicts and to gain binding affinity for OSC, OSC inhibitors have to be shorter than comparable SHC inhibitors. Differences in aromatic residues and their location close to the entrance part of the binding site require different steric and electronic properties of OSC inhibitor substituents (*p*-C₄H₄-Cl in [4-[6-(allyl-methyl-amino)-hexyloxy]-2-fluoro-phenyl]-(4-bromo-phenyl)-methanone fumarate) to improve binding properties.

**Table 1:**

| Data collection and refinement statistics | | |
|---|---|---|
| Data Collection | apo | Inhibitor complex |
| Wavelength (Å) | 0.98 | 0.92 |
| Resolution¹ (Å) | 20-2.00 (2.09-2.00) | 20-2.20 (2.26-2.20) |
| Unique reflections¹ | 79898 (9547) | 60002 (4884) |
| Completeness (%)¹ | 99.2 (96.3) | 98.0 (97.5) |
| R_{merge} (%)^{1,2} | 7.6 (48.9) | 9.9 (37.6) |
| <I/σ>¹ | 14.4 (3.1) | 7.7 (2.5) |
| Unit Cell (Space group C222₁) | 189.6 A 201.5 Å 62.1 Å | 189.9 A 202.4 Å 62.6 Å |
| | | |
| Refinement | | |
| Resolution (Å) | 20-2.1 (2.155-2.10) | 20-2.2 (2.26-2.20) |
| R_{cryst}^{1,3} | 17.4 (22.5) | 19.2 (26.2) |
| R_{free}^{1,4} | 19.3 (27.6) | 22.5 (30.2) |
| Average B-factor (Å²) | 34.8 | 38.1 |
| R.m.s. deviations from ideality | | |
| Bond lengths (Å) | 0.01 | 0.01 |
| Bond angles (°) | 1.1 | 1.1 |
| Main chain dihedral angles (%)⁵ | | |
| Most favored | 93.2 | 92.3 |
| allowed | 6.0 | 6.4 |

| | | |
|---|---|---|
| ¹ Values in parentheses refer to the highest resolution bins. | | |
| ² R_{merge}=Σ\|I-<I>\|/ΣI where I is intensity. | | |
| ³ R_{cryst}=Σ\|Fₒ-<F_{c}>\|/ΣFₒ where Fₒ is the observed and F_{c} is the calculated structure factor amplitude. | | |
| ⁴ R_{free} was calculated based on 5% of the total data omited during structure refinement. | | |
| ⁵ Calculated with PROCHECK (Laskowski, R.A., MacArthur, M.W., Moss, D.S. & Thornton, J.M. (1993). *PROCHECK: a program to check the stereochemical quality of protein structure.* J. Appl. Crystallogr. 26, 283-291). | | |

**Table 2:**

| **Oligomer distribution obtained with analytical ultracentrifuge** | | | | | | |
|---|---|---|---|---|---|---|
| Oligomer distributions found in the supernatants at equilibrium at 14'000 rpm. In most cases the solvent density was matched to the detergent density (see text).The losses correspond to the amount of pelleted aggregates with molecular weights > 400 kDa. The initial protein concentrations were 0.2 - 0.6 mg/ml in buffer B. | | | | | | |
| detergent^{†} | density ρₛₒₗᵥₑₙₜ [g/cm³] | Losses [%] | monomer [%] | dimer [%] | trimer [%] | tetrame r [%] |
| β-OG (0.2%) below CMC | 1.018 | 55 | 0 | 71 | 11 | 18 |
| β-OG (0.8%) | 1.092 | 6 | 100 | - | | - |
| DDM (0.016 %) | 1.152 | 42 | 70 | 23 | - | 7 |
| Triton X-100 (0.2%) | 1.052 | n.d. | 100 | - | - | - |
| LDAO (0.06%) | 1.102 | 42 | 94 | 6 | - | - |
| C₈E₅ (0.6%) | 1.015 | 51 | 100 | - | - | - |

| | | | | | | |
|---|---|---|---|---|---|---|
| ^{†} = if not otherwise stated two times CMC n.d.: not determined. Because of the high absorption of Triton X-100 at 280 nm the characterization performed with the interference optics did not allow a reliable evaluation of possible sedimentation losses | | | | | | |

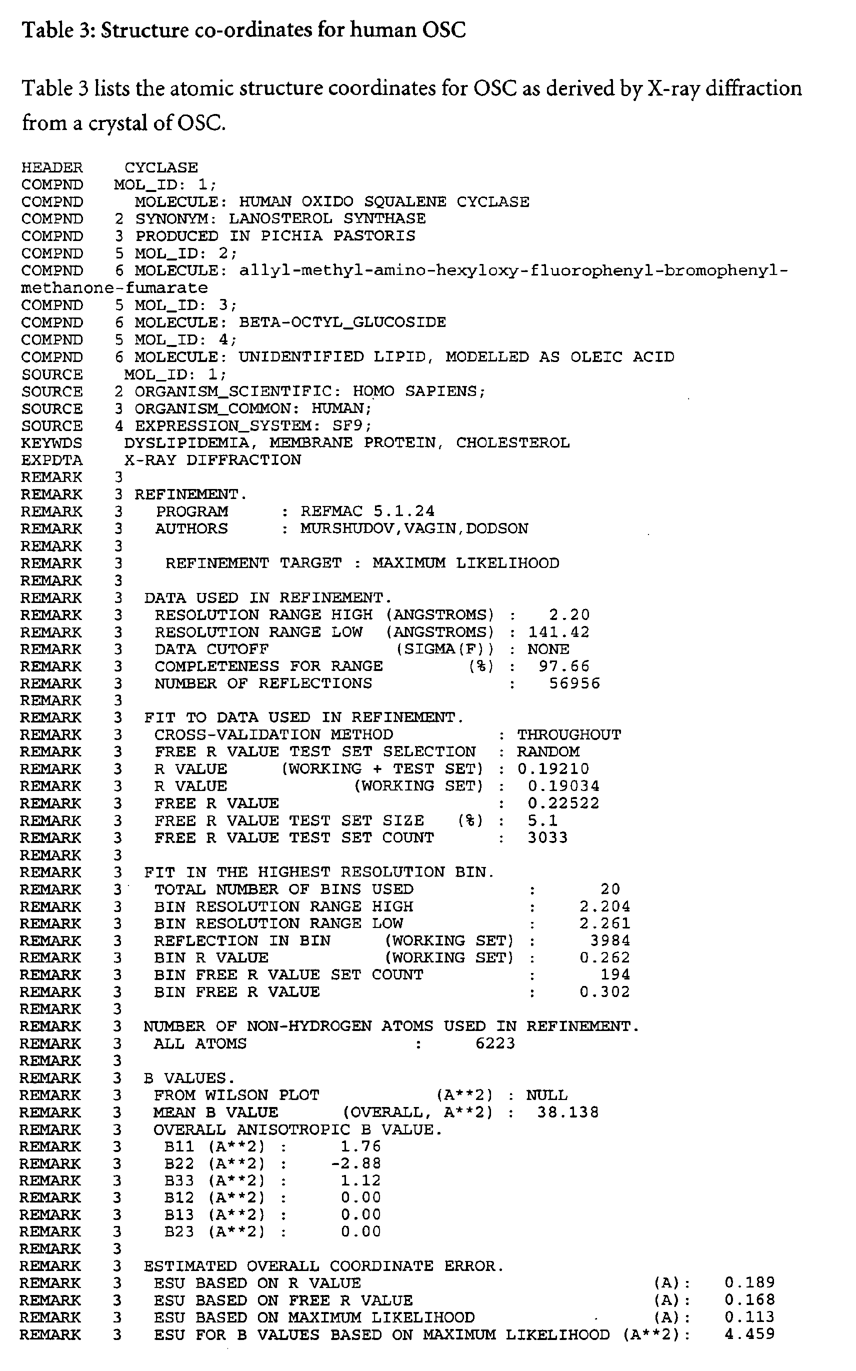

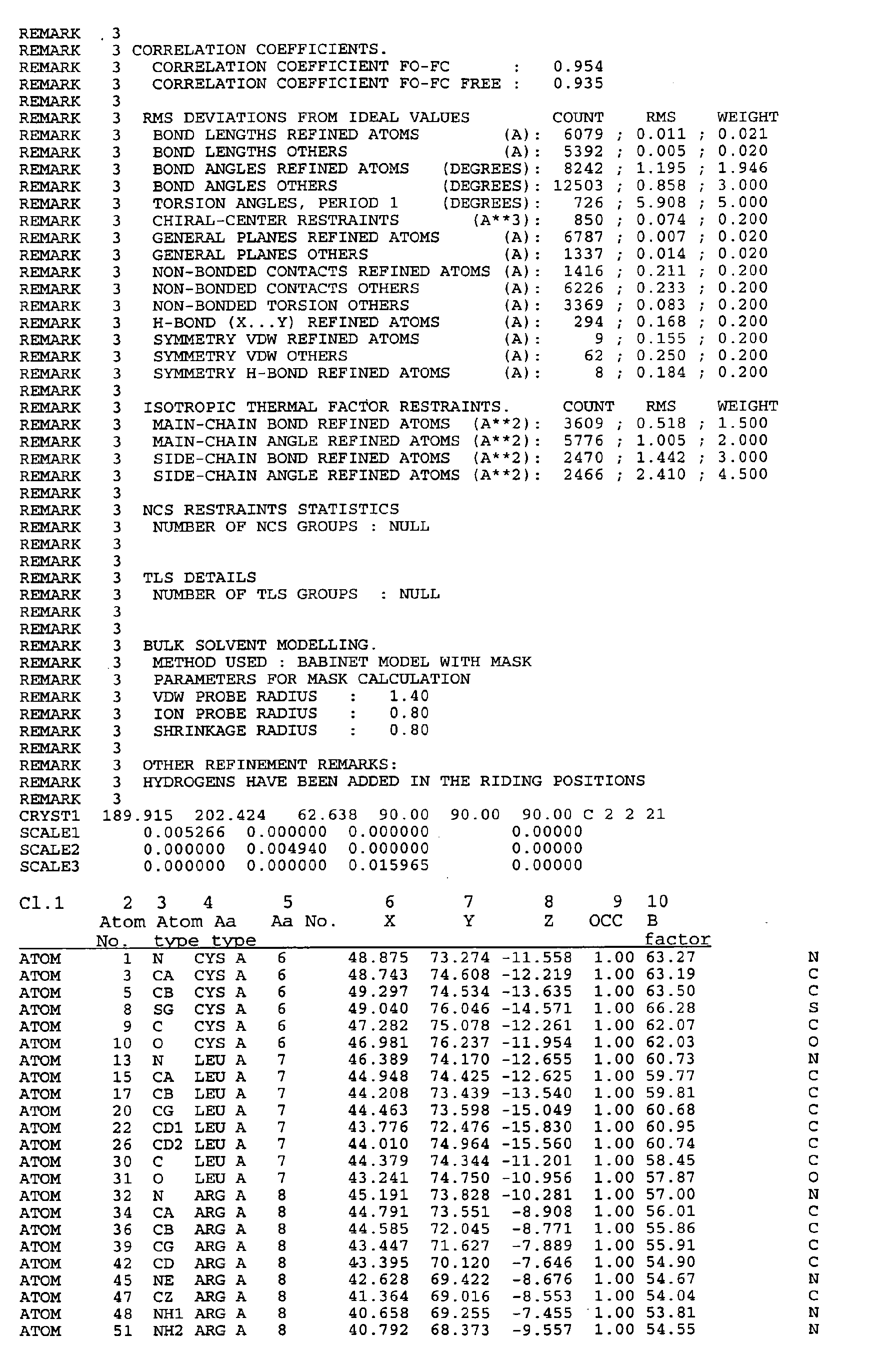

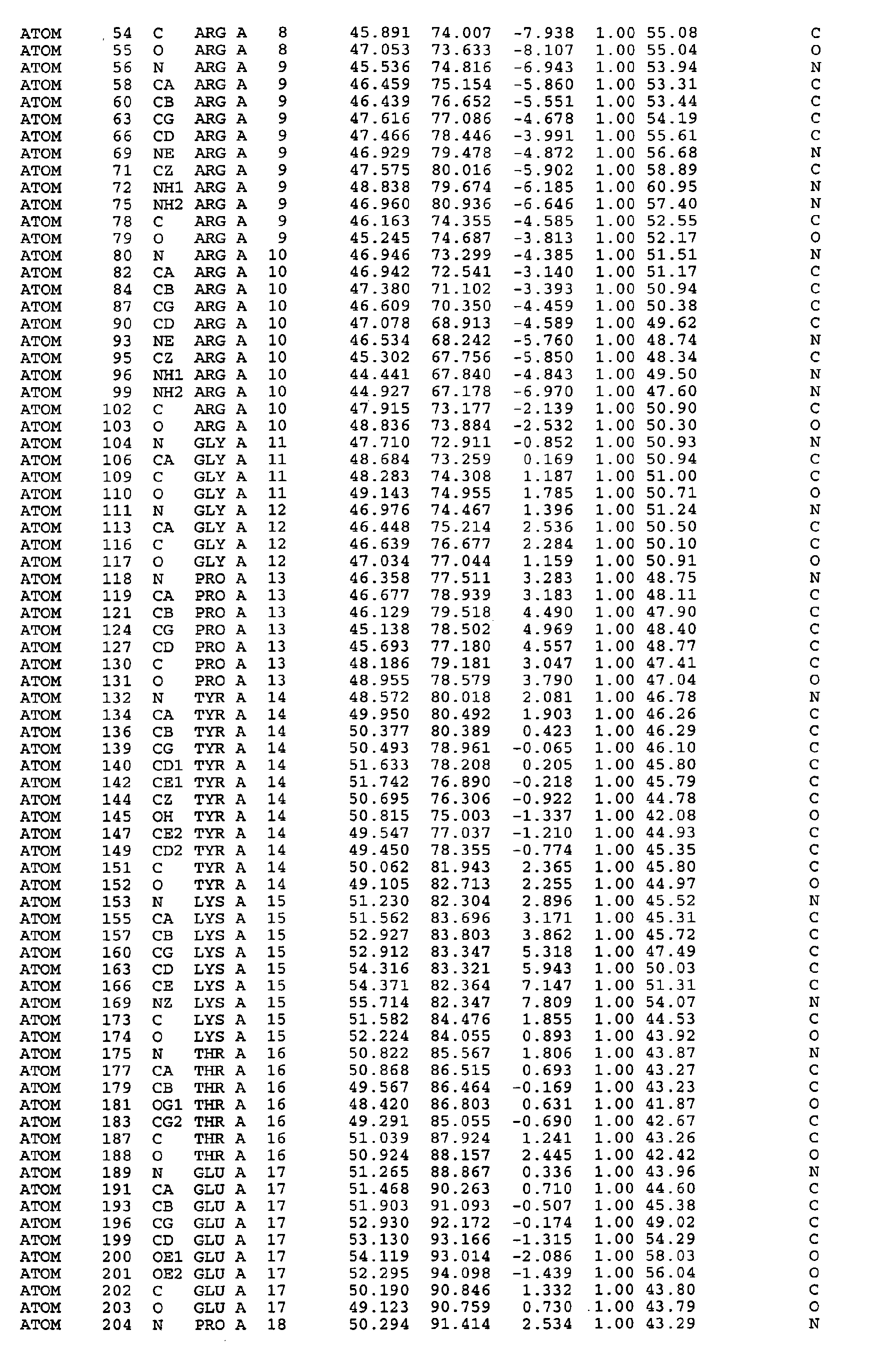

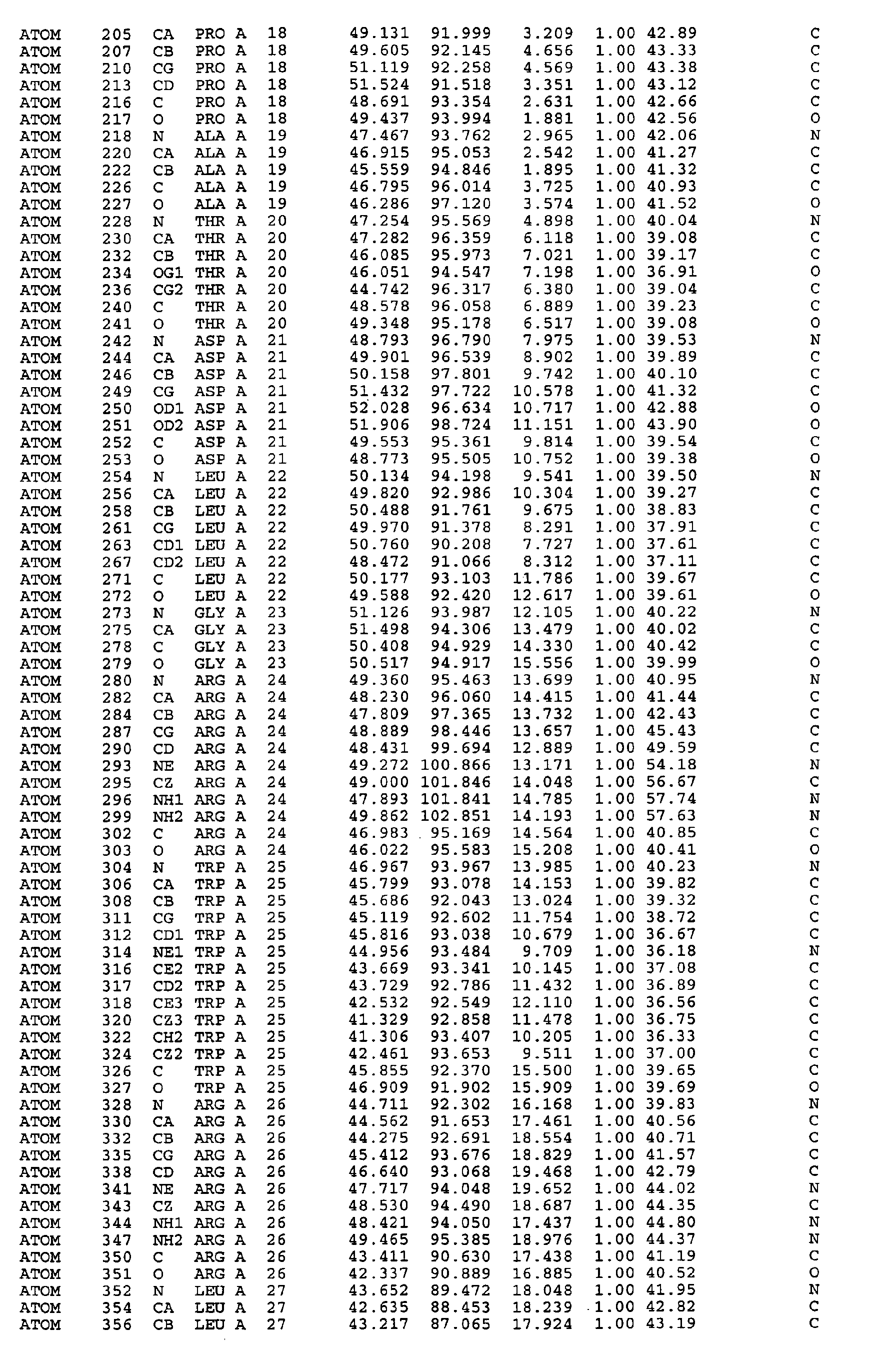

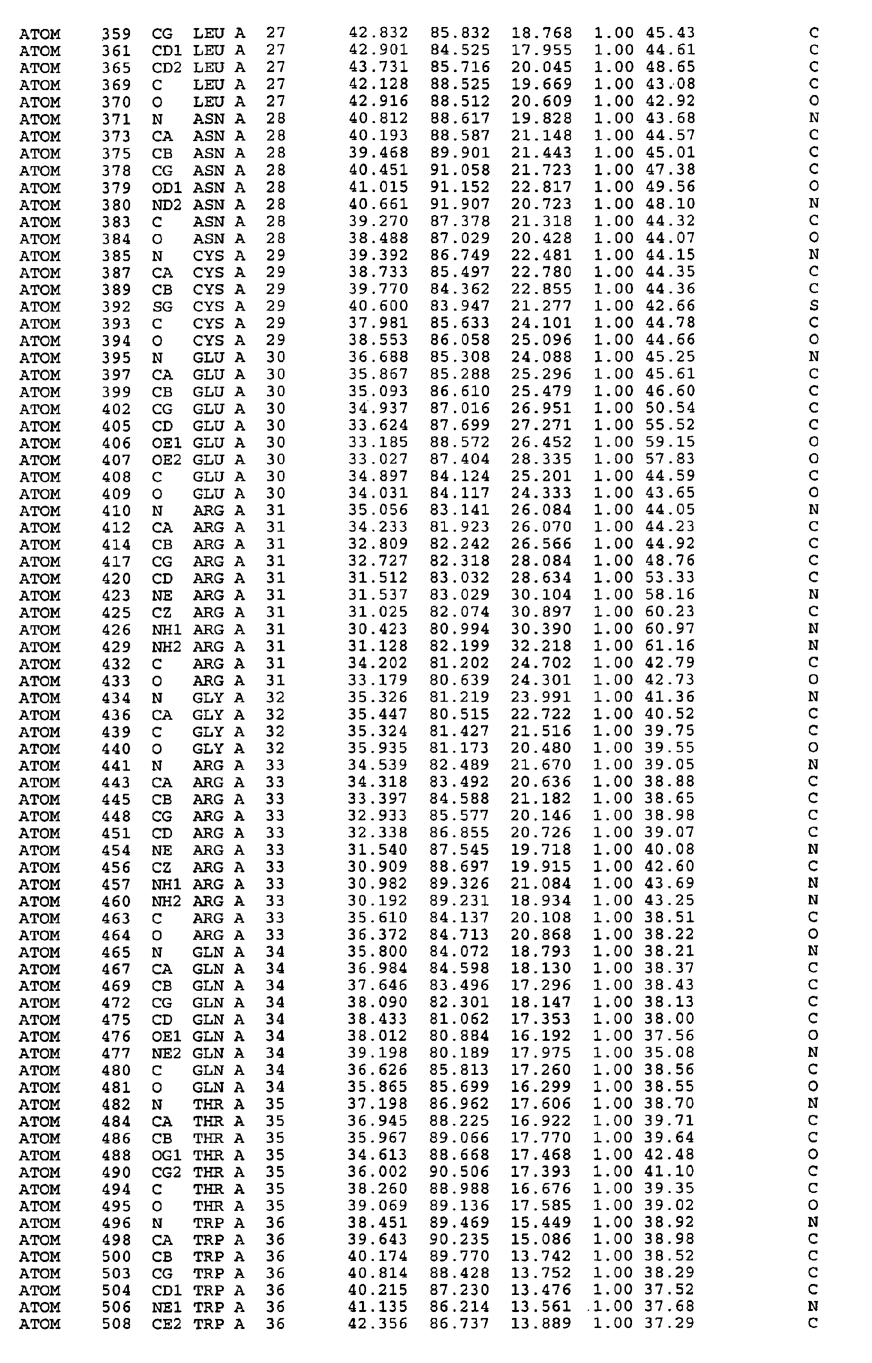

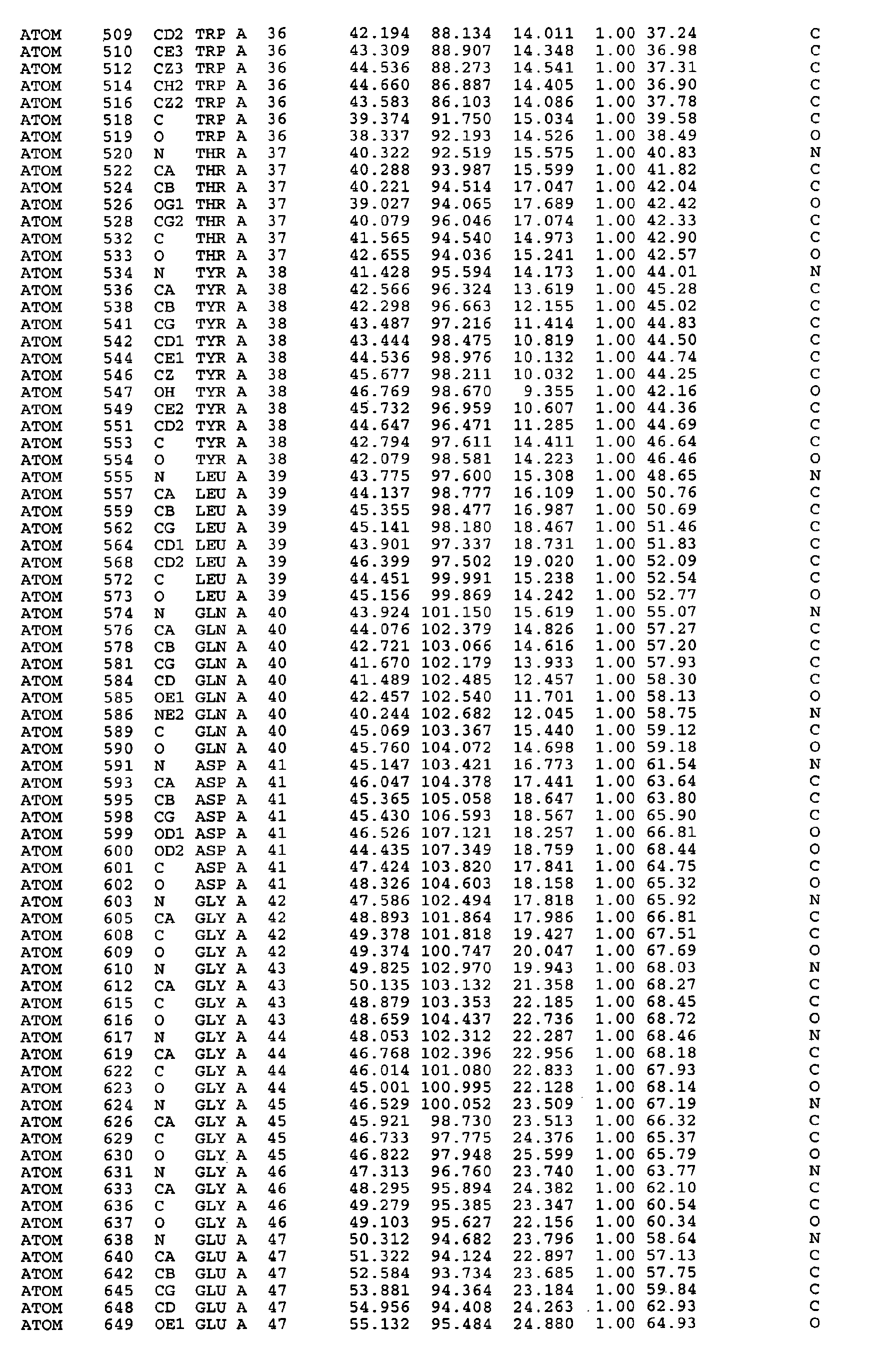

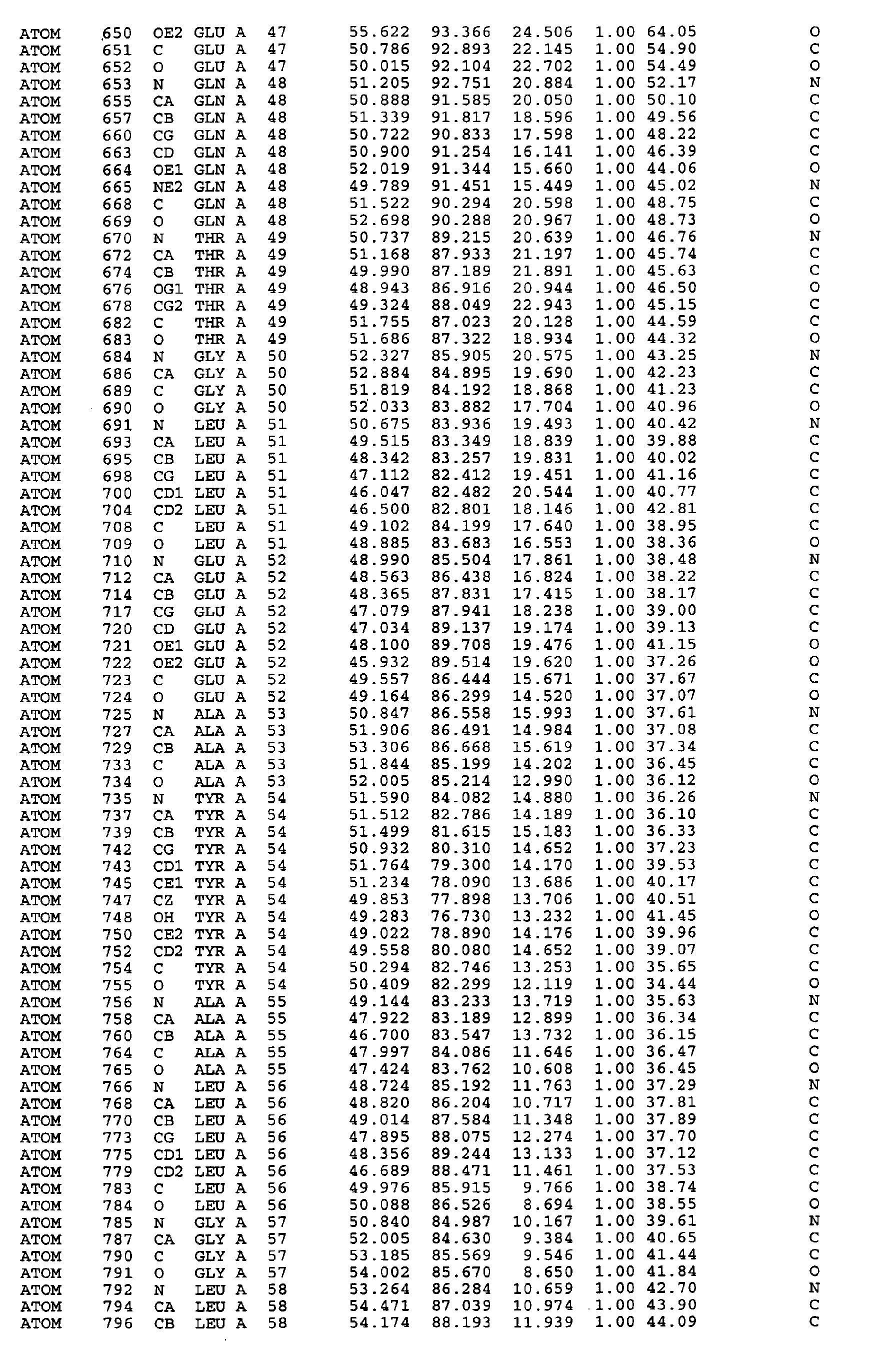

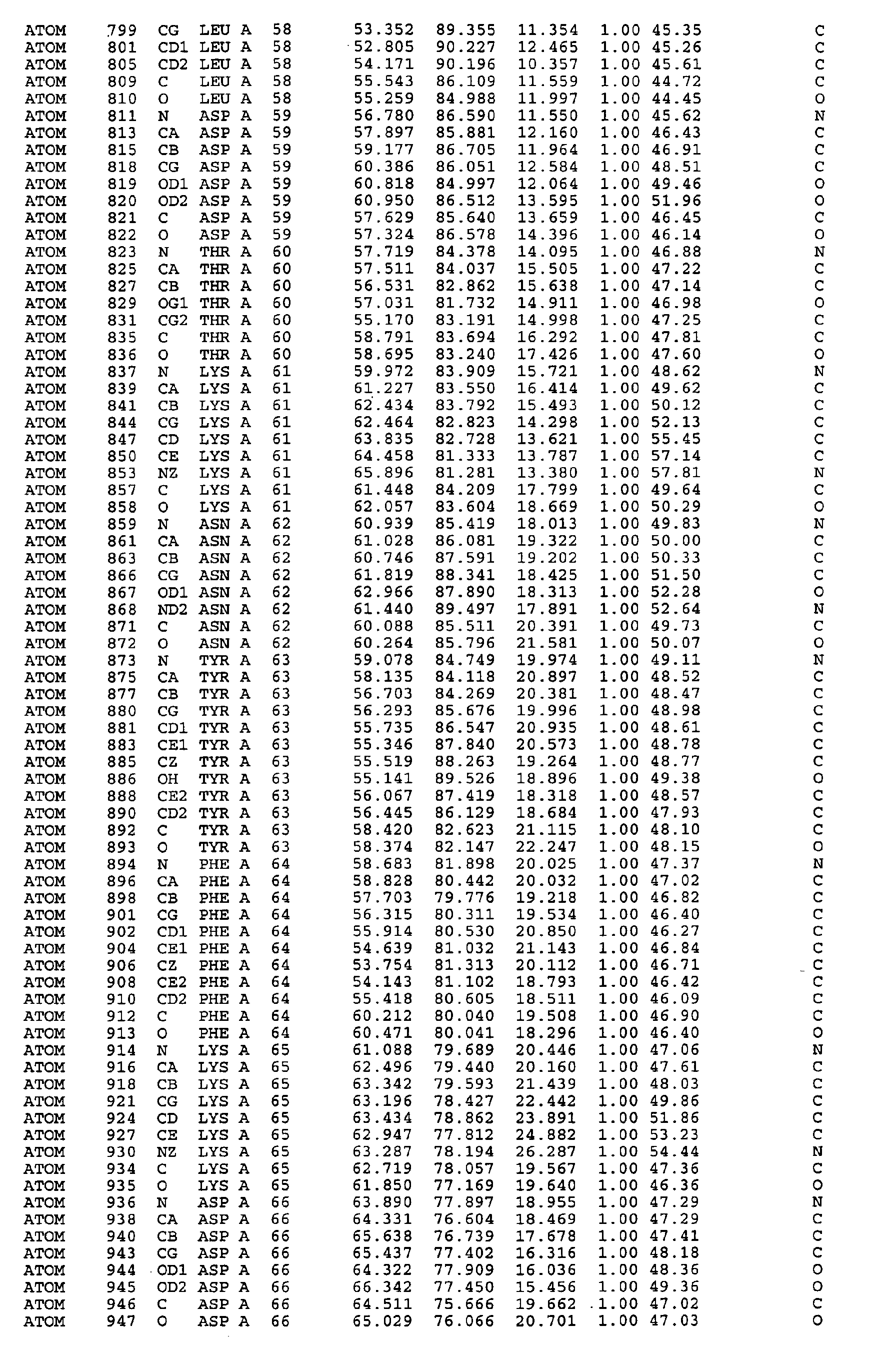

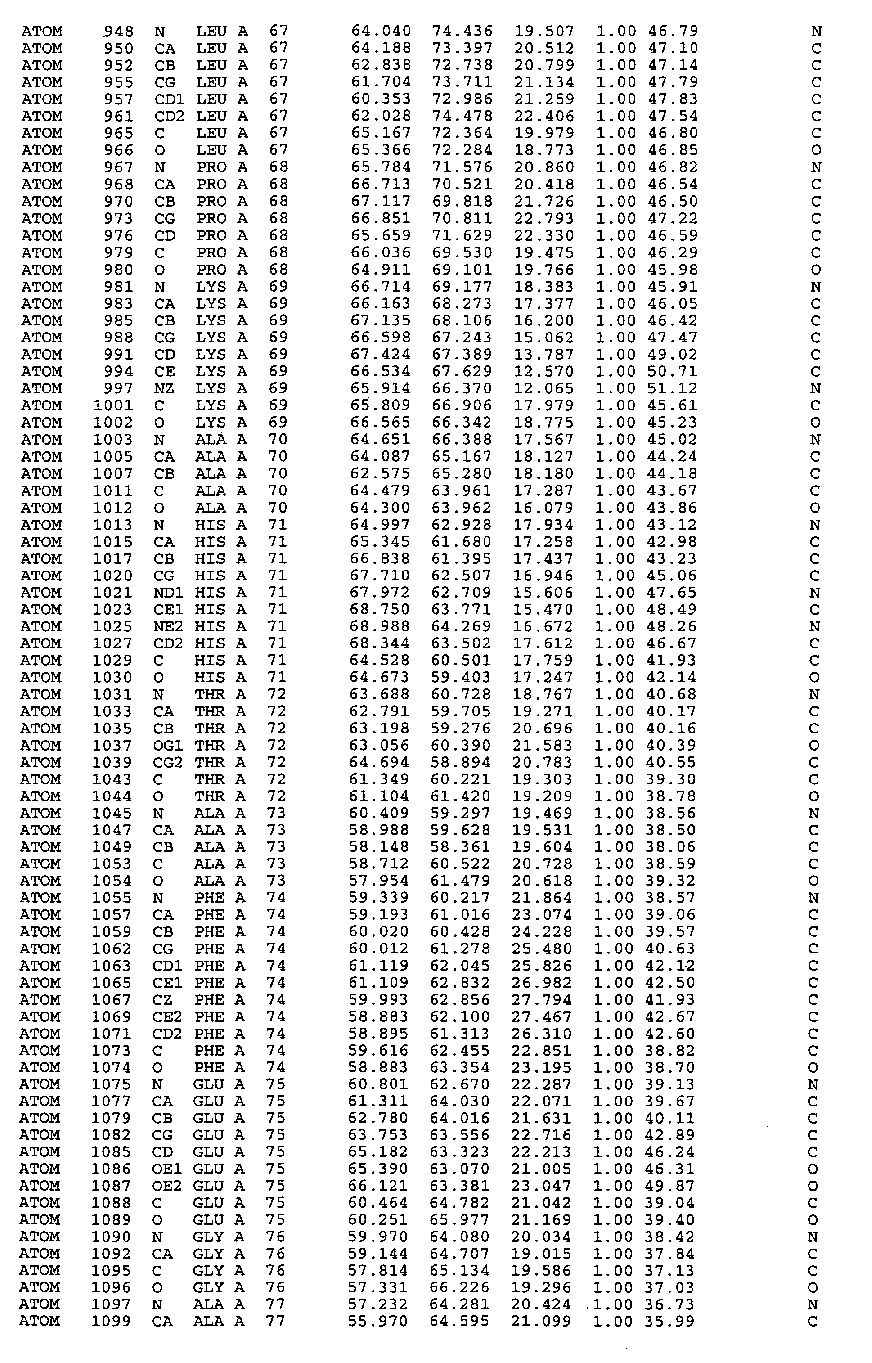

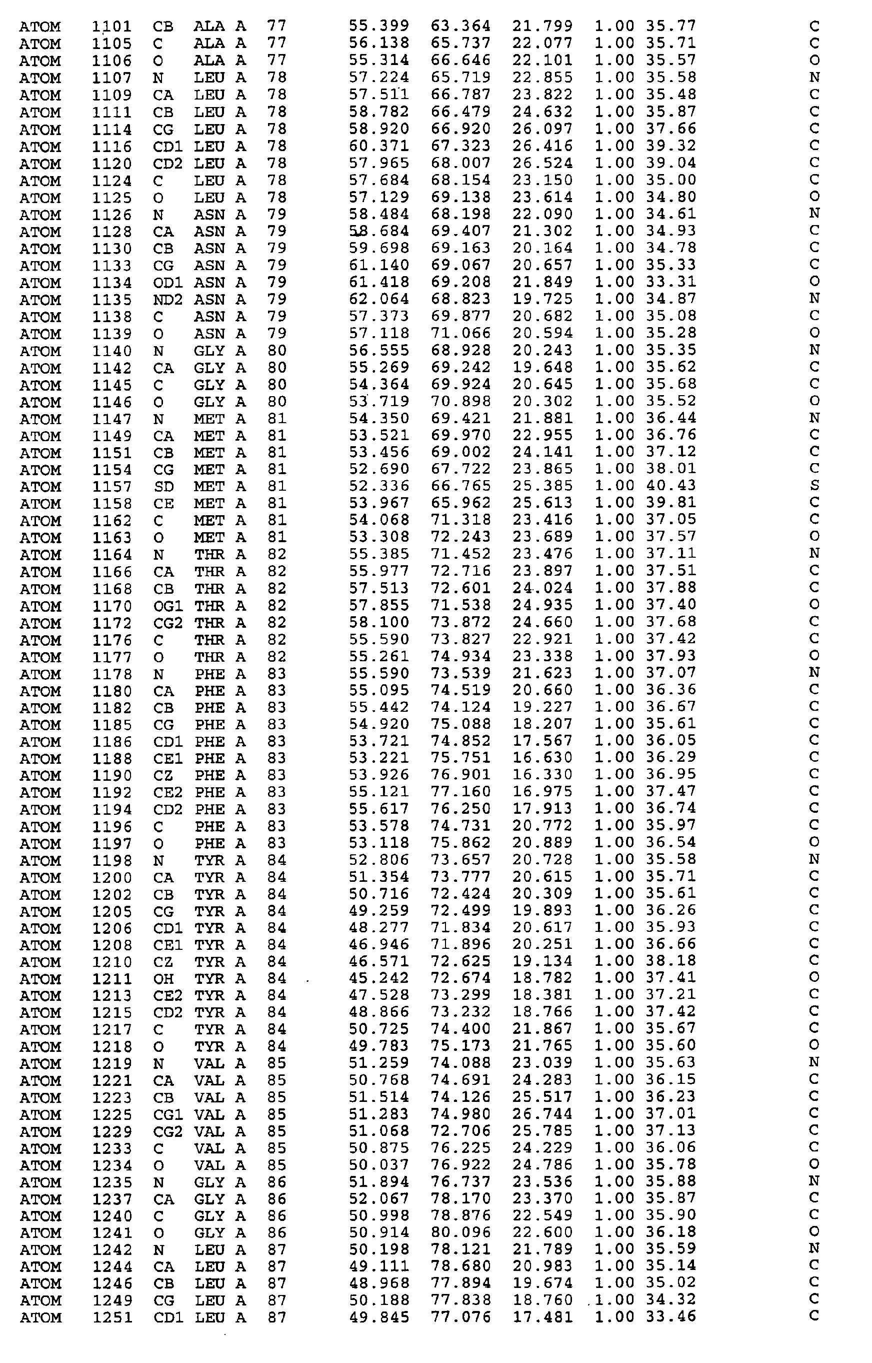

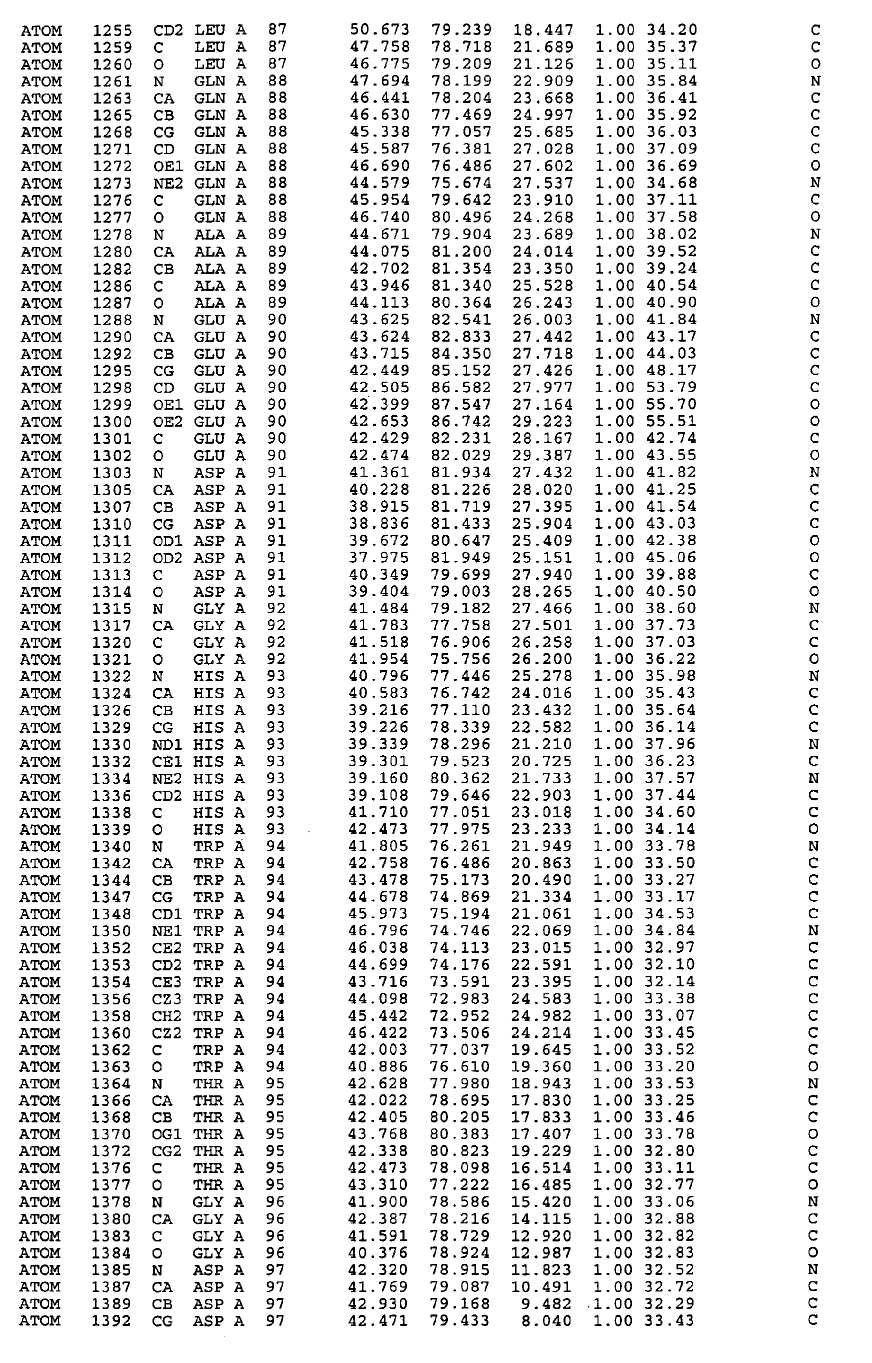

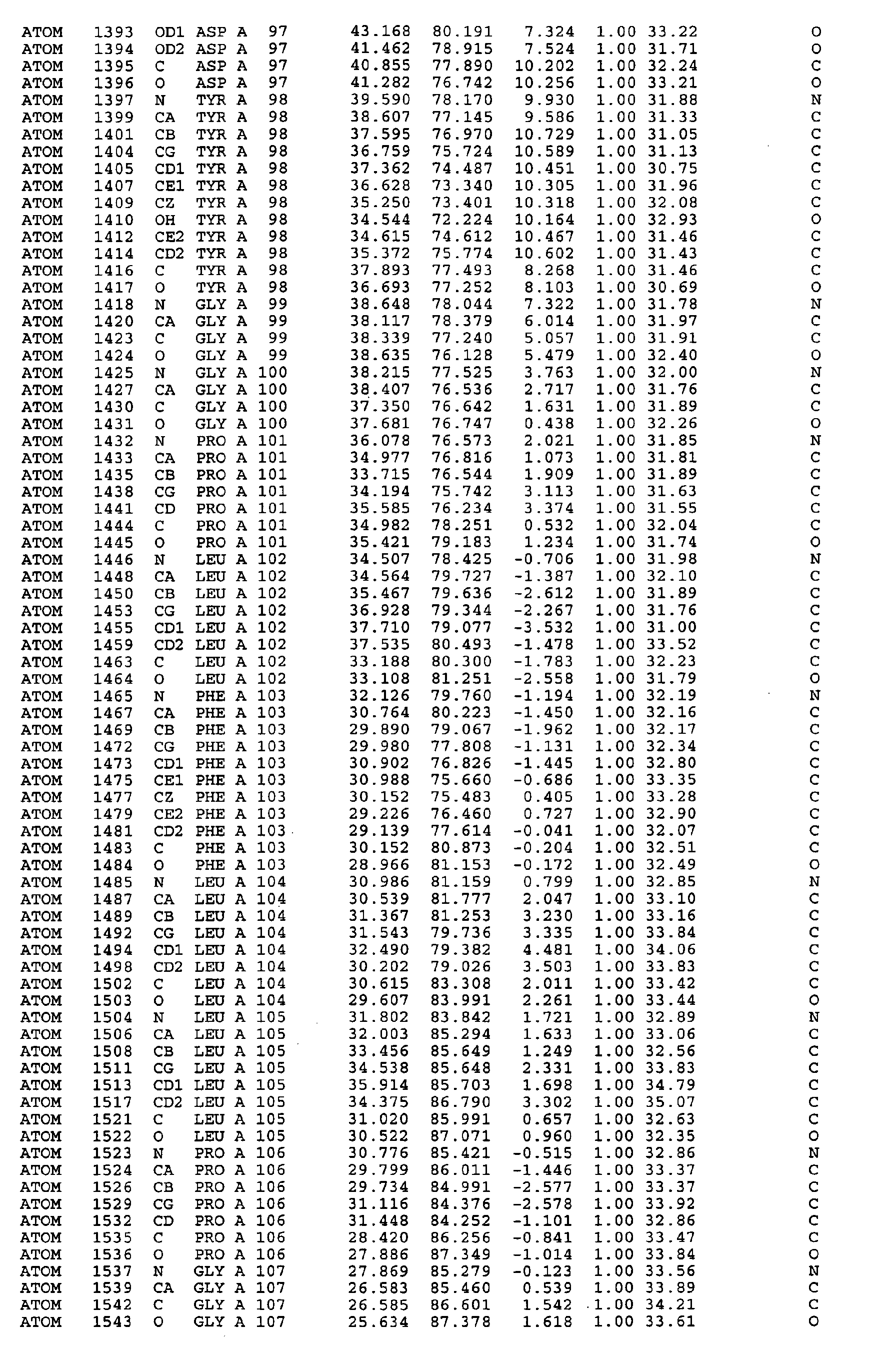

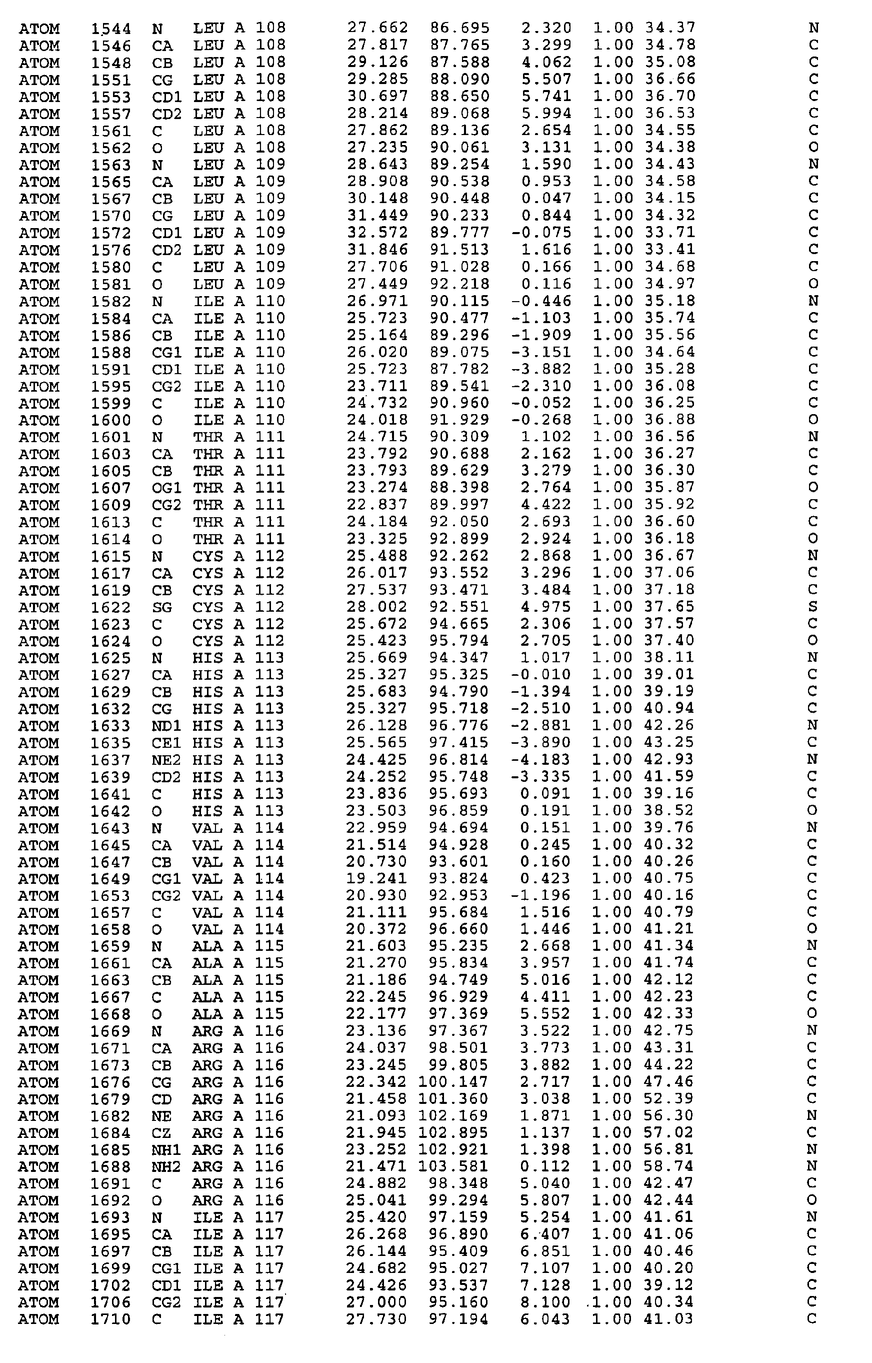

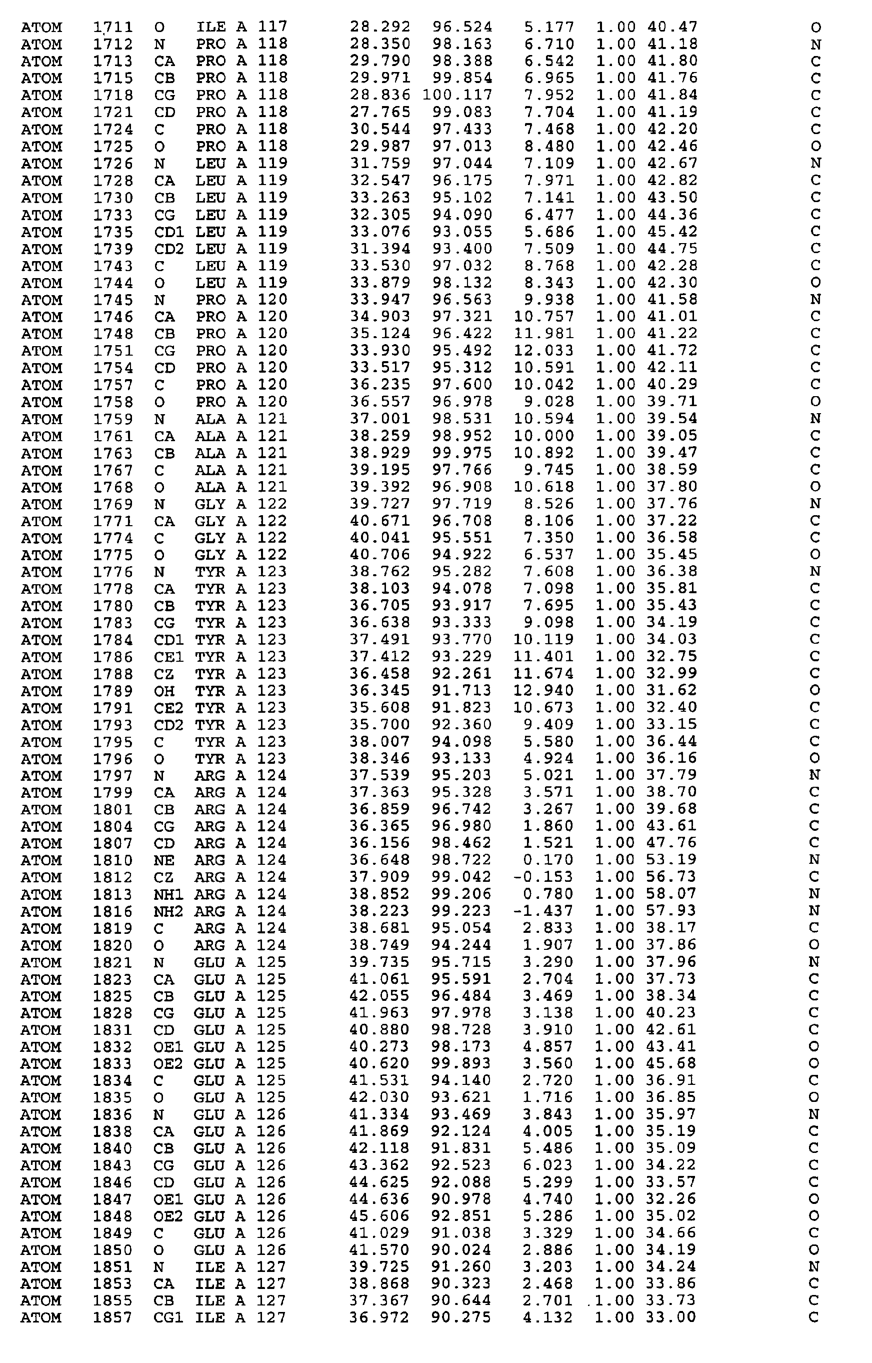

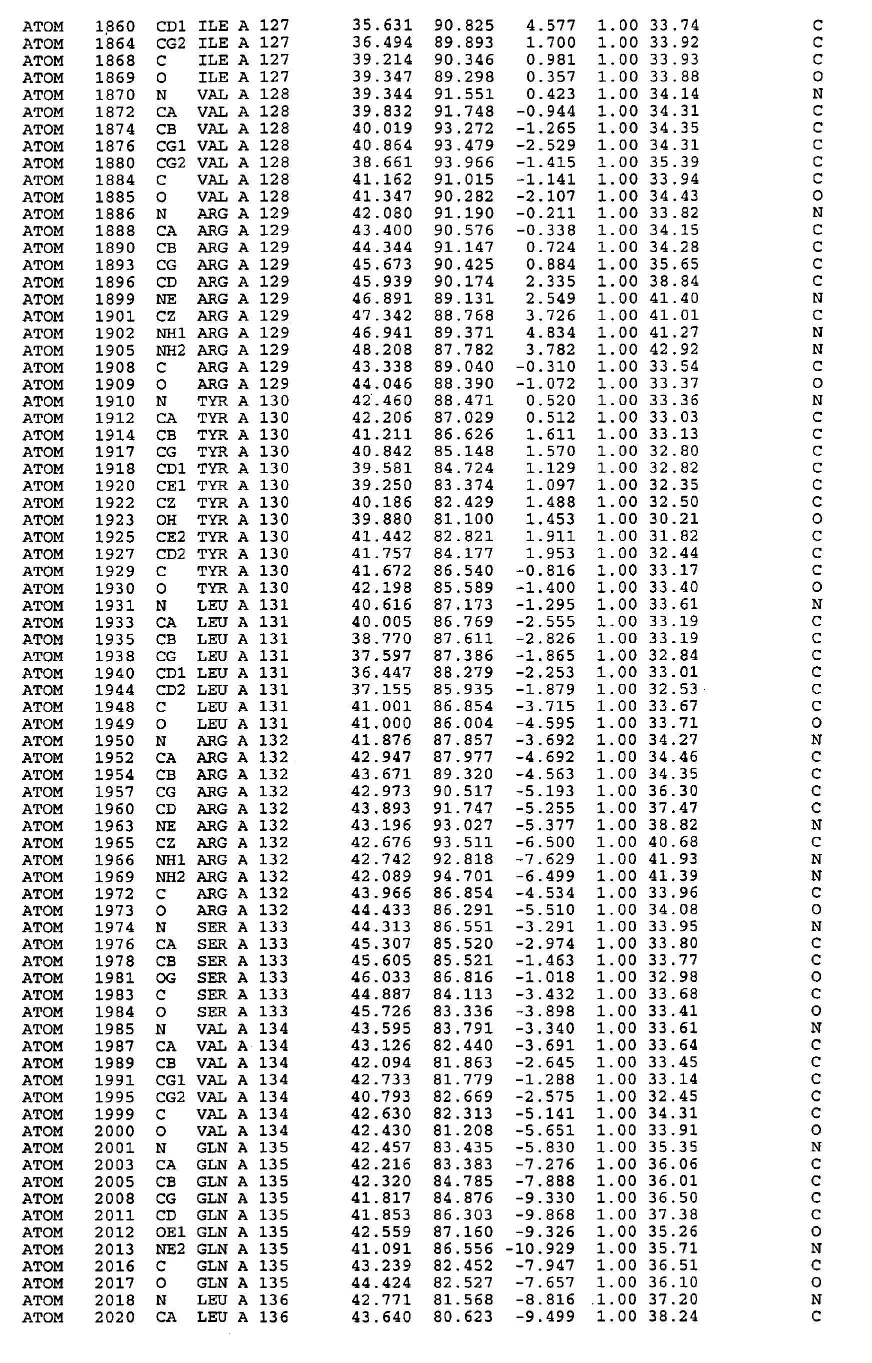

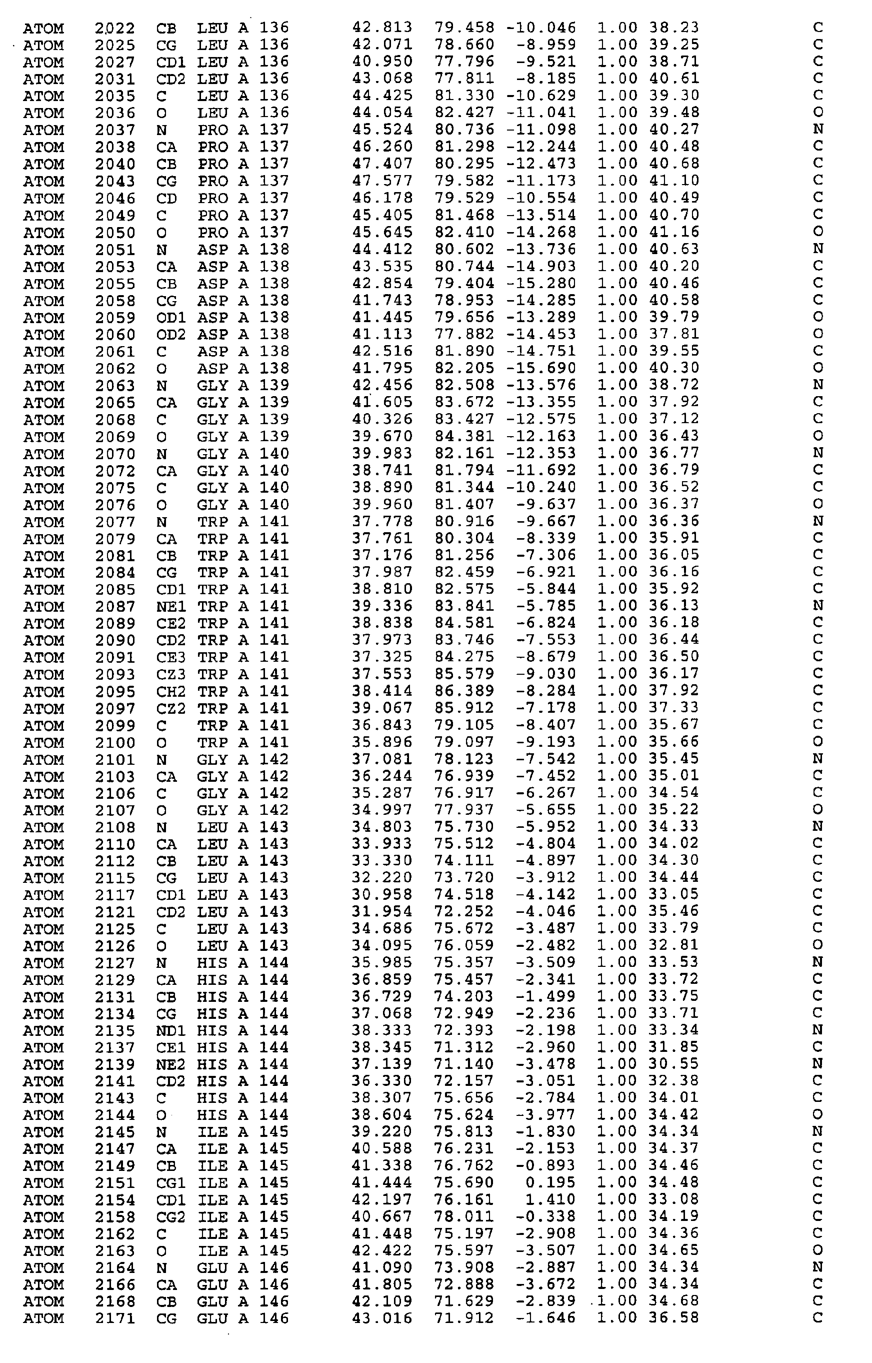

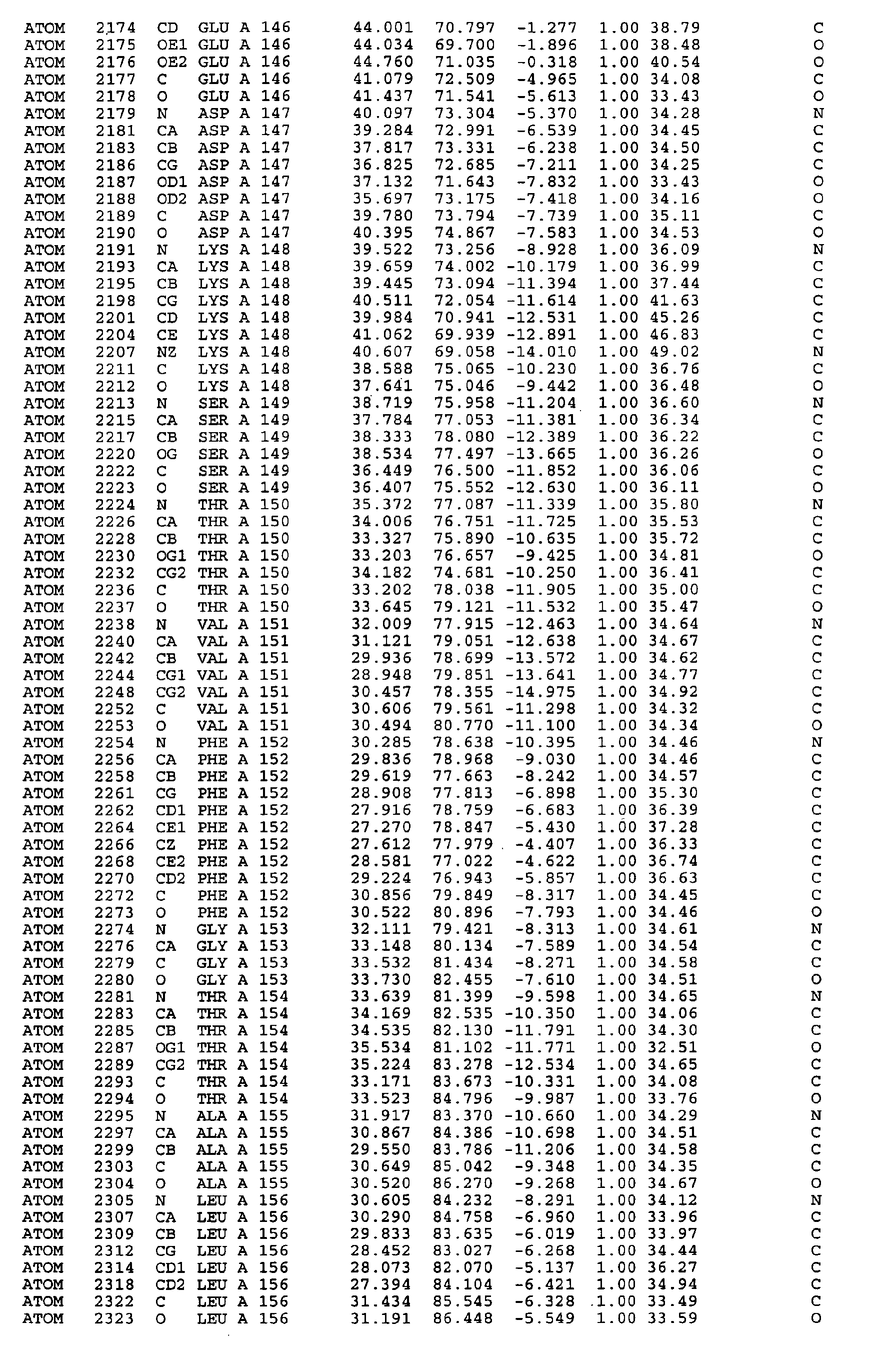

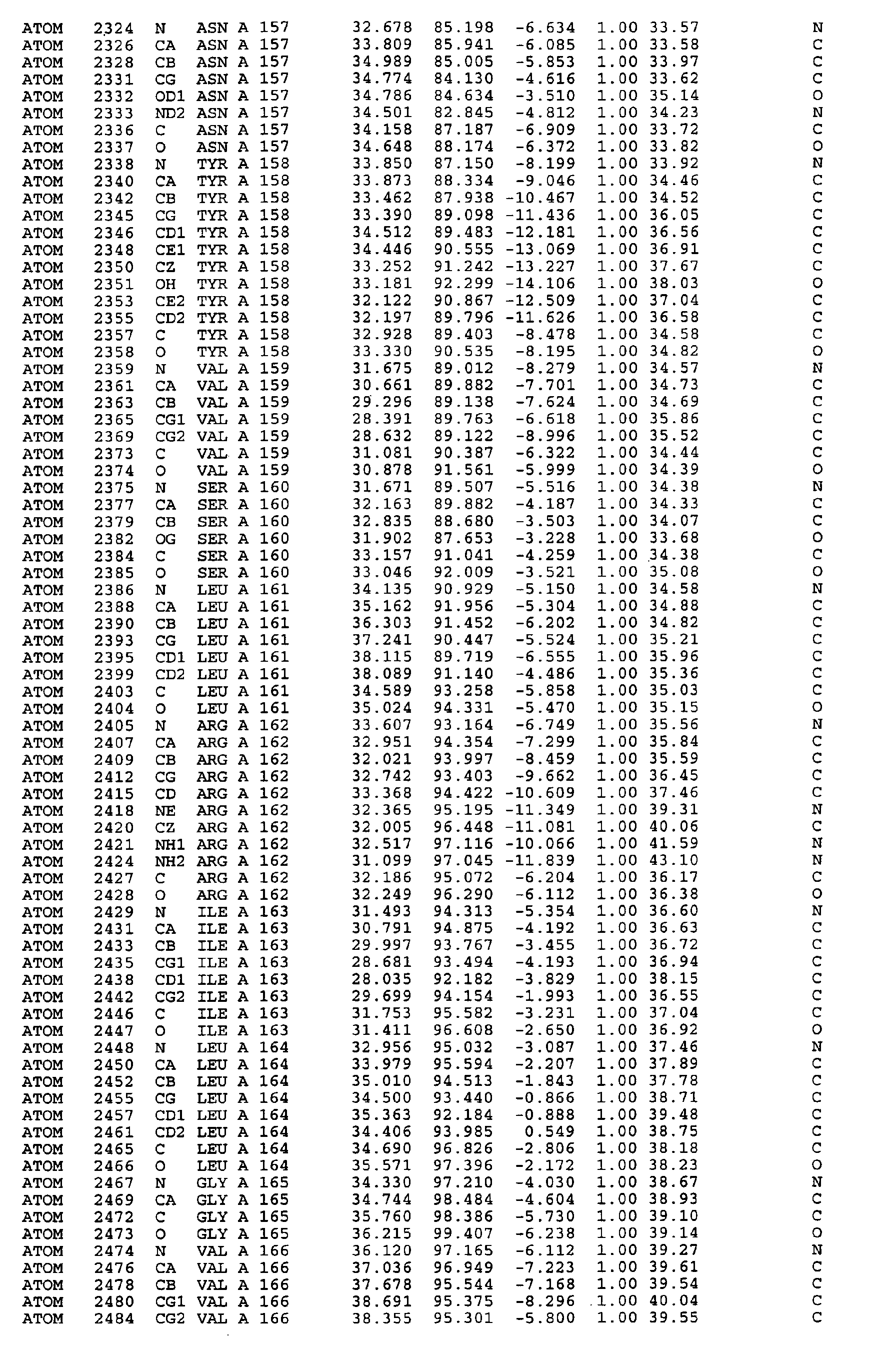

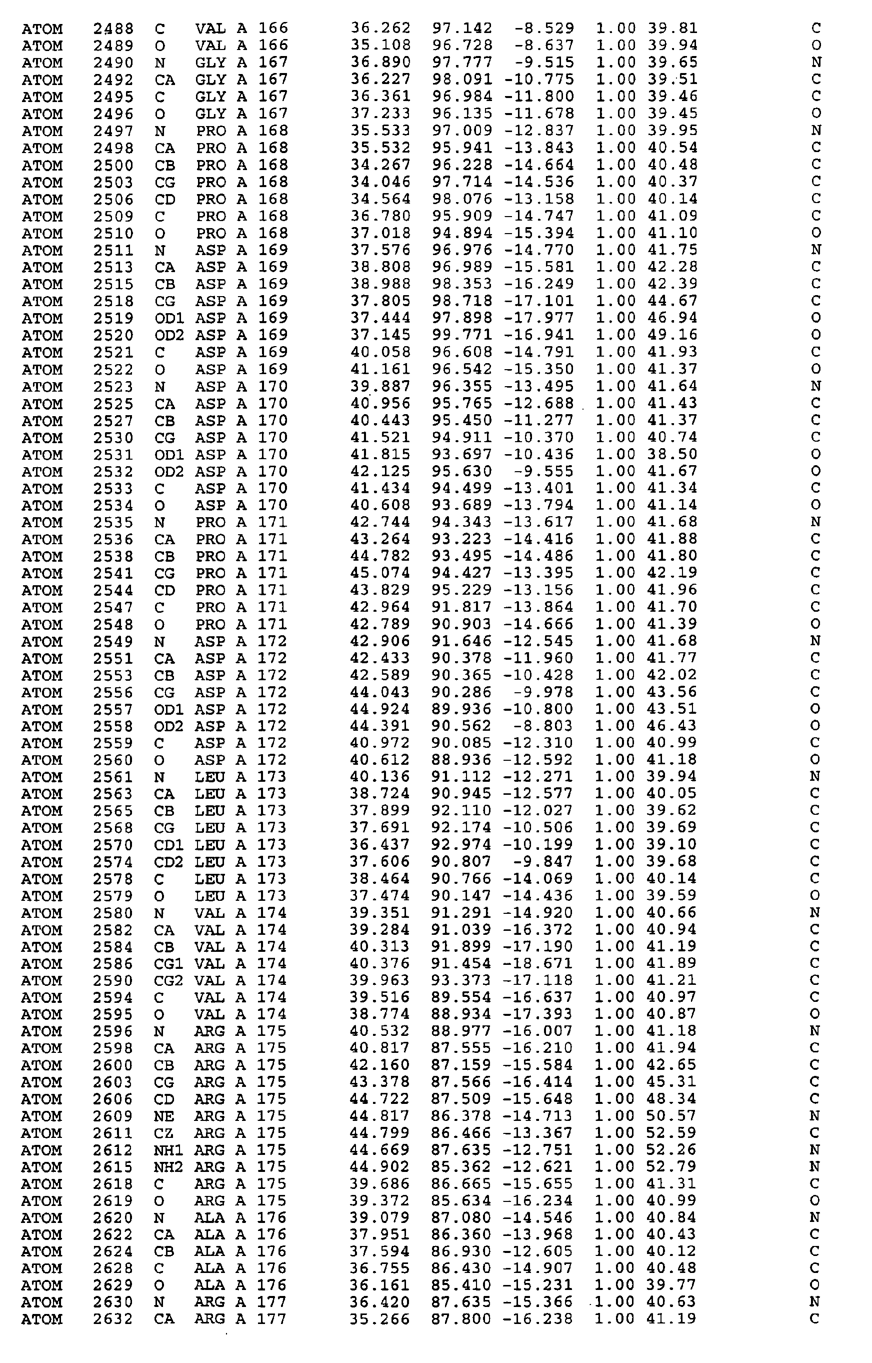

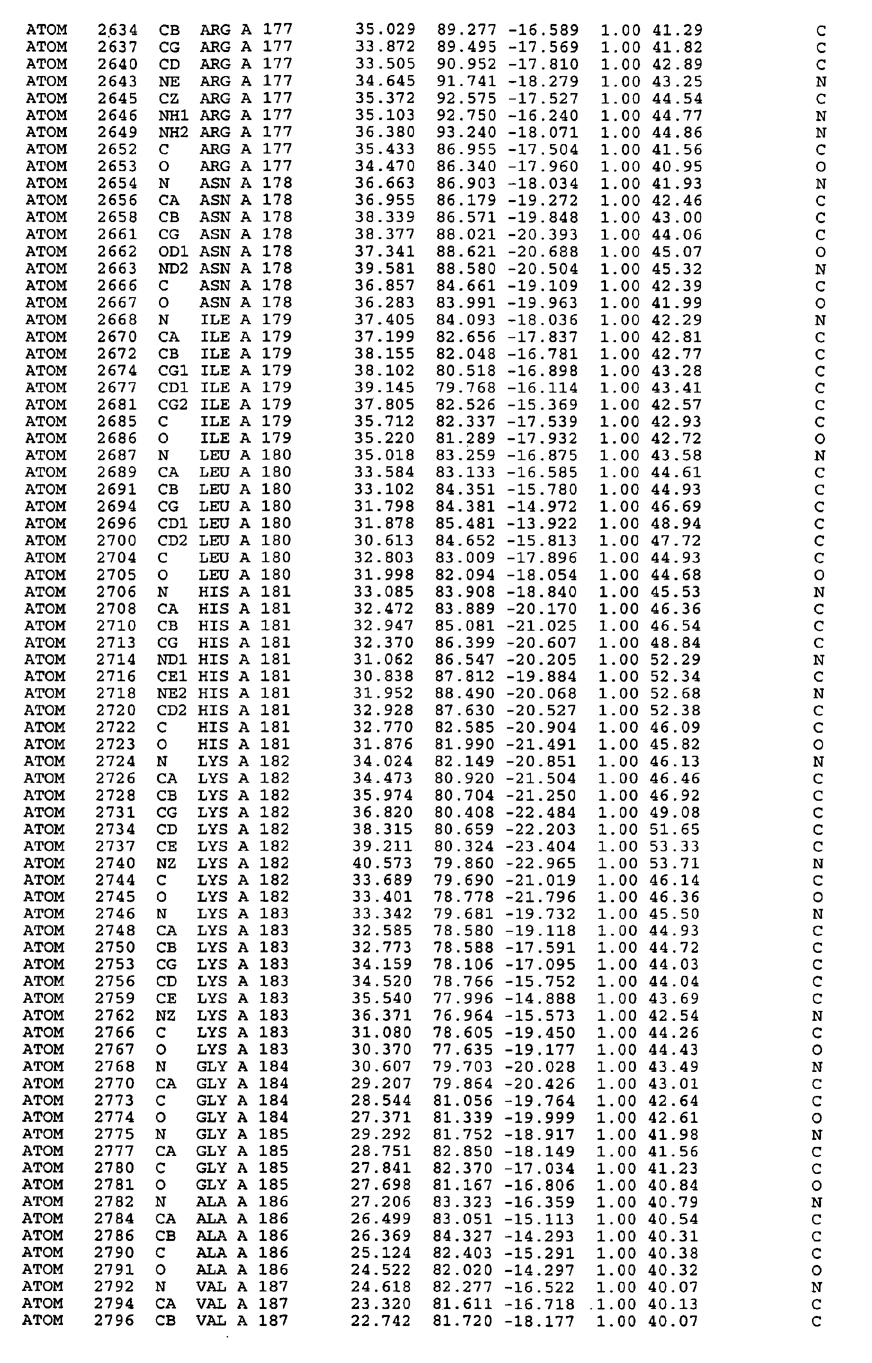

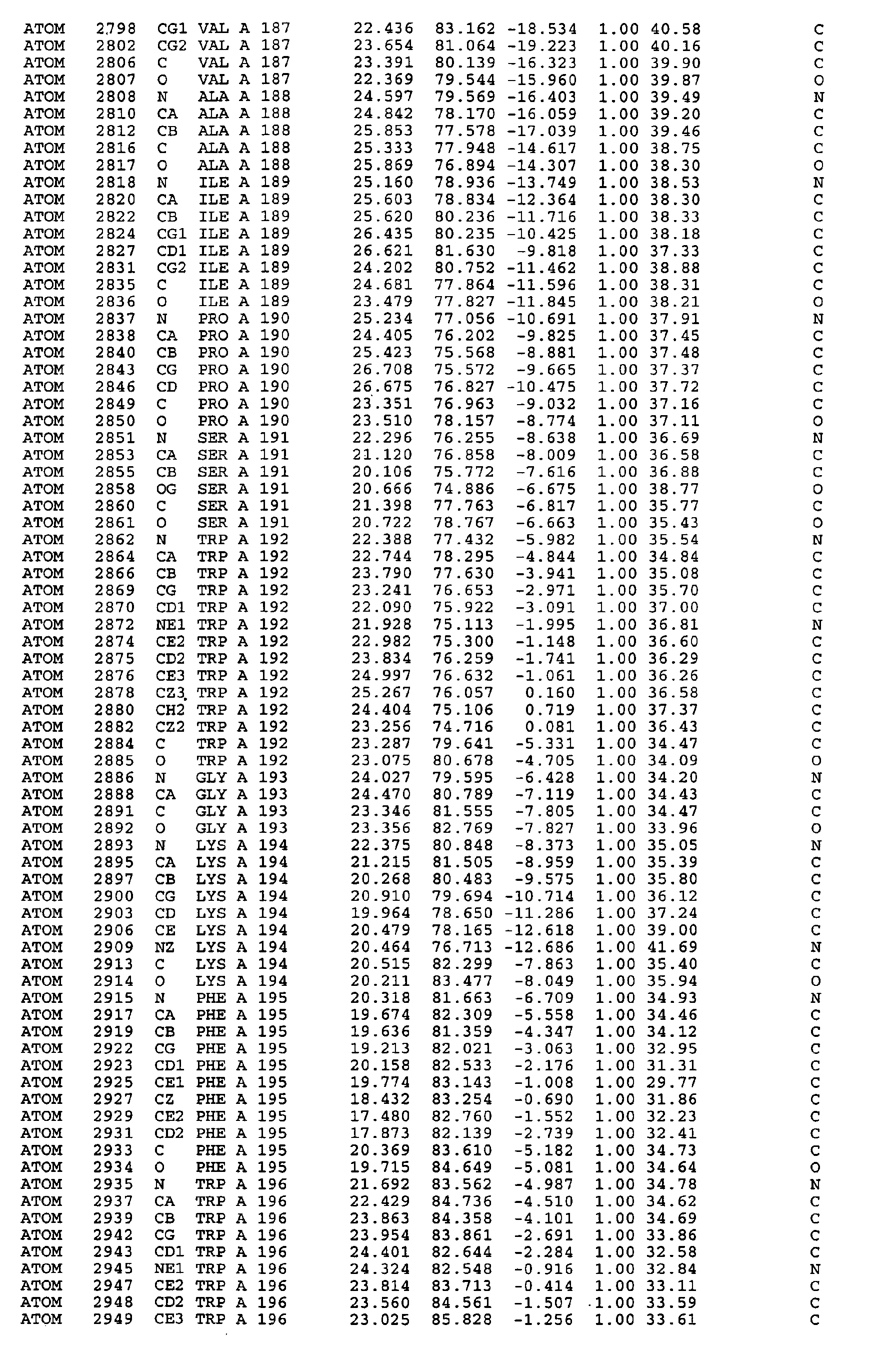

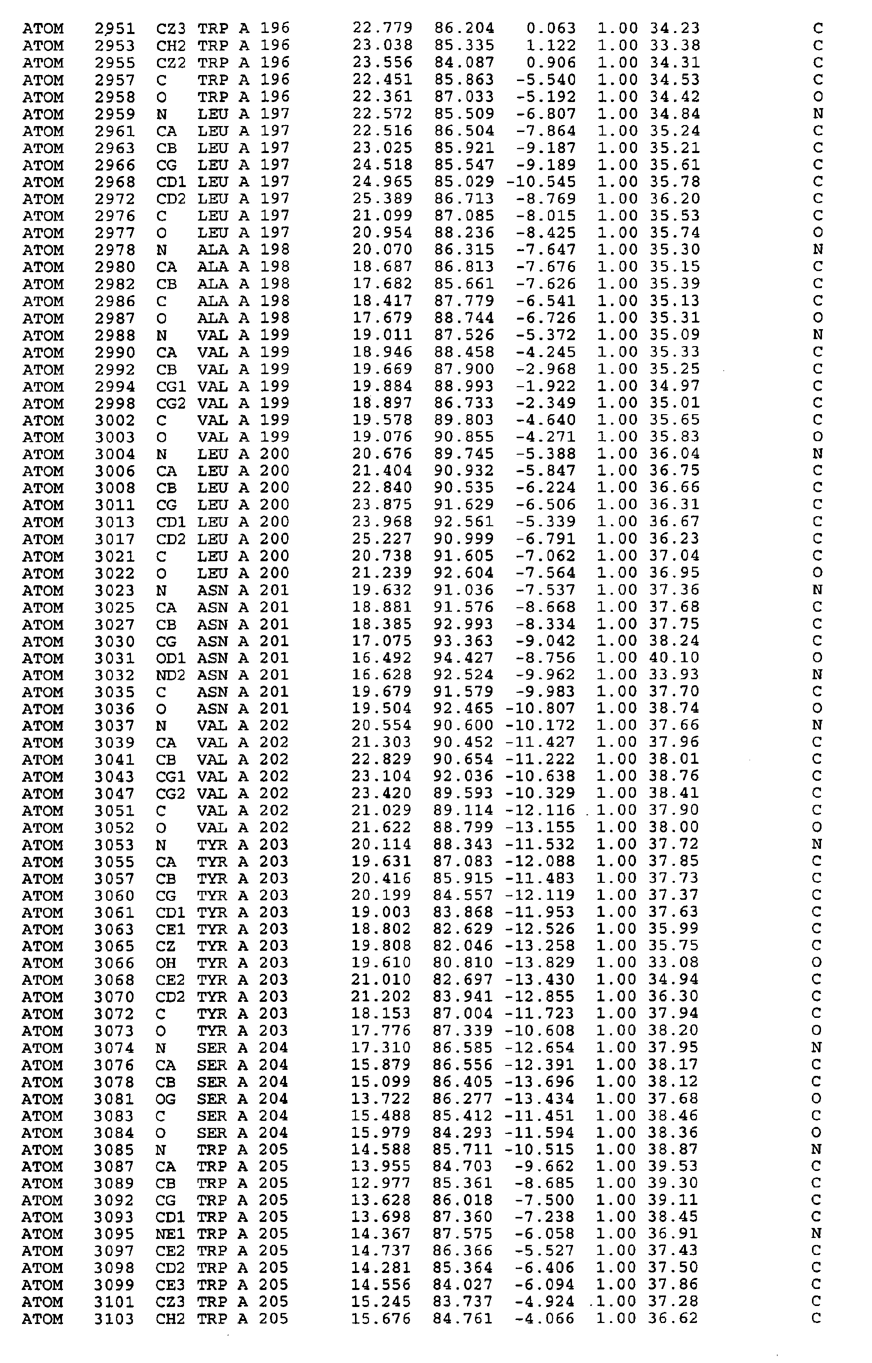

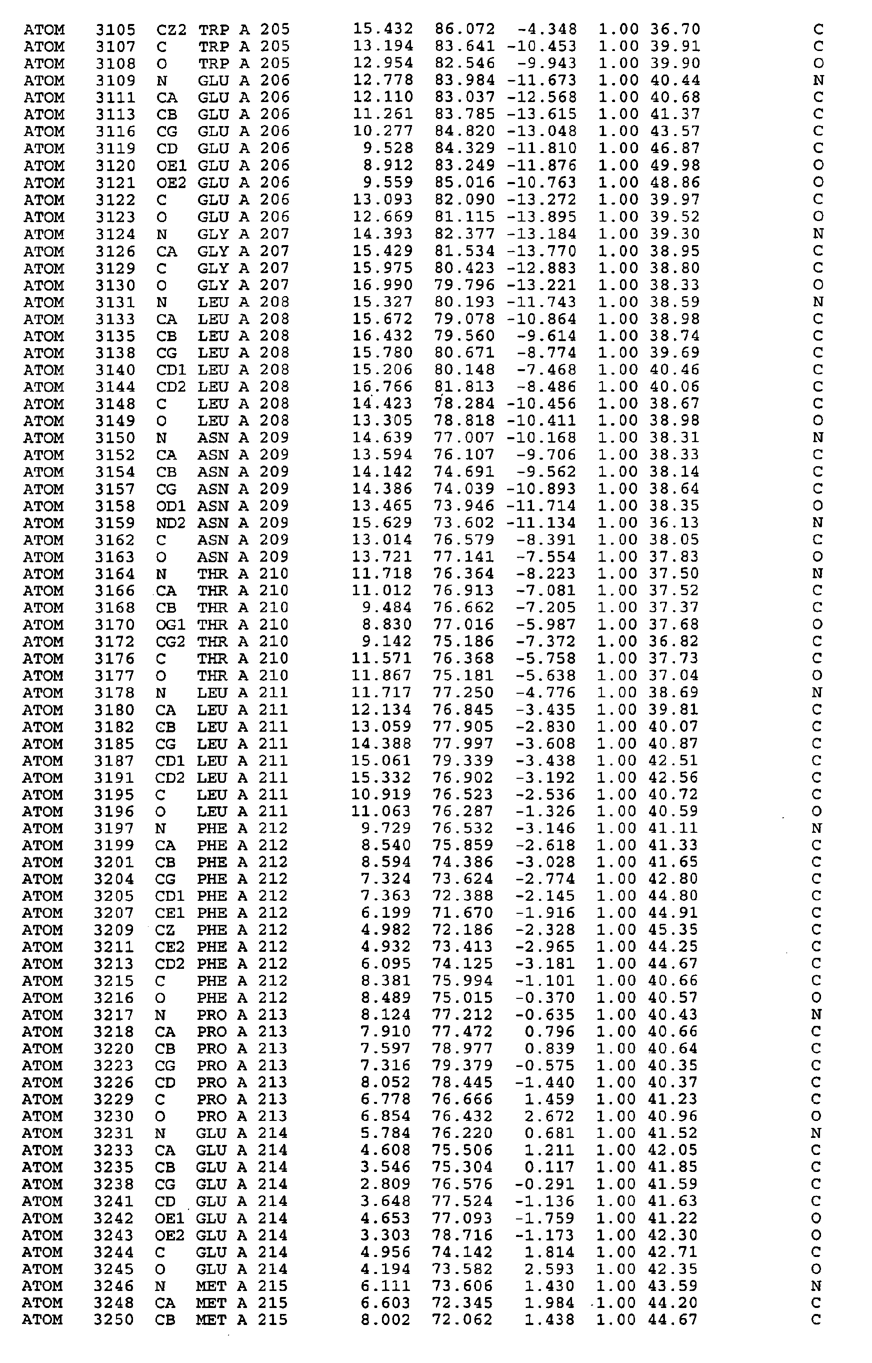

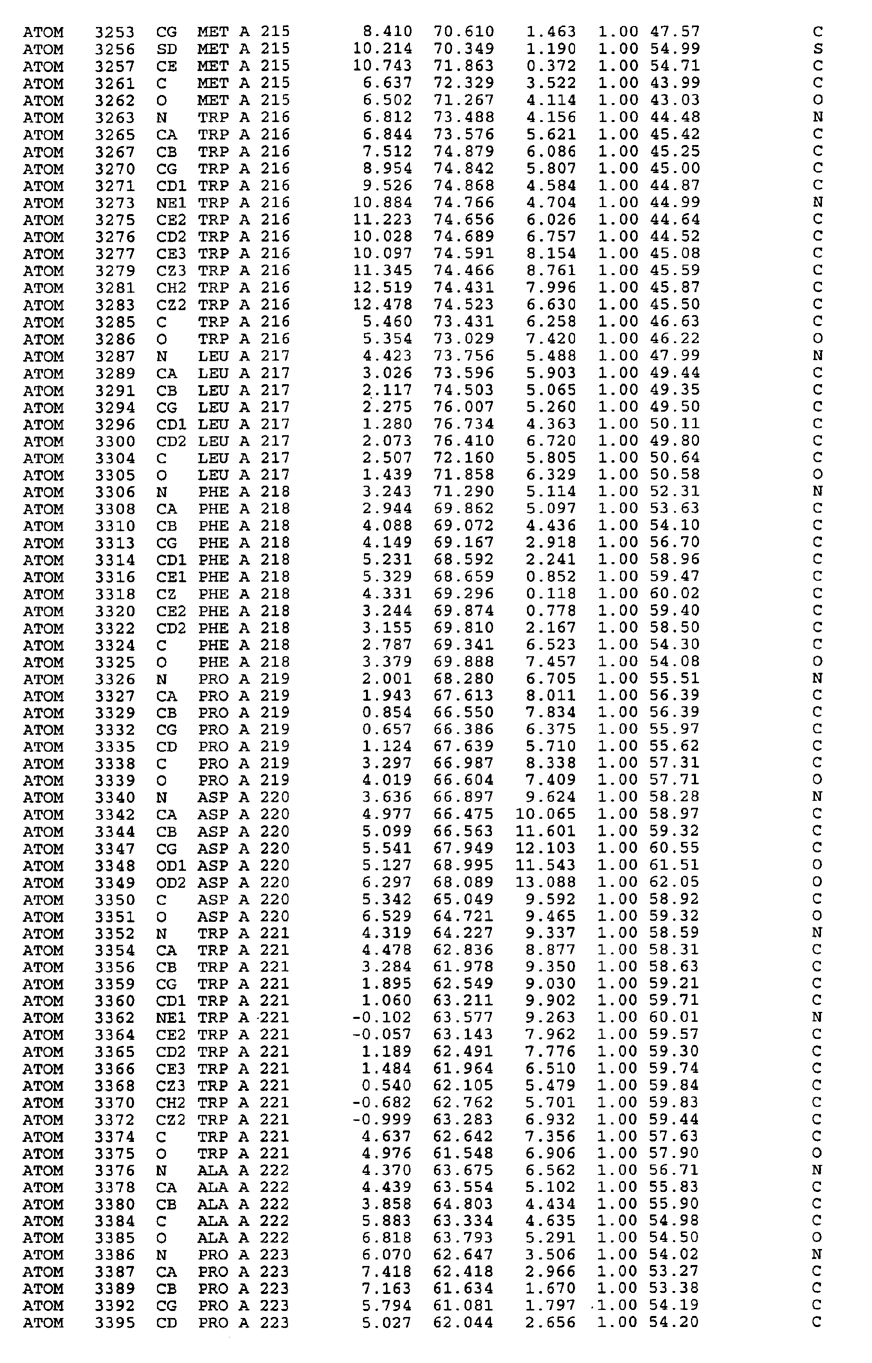

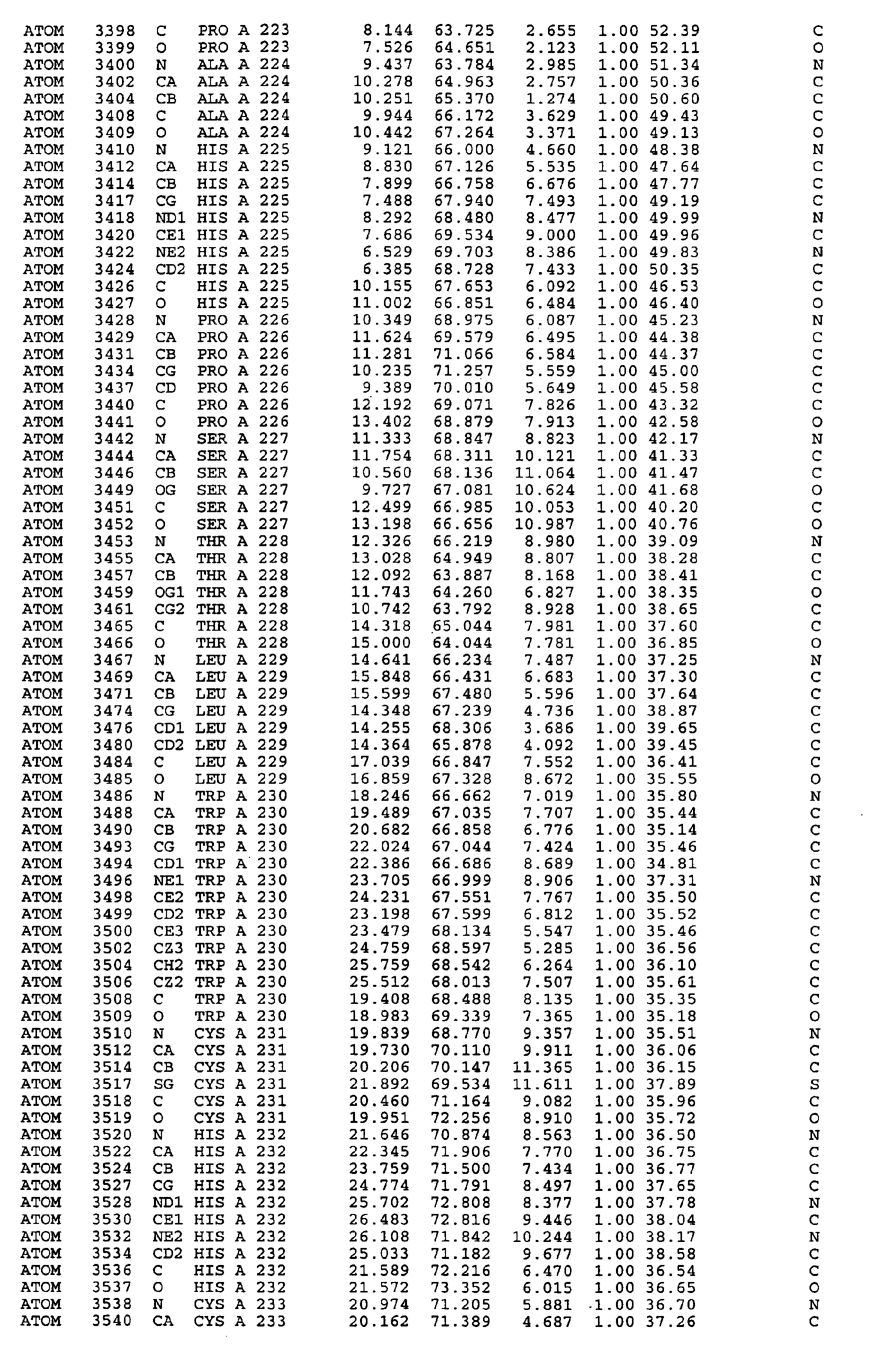

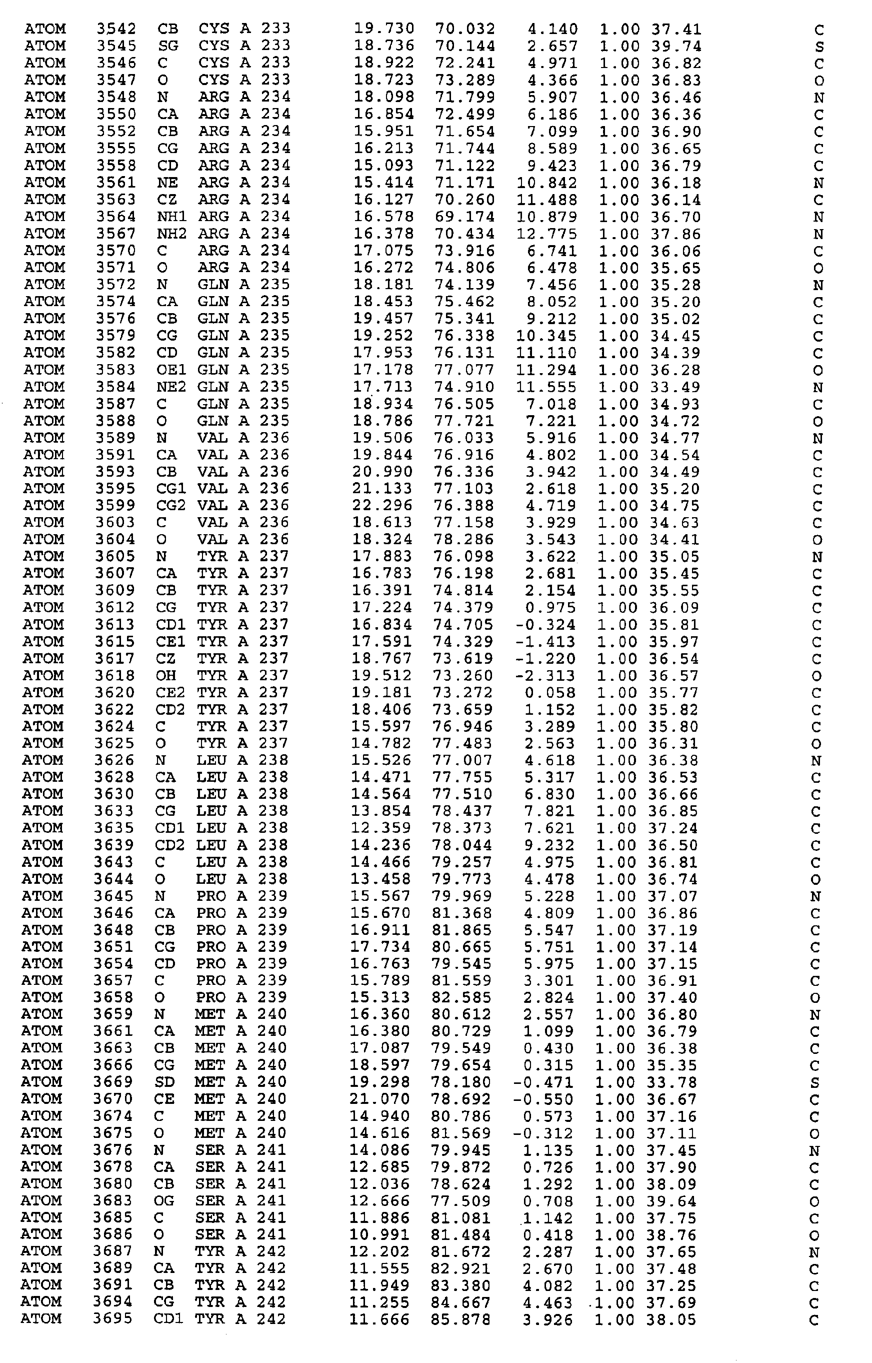

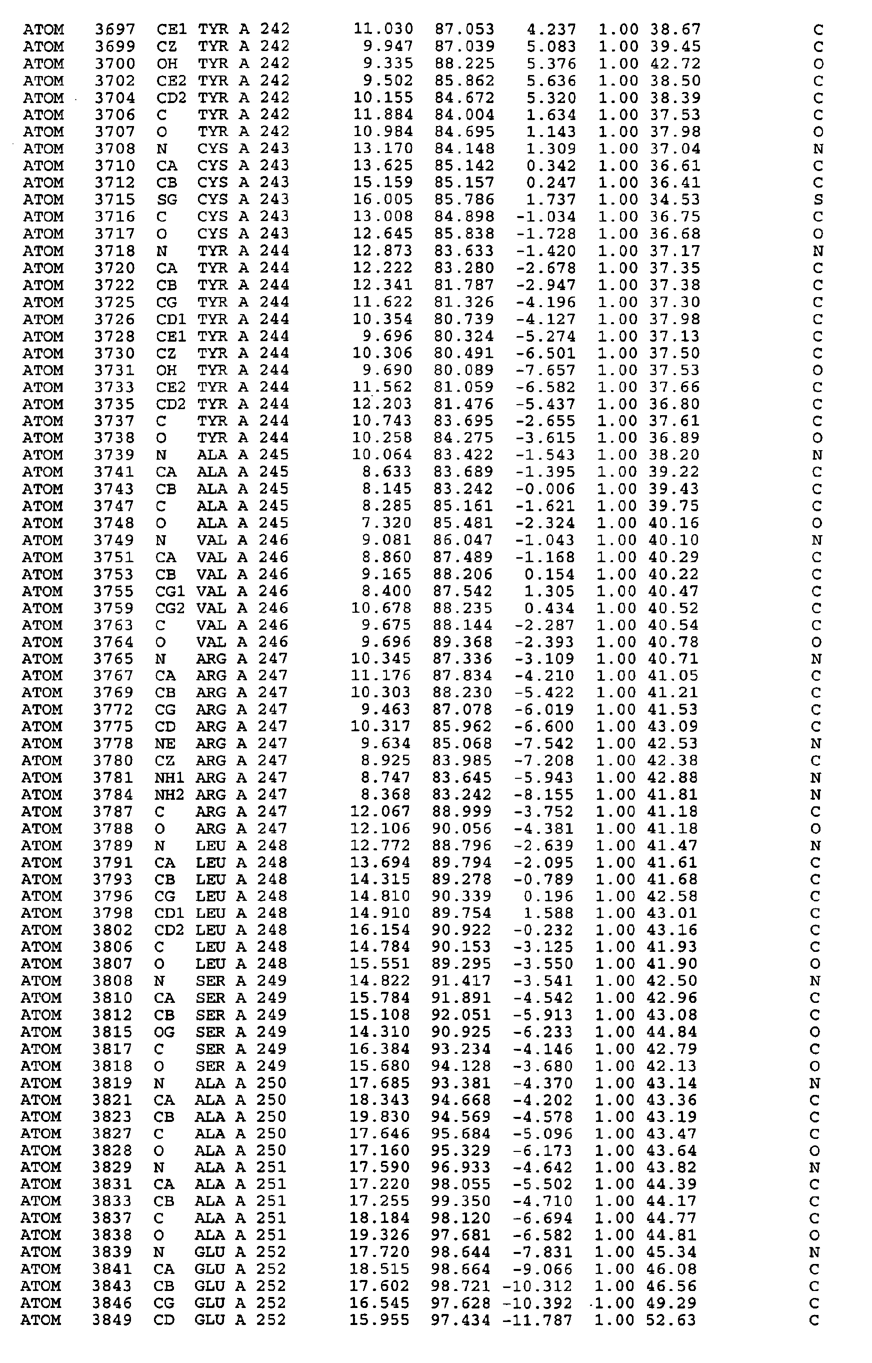

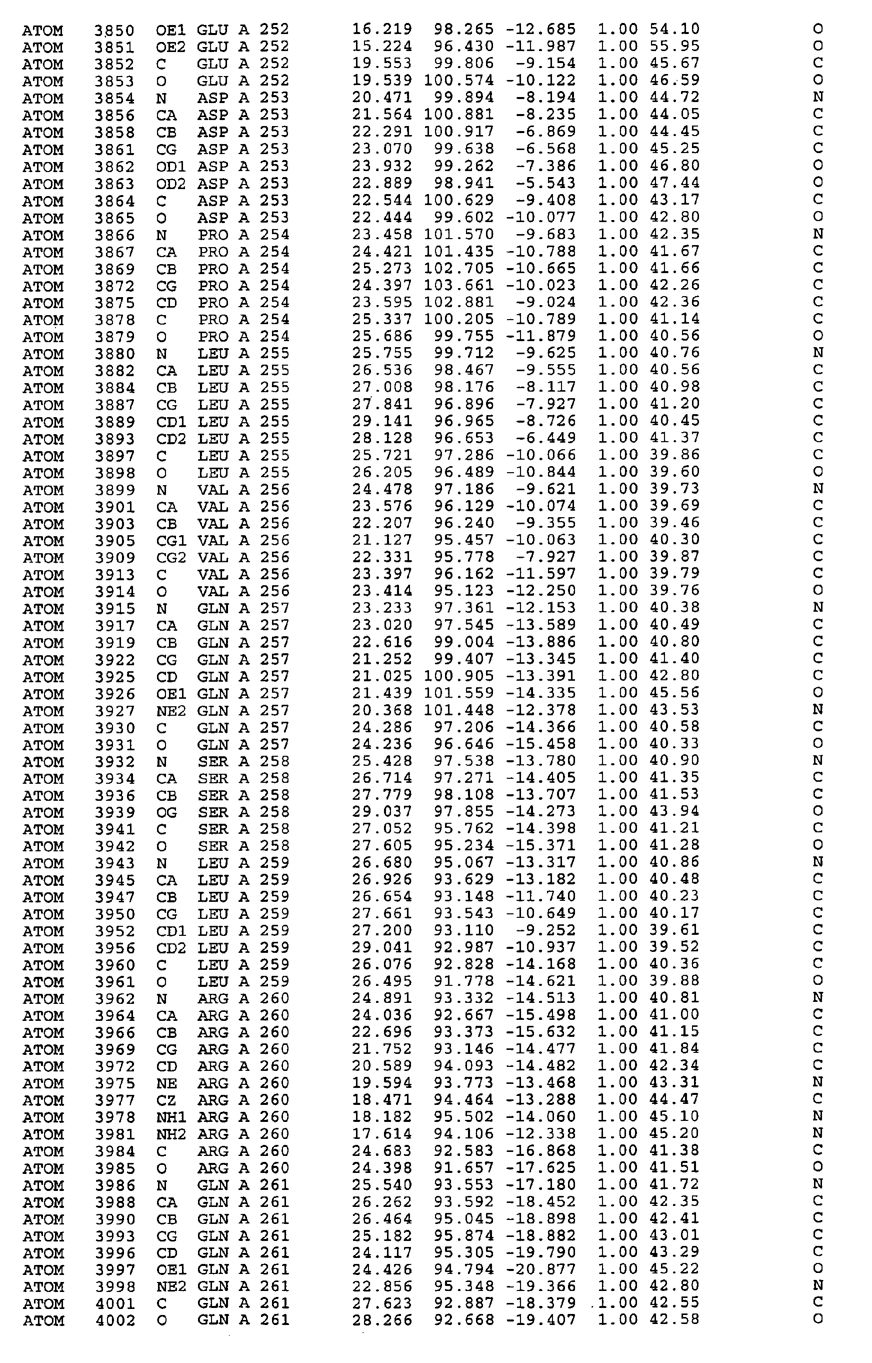

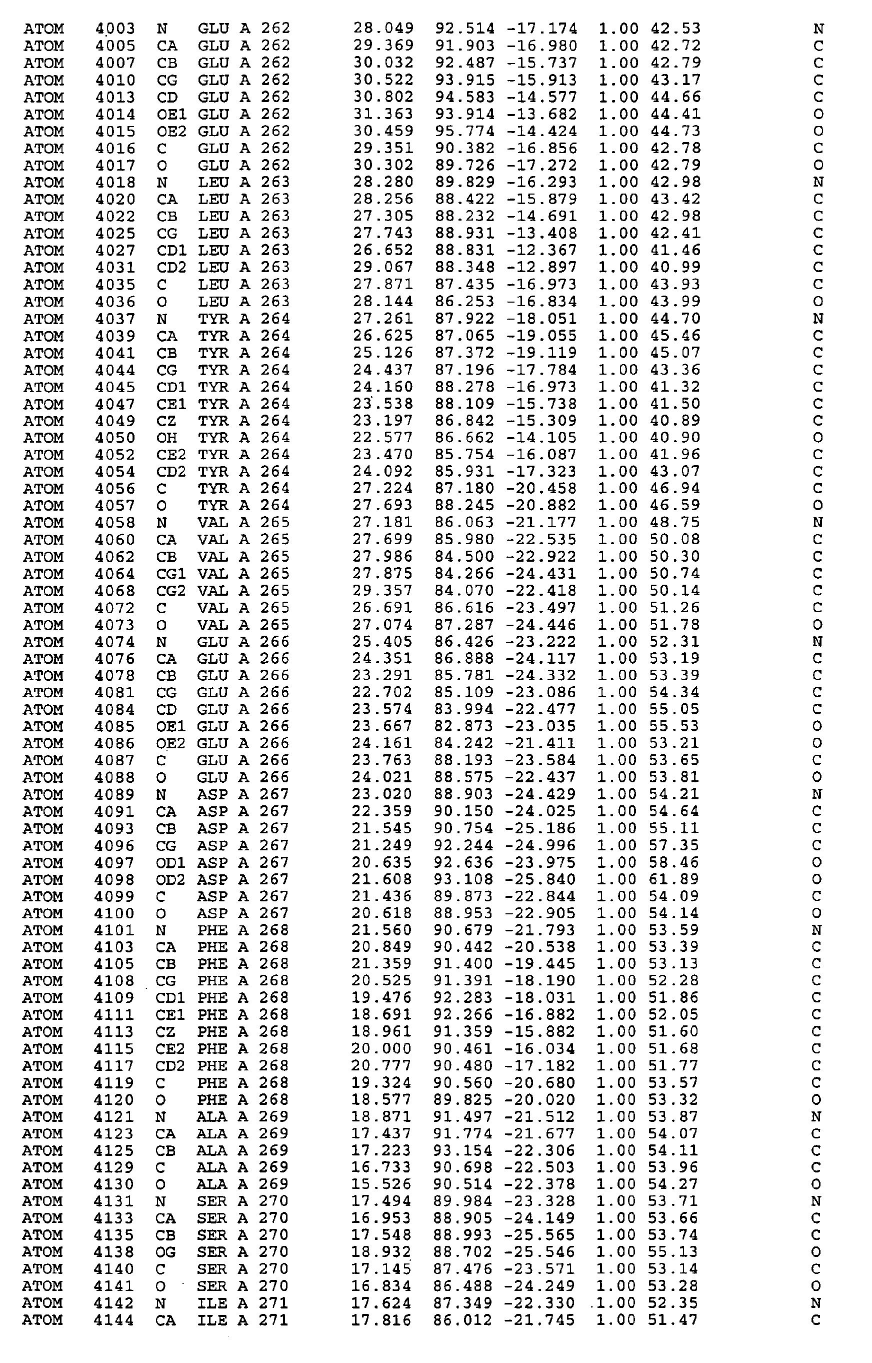

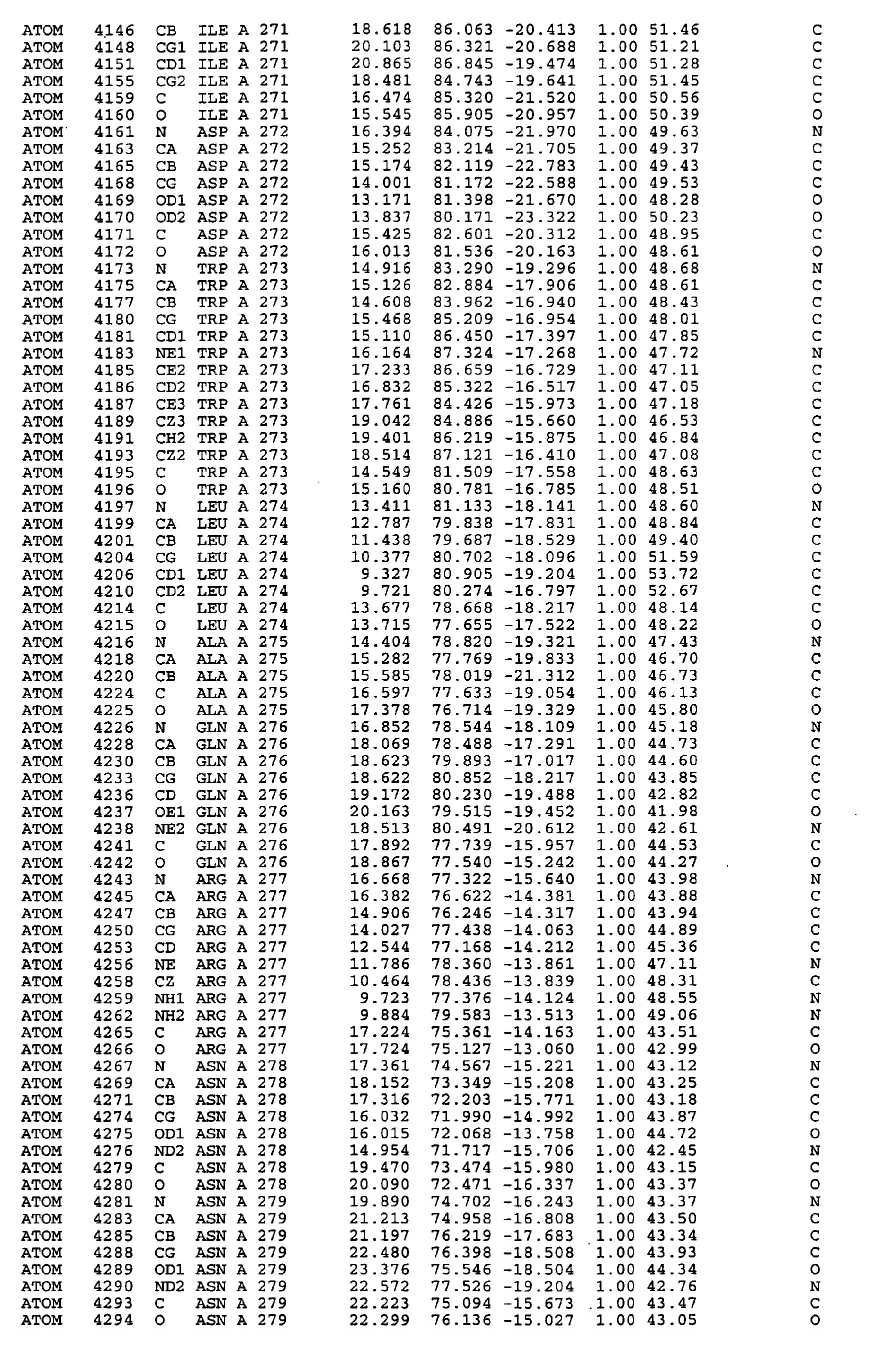

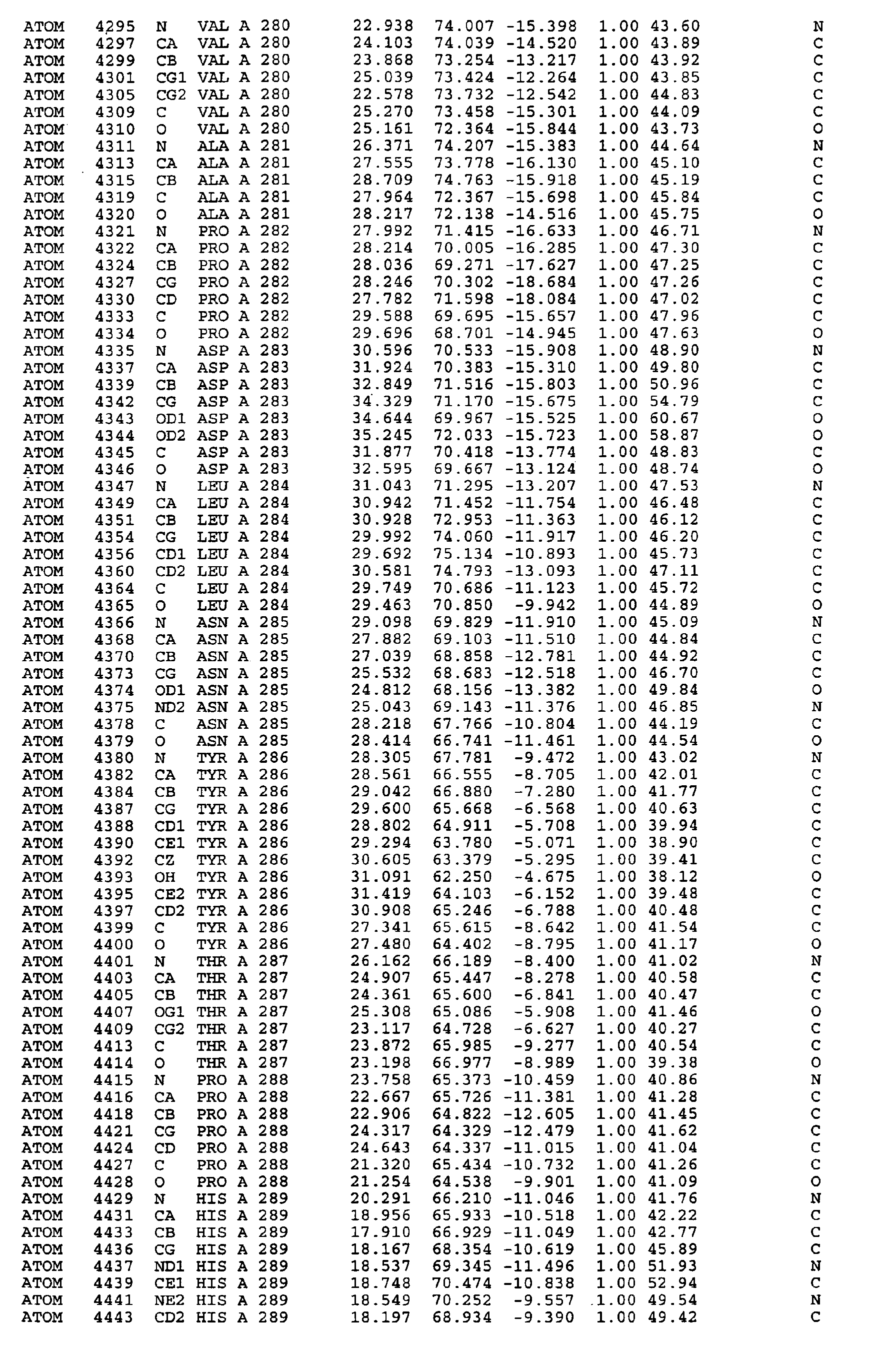

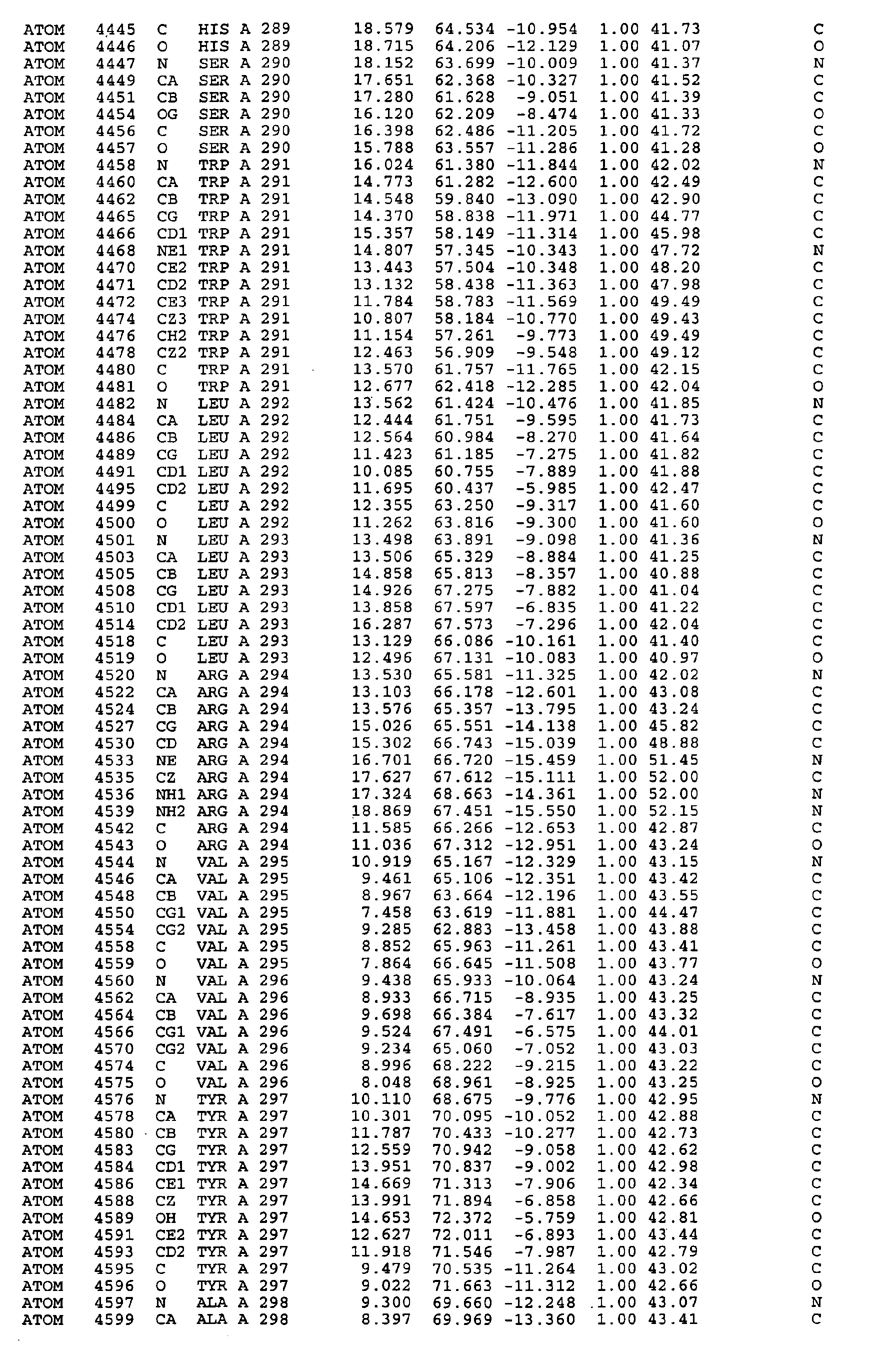

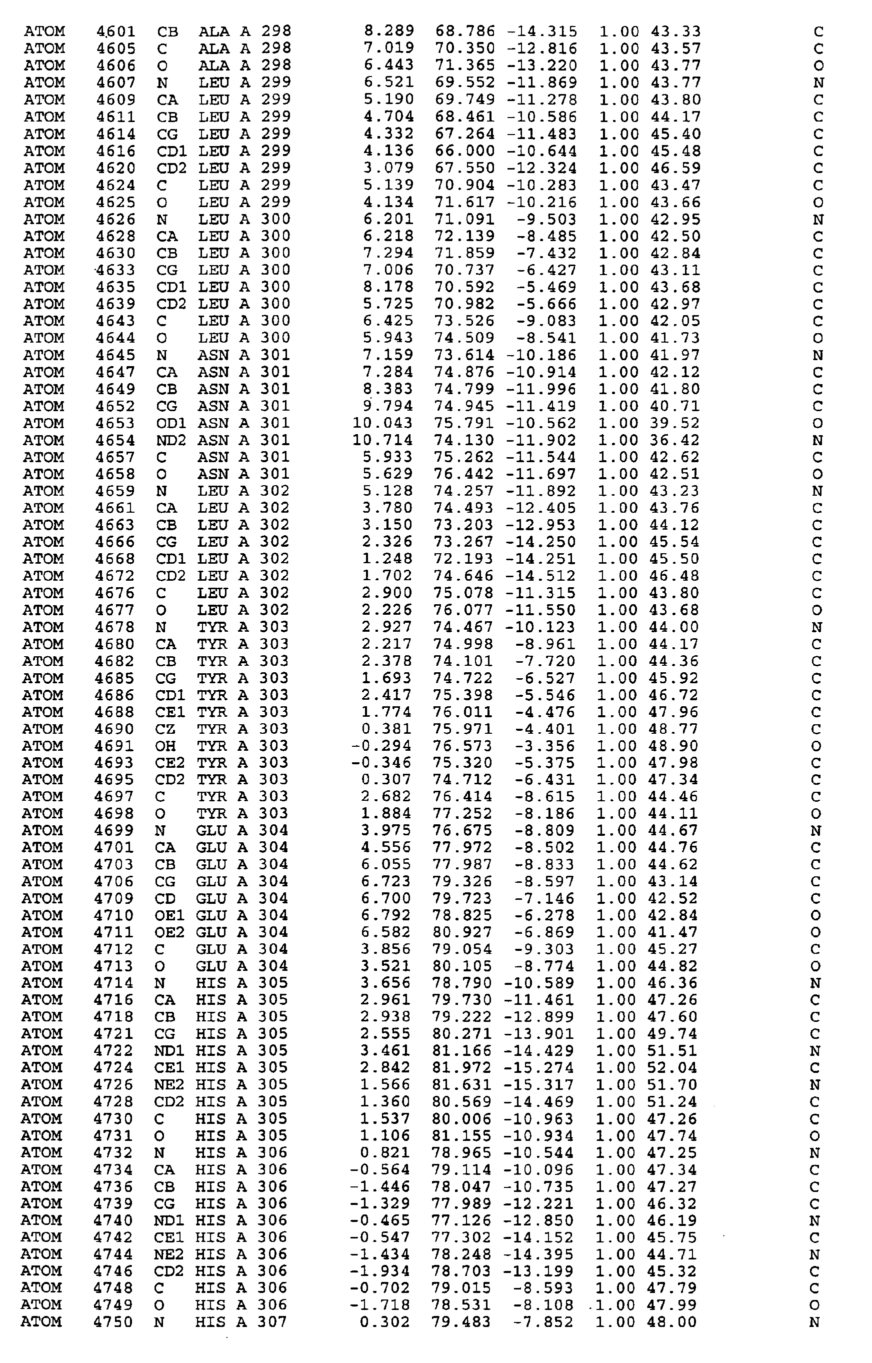

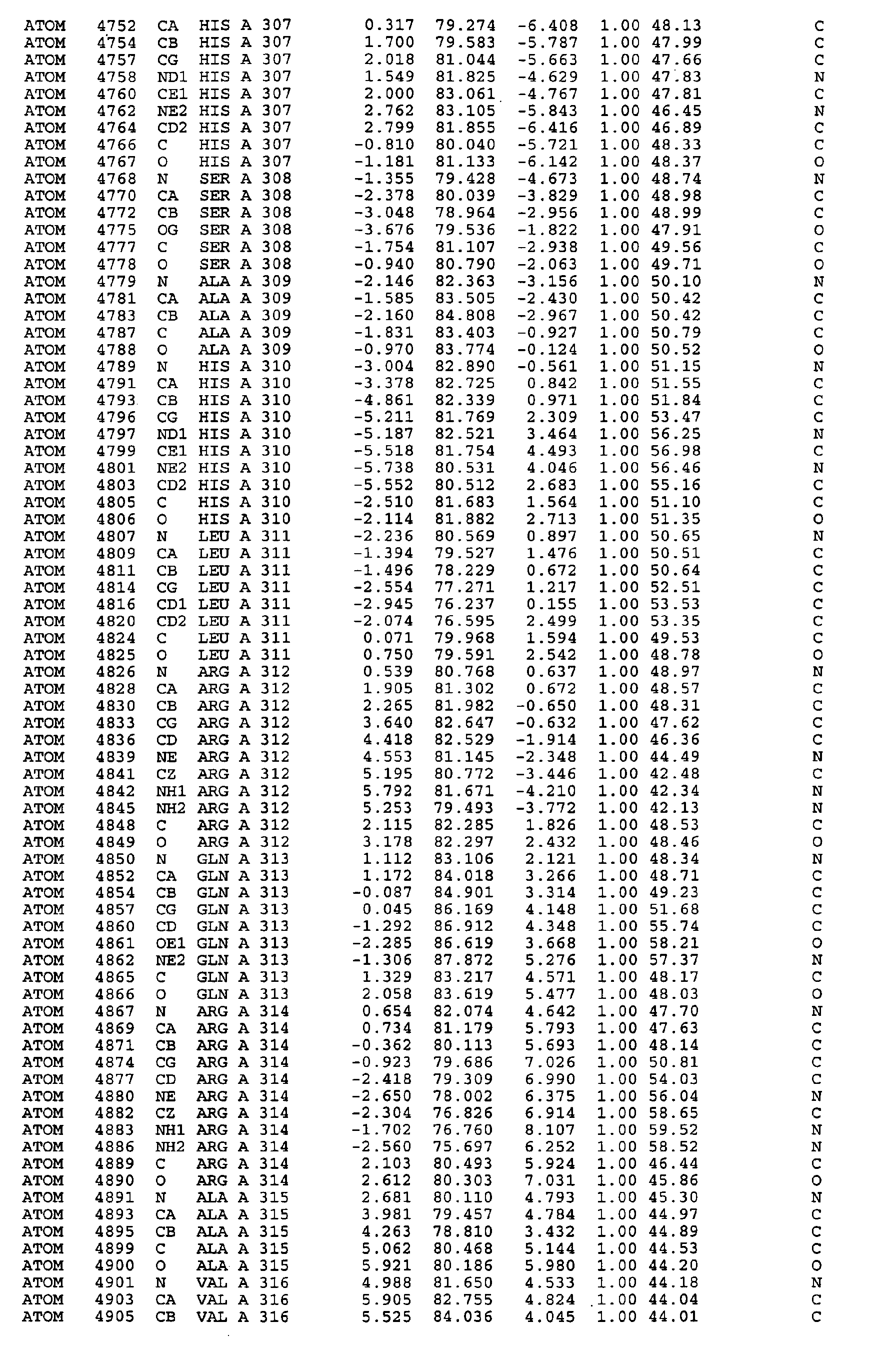

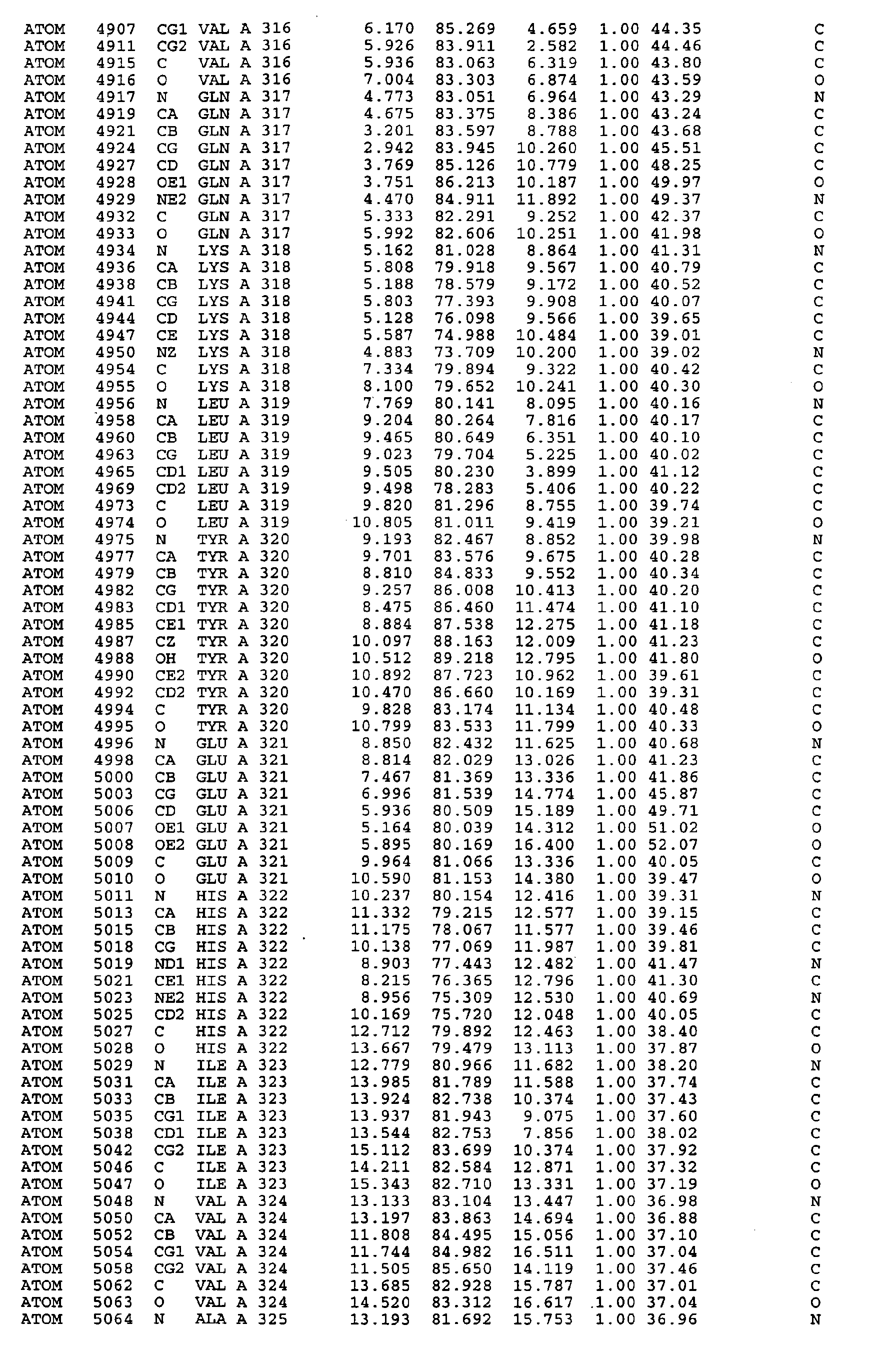

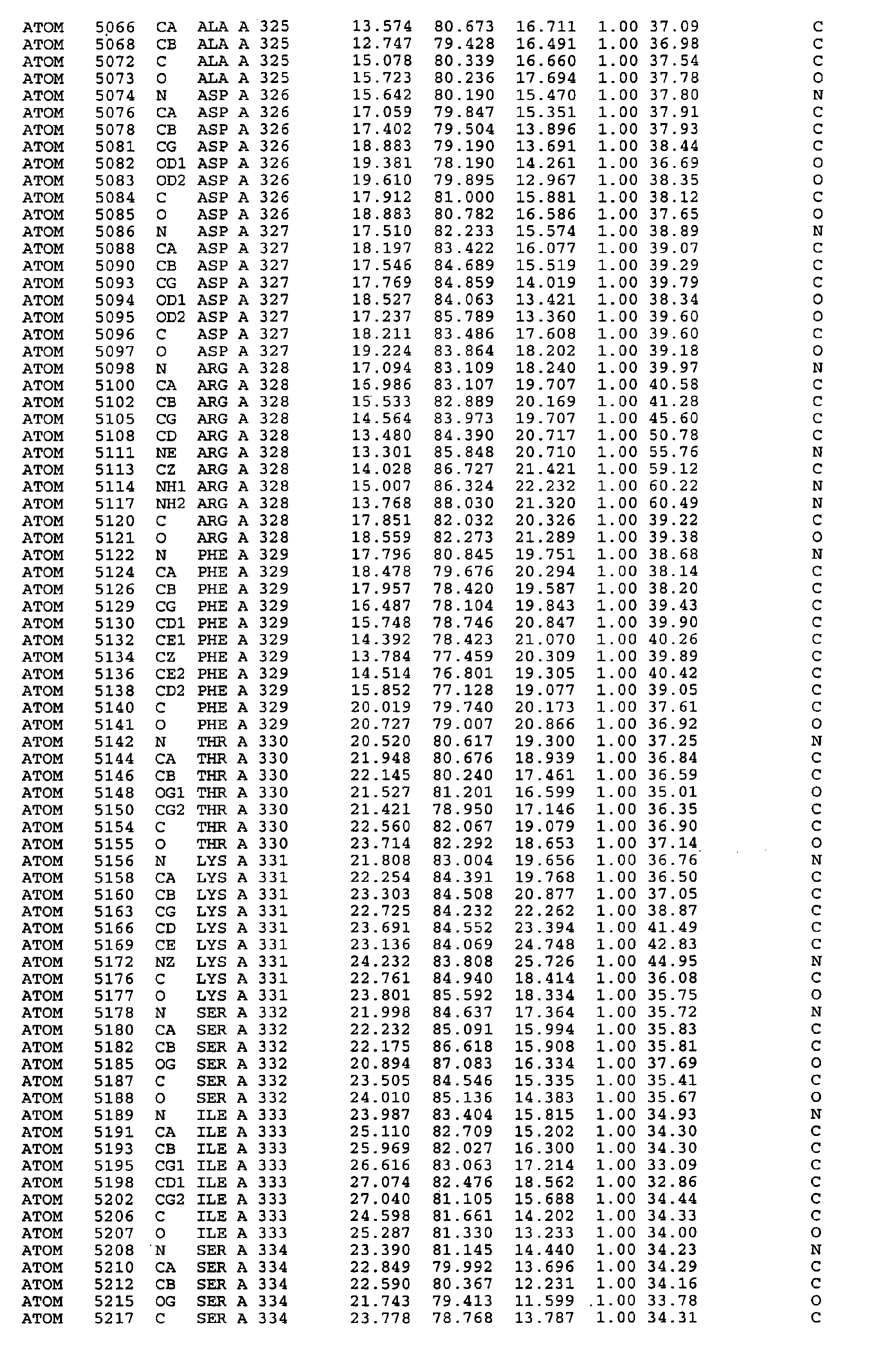

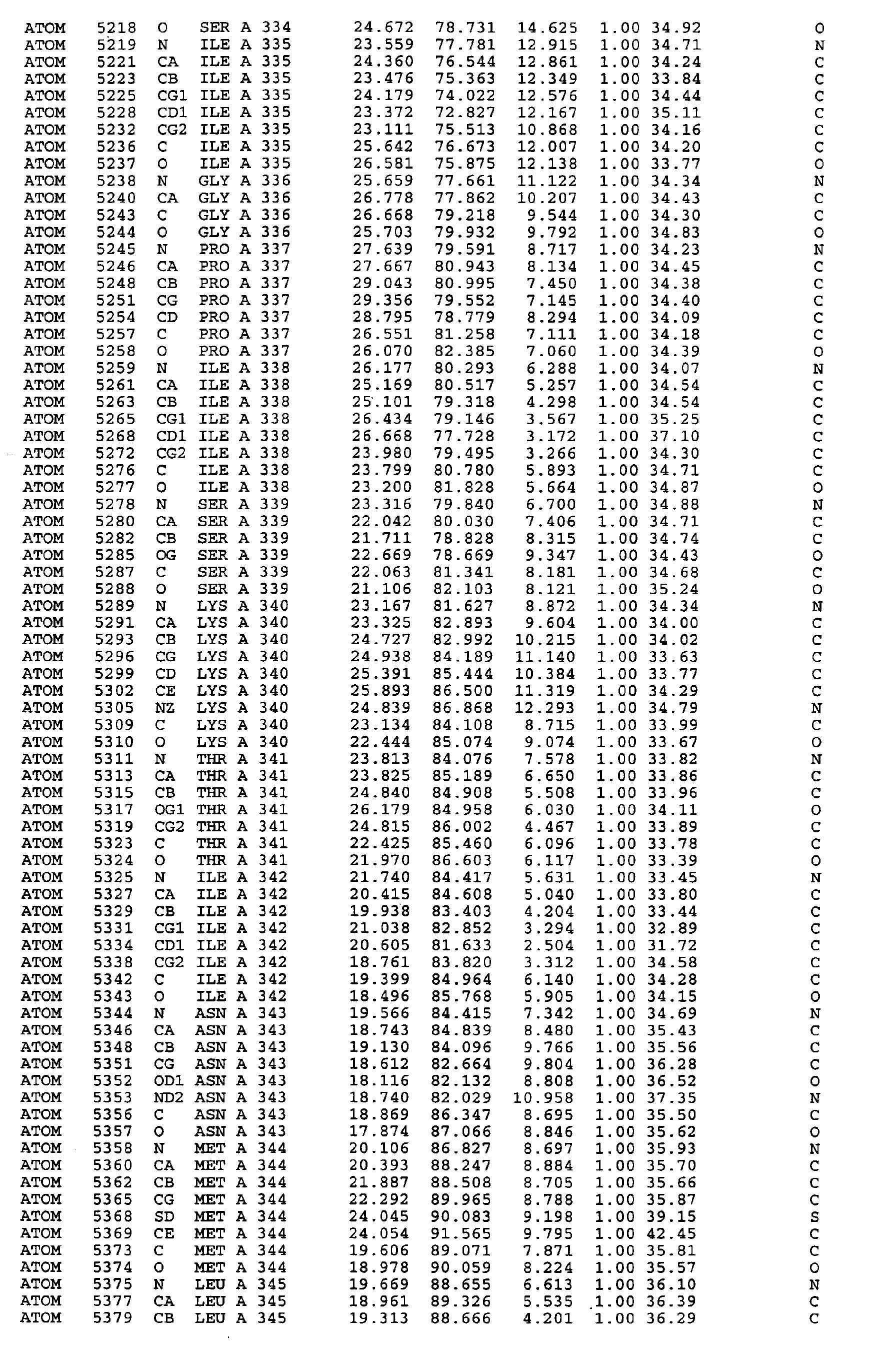

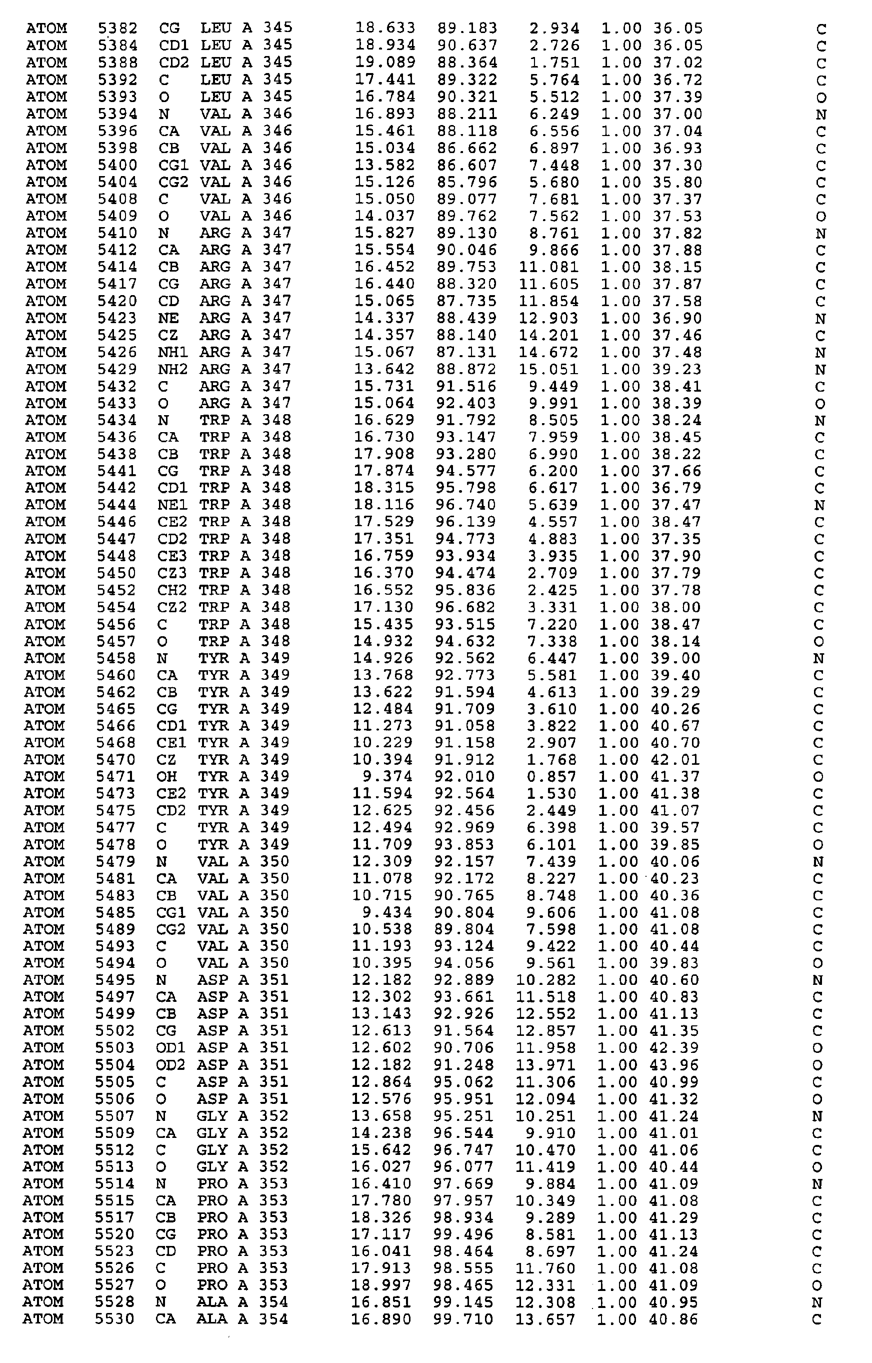

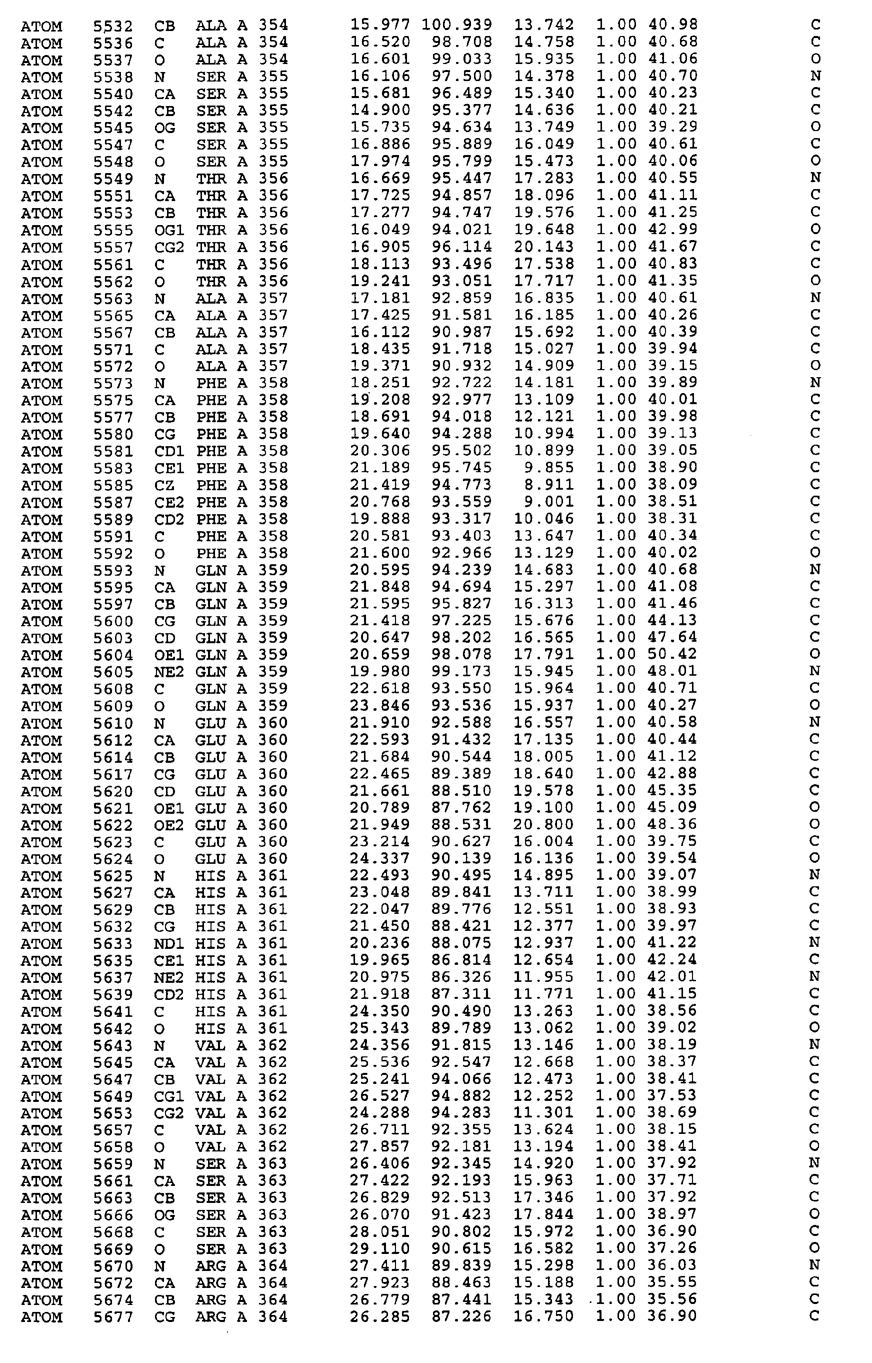

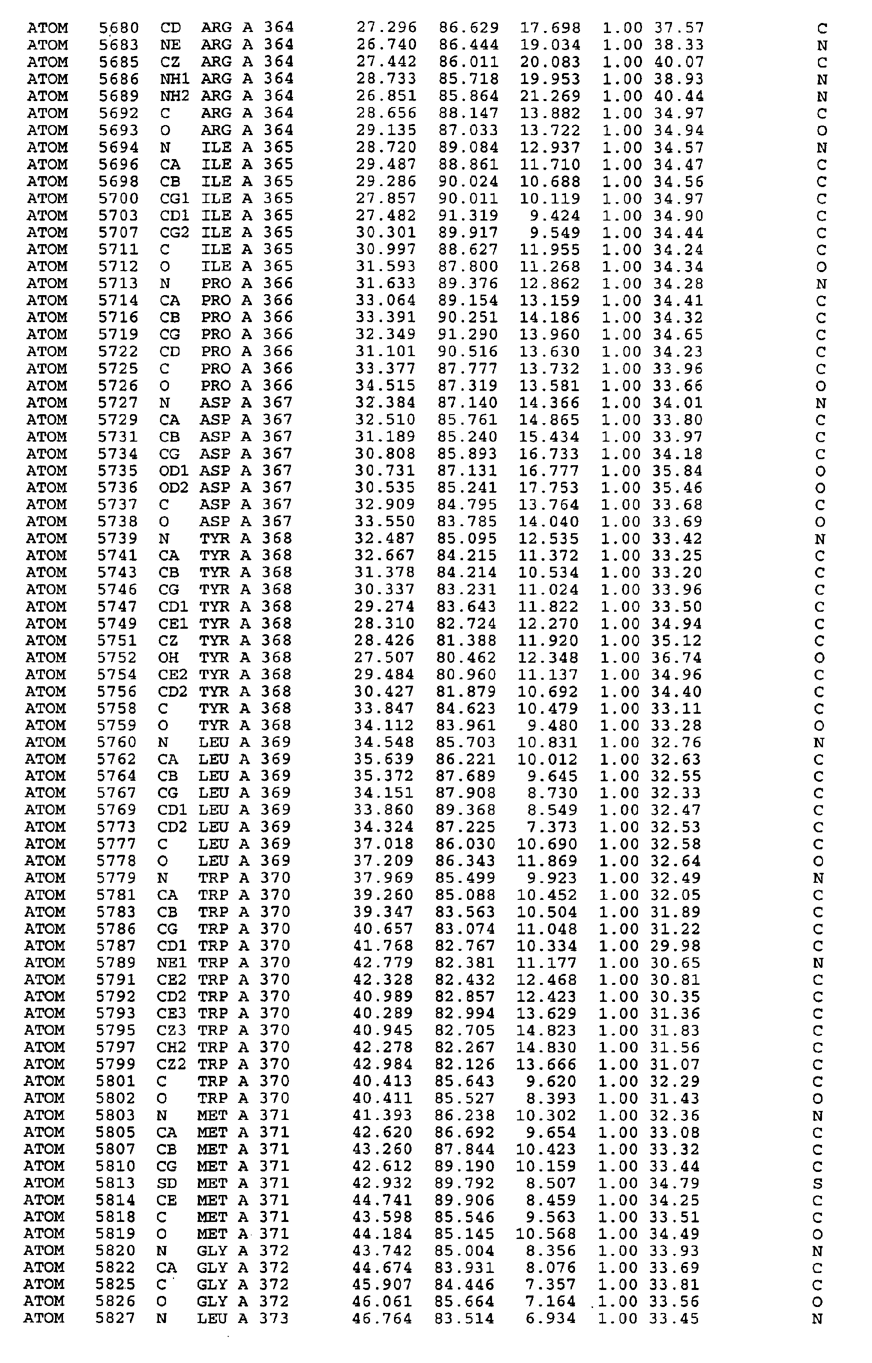

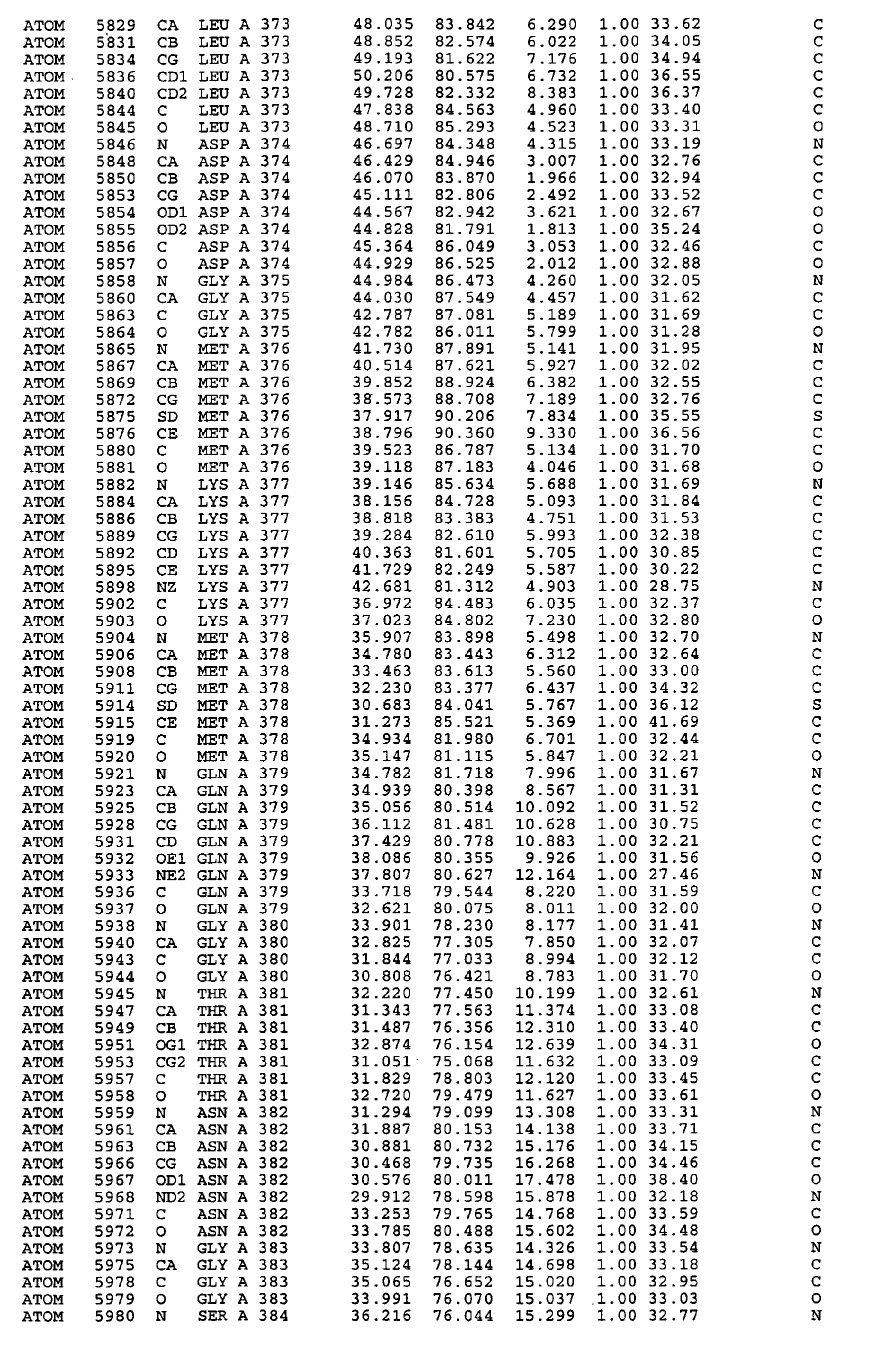

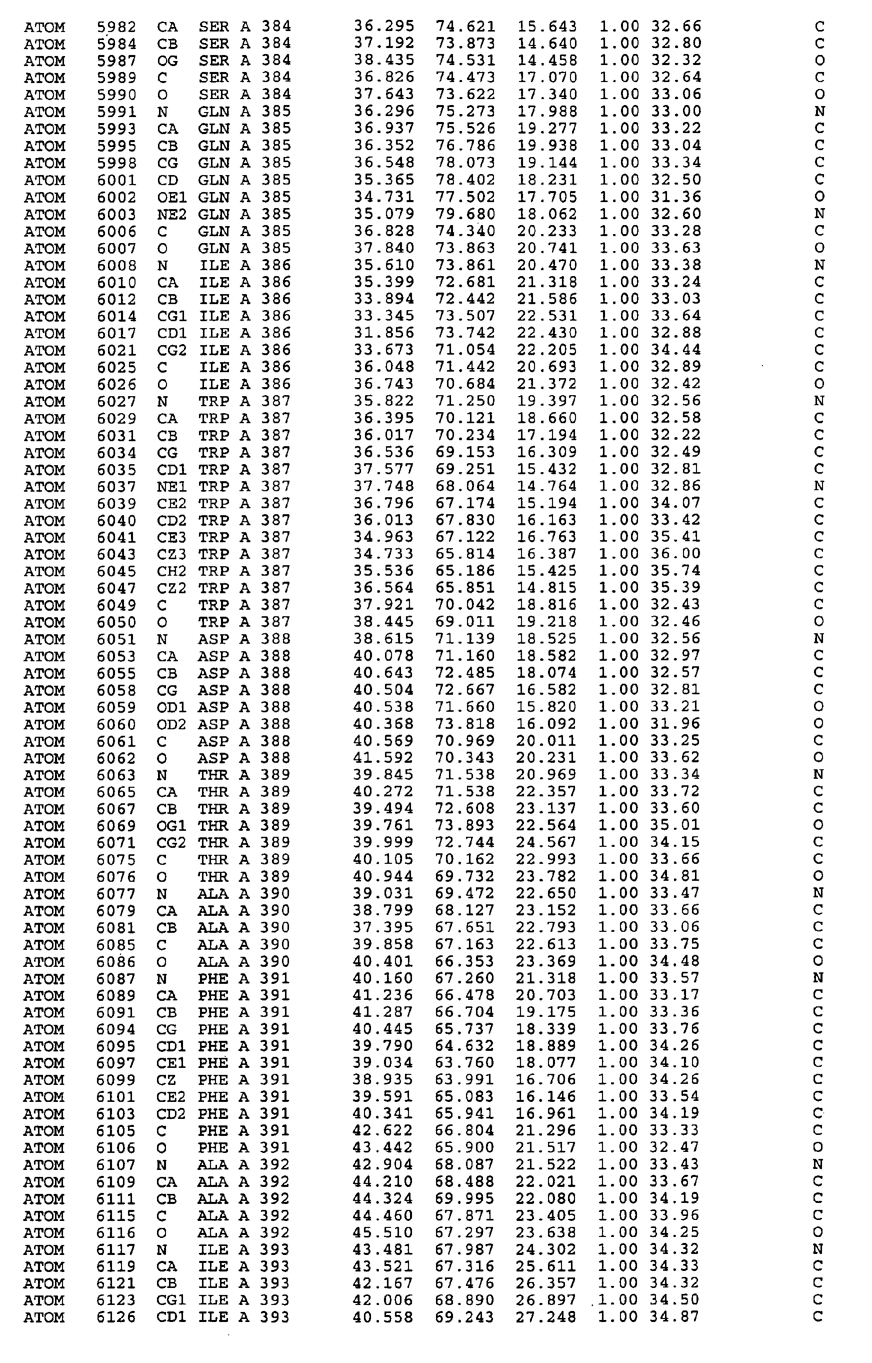

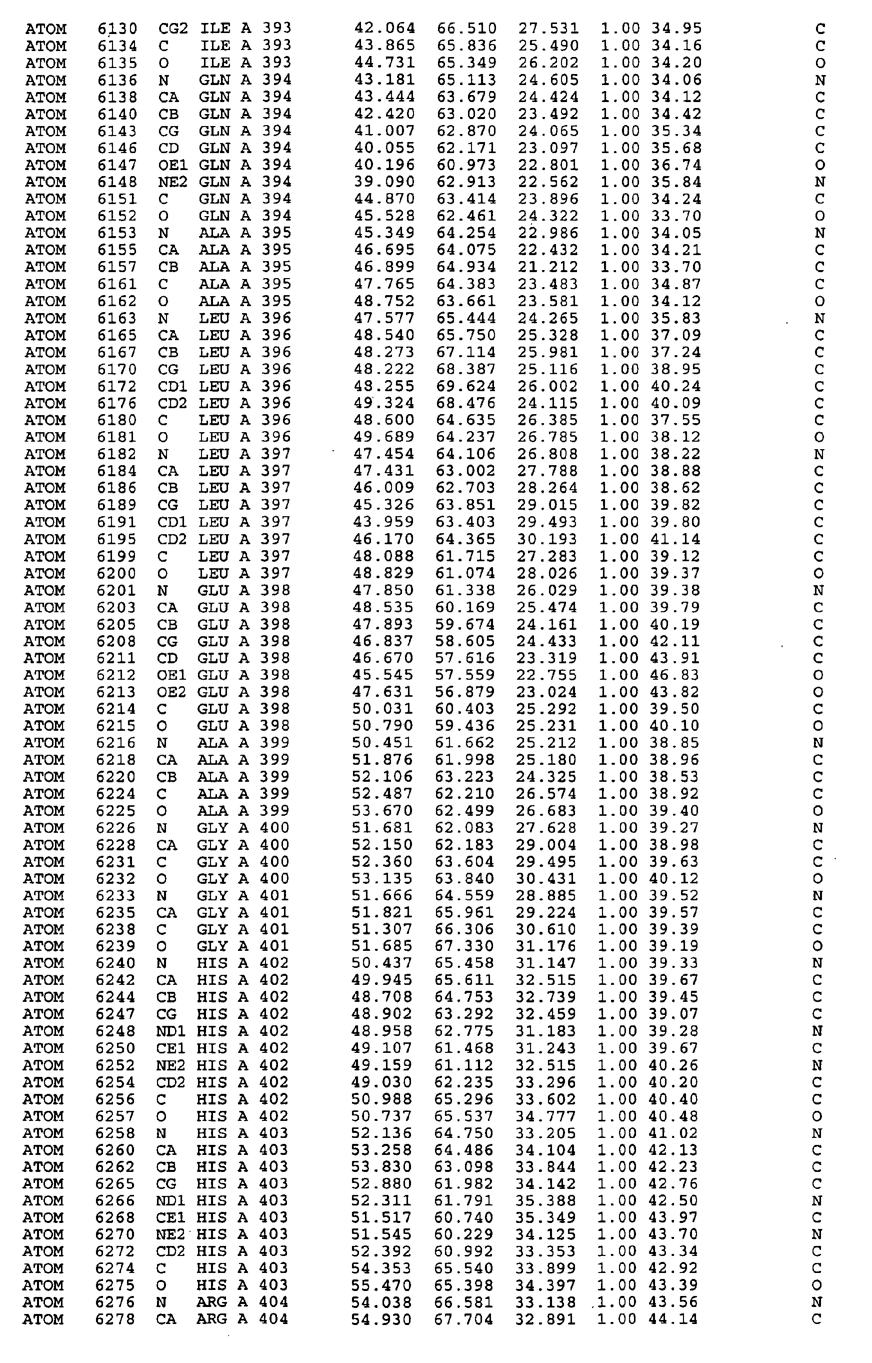

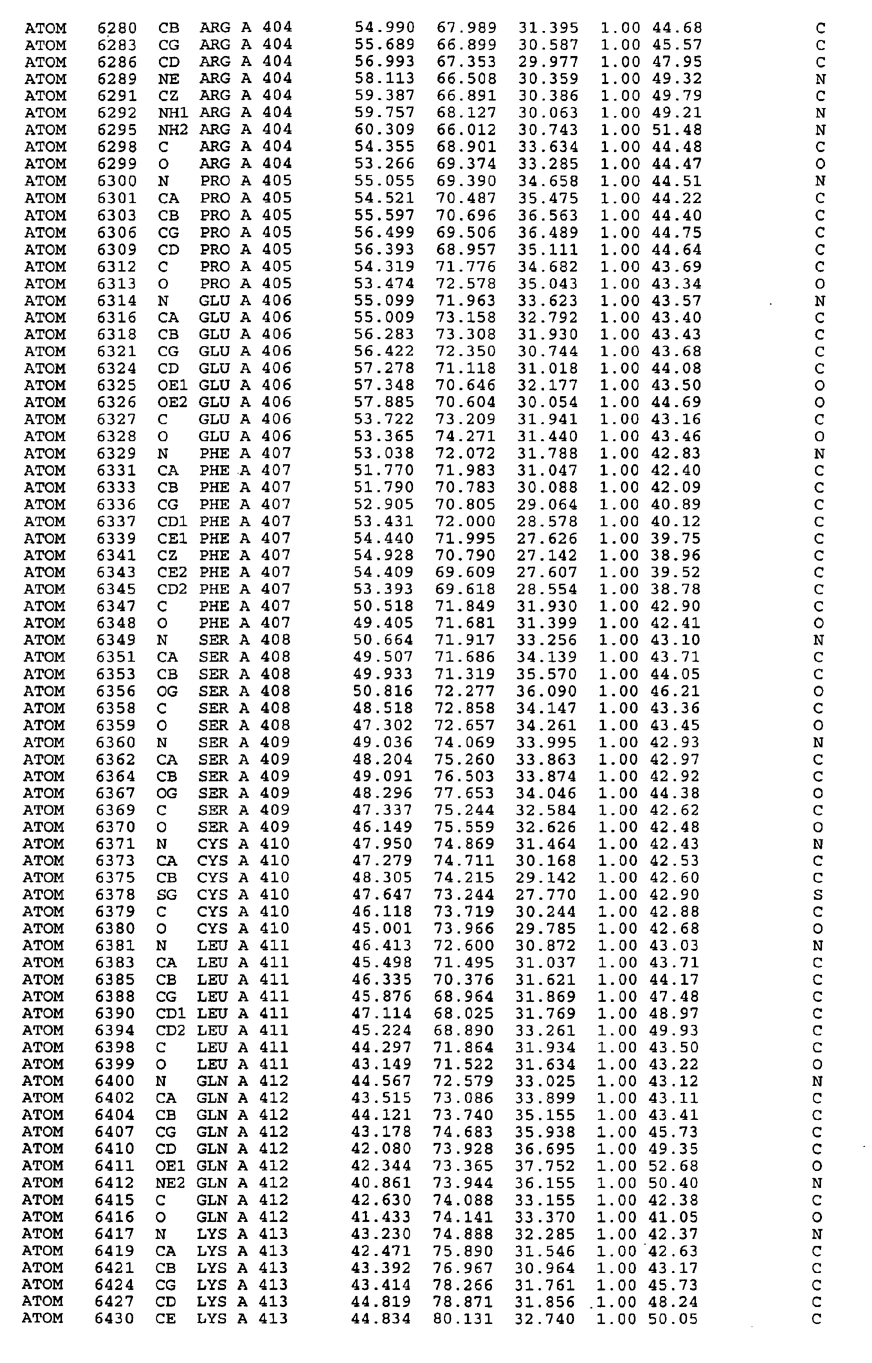

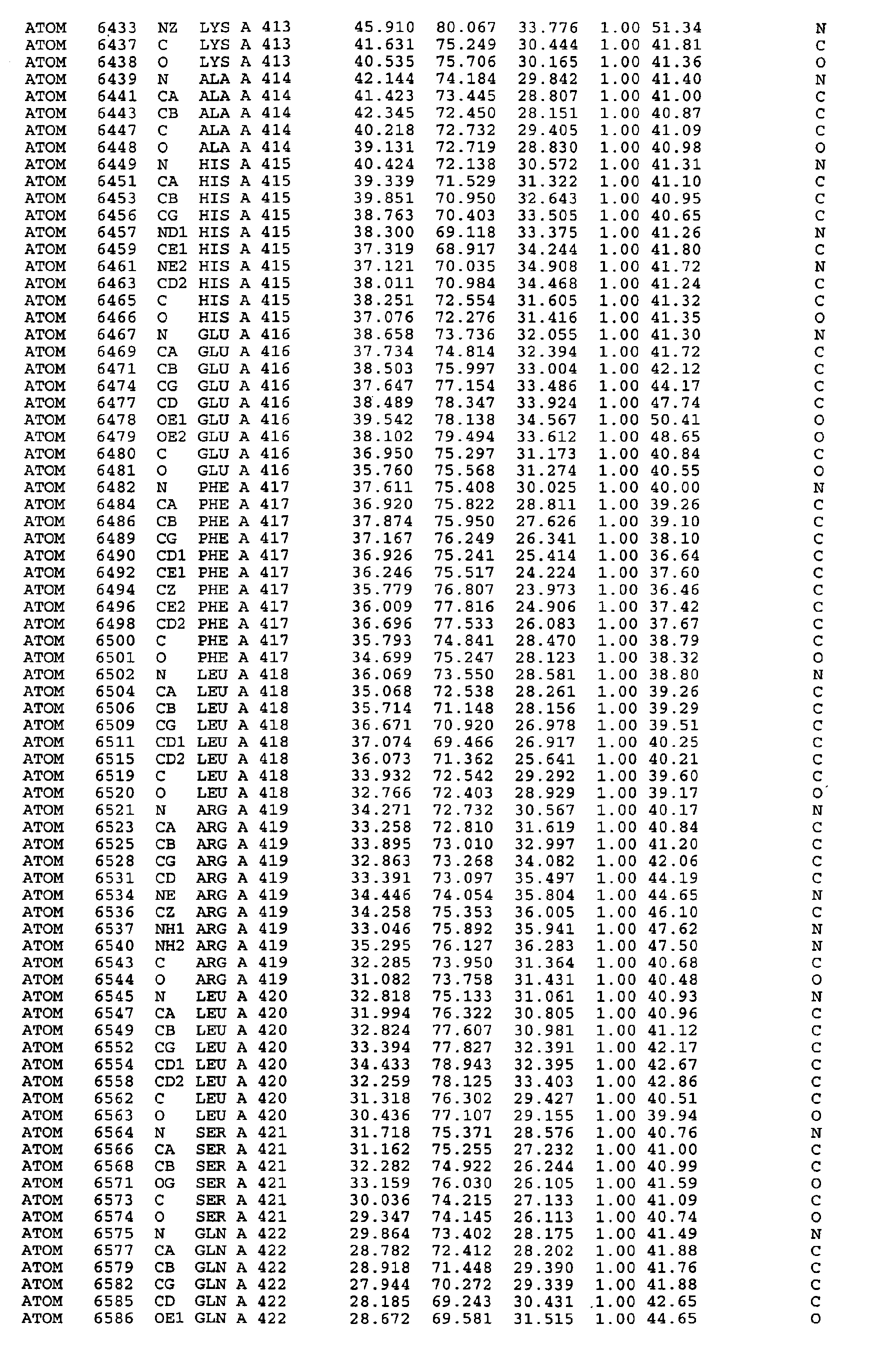

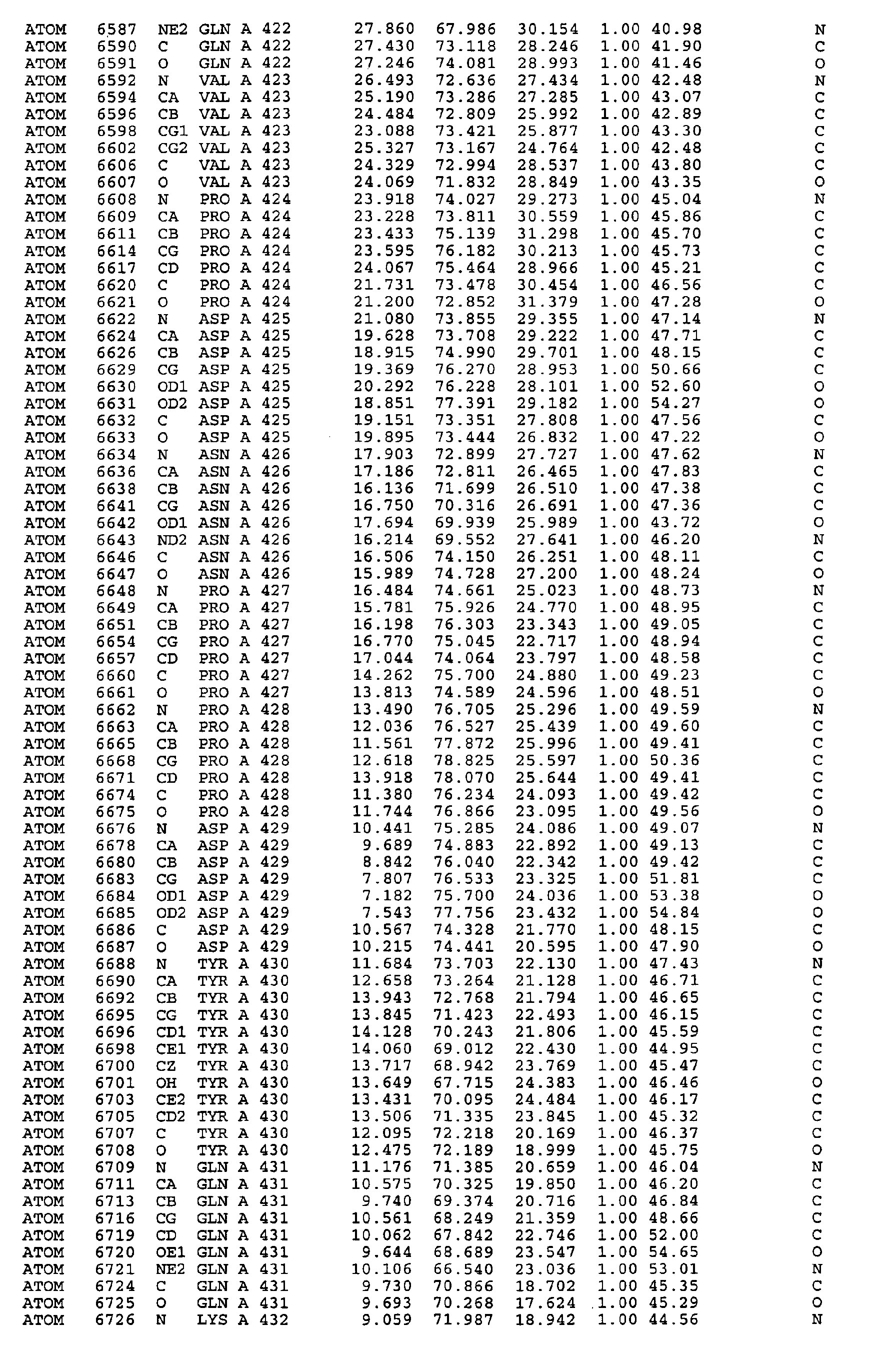

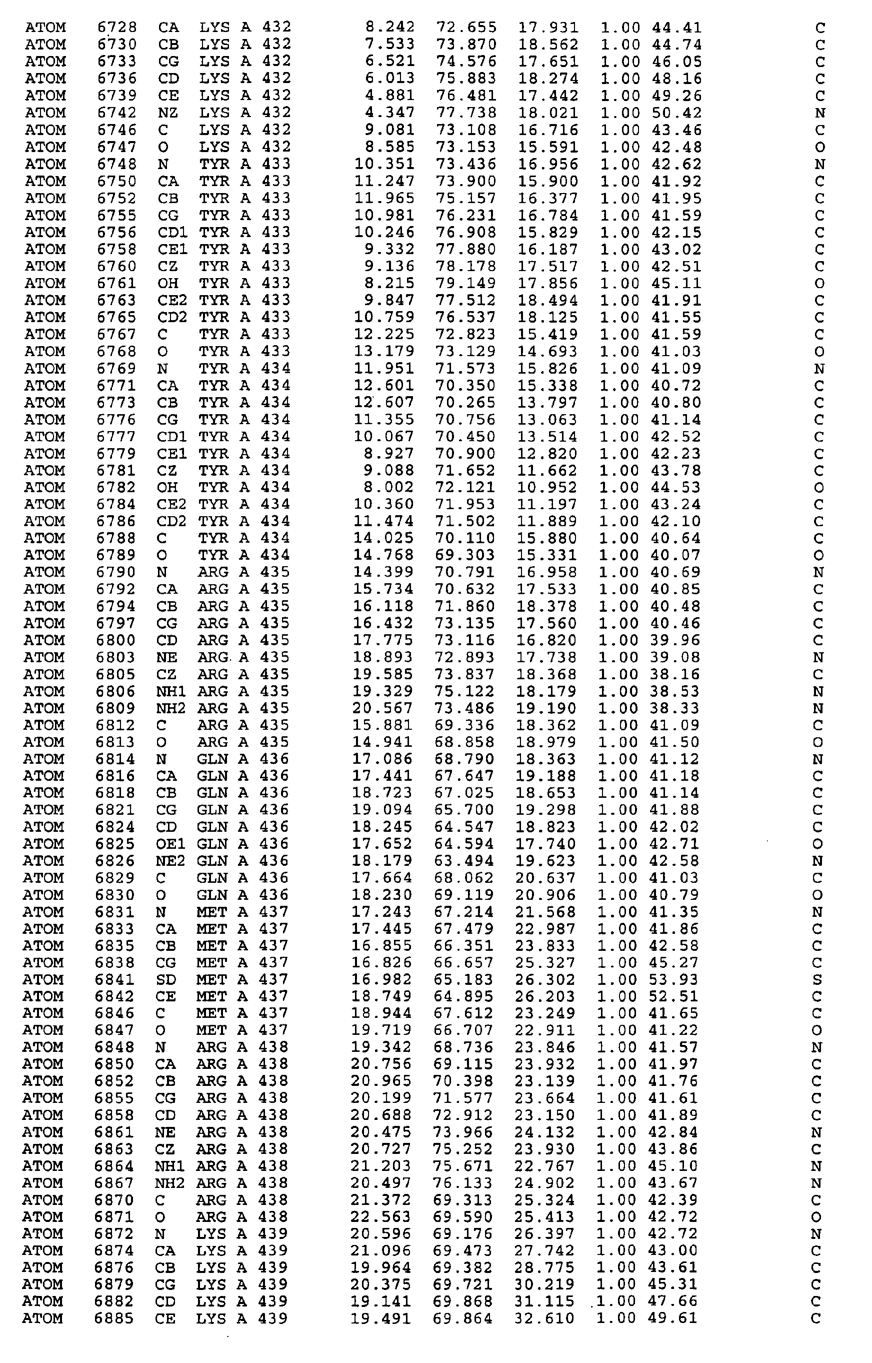

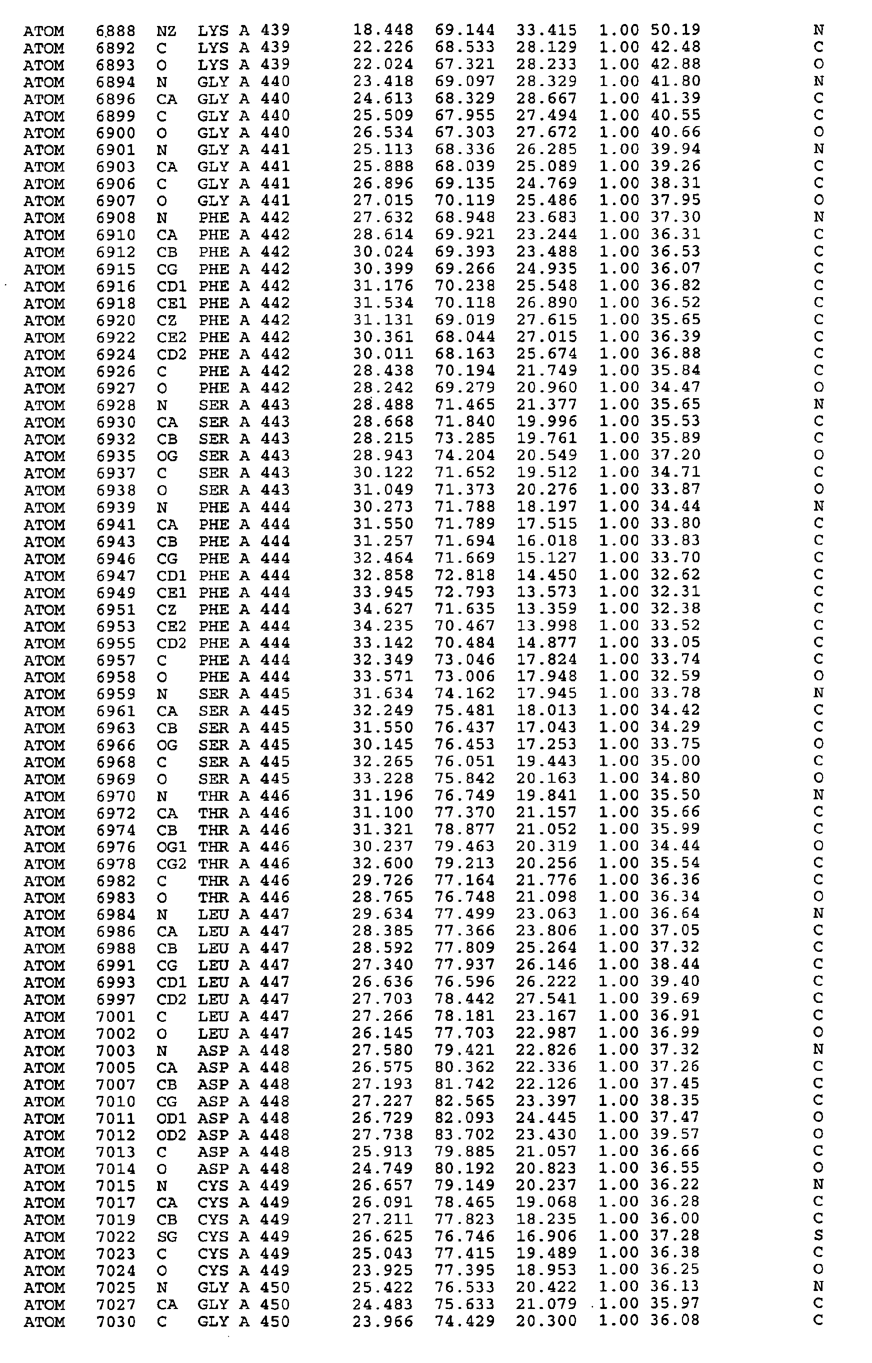

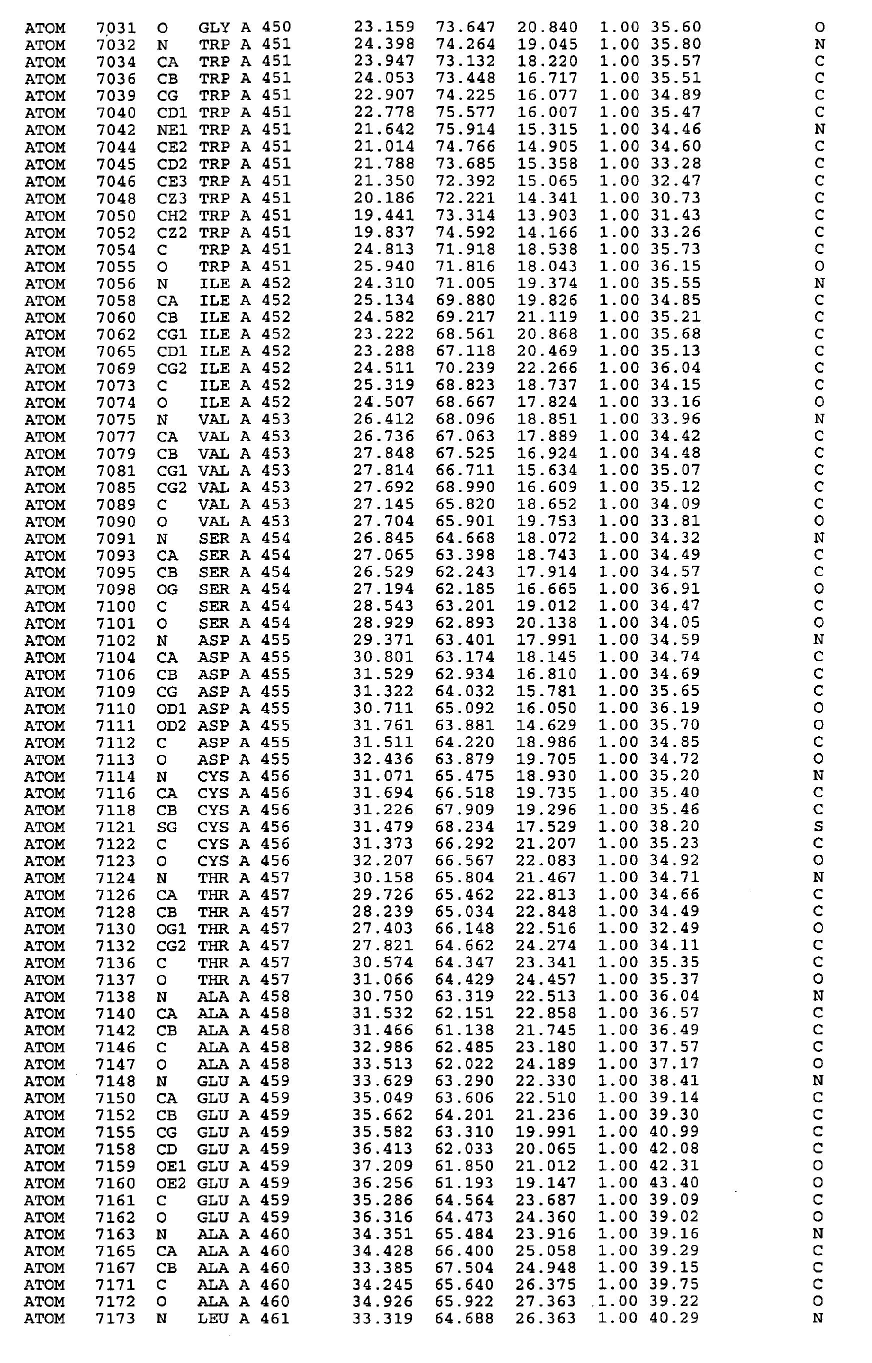

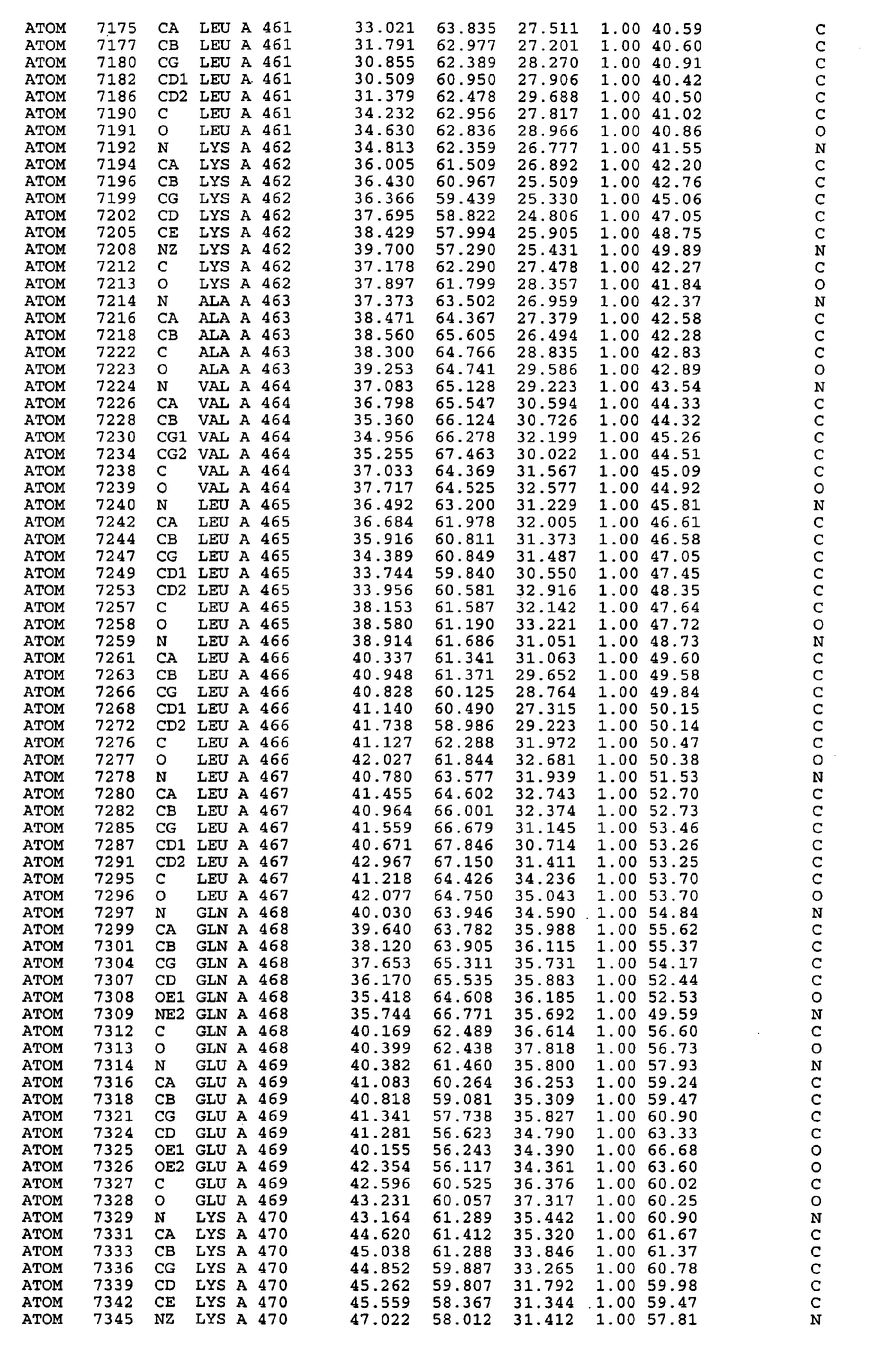

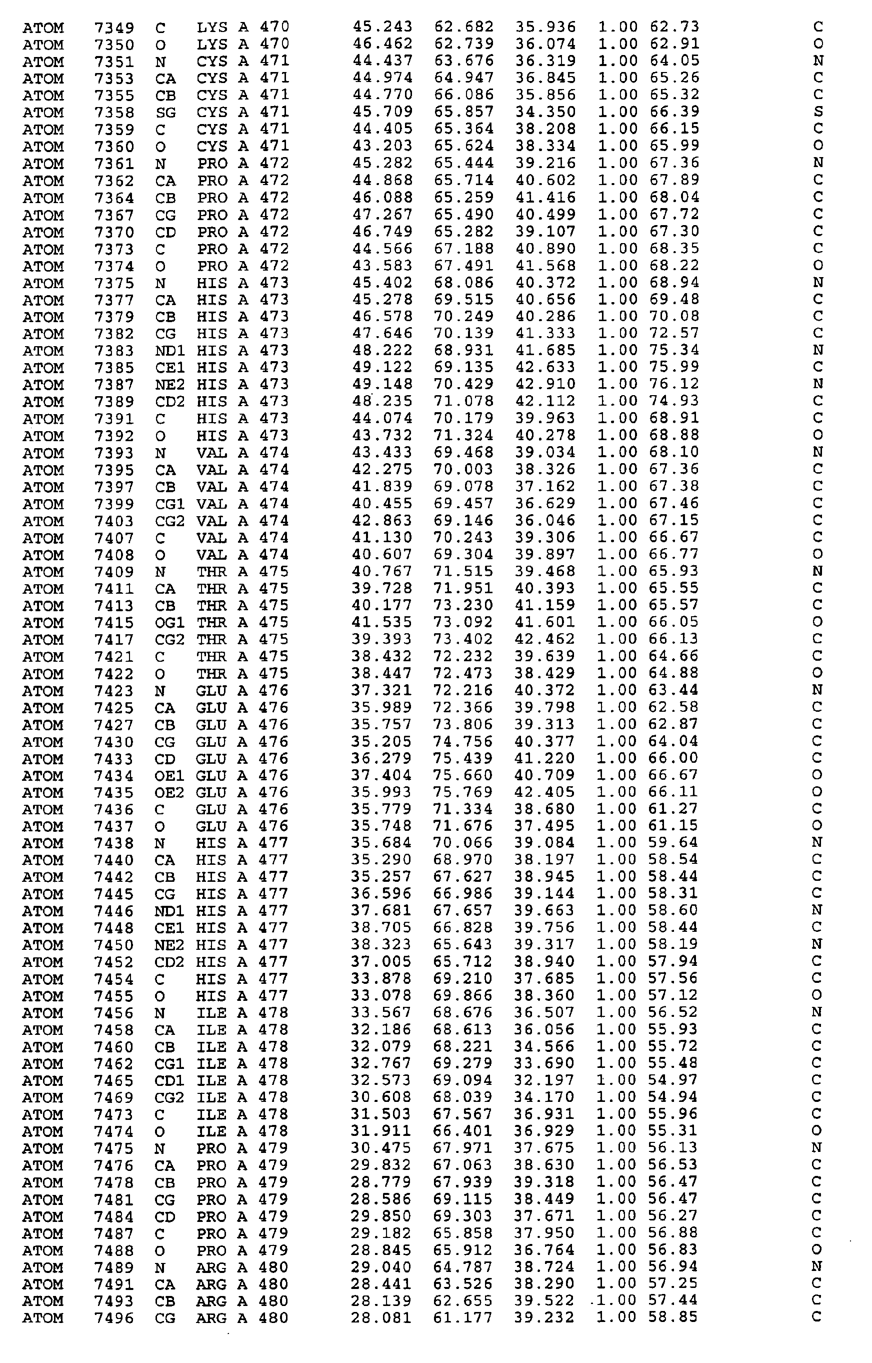

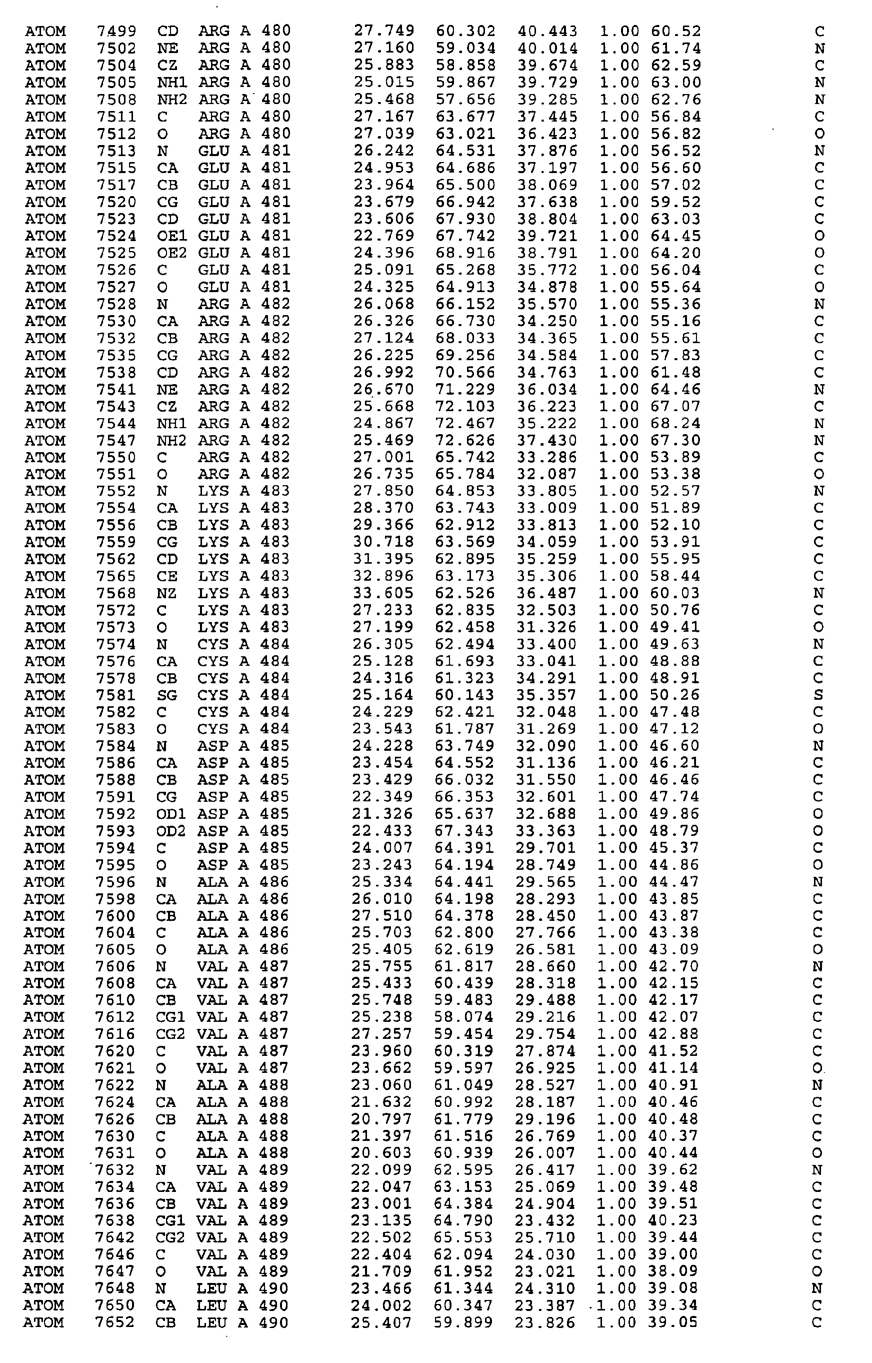

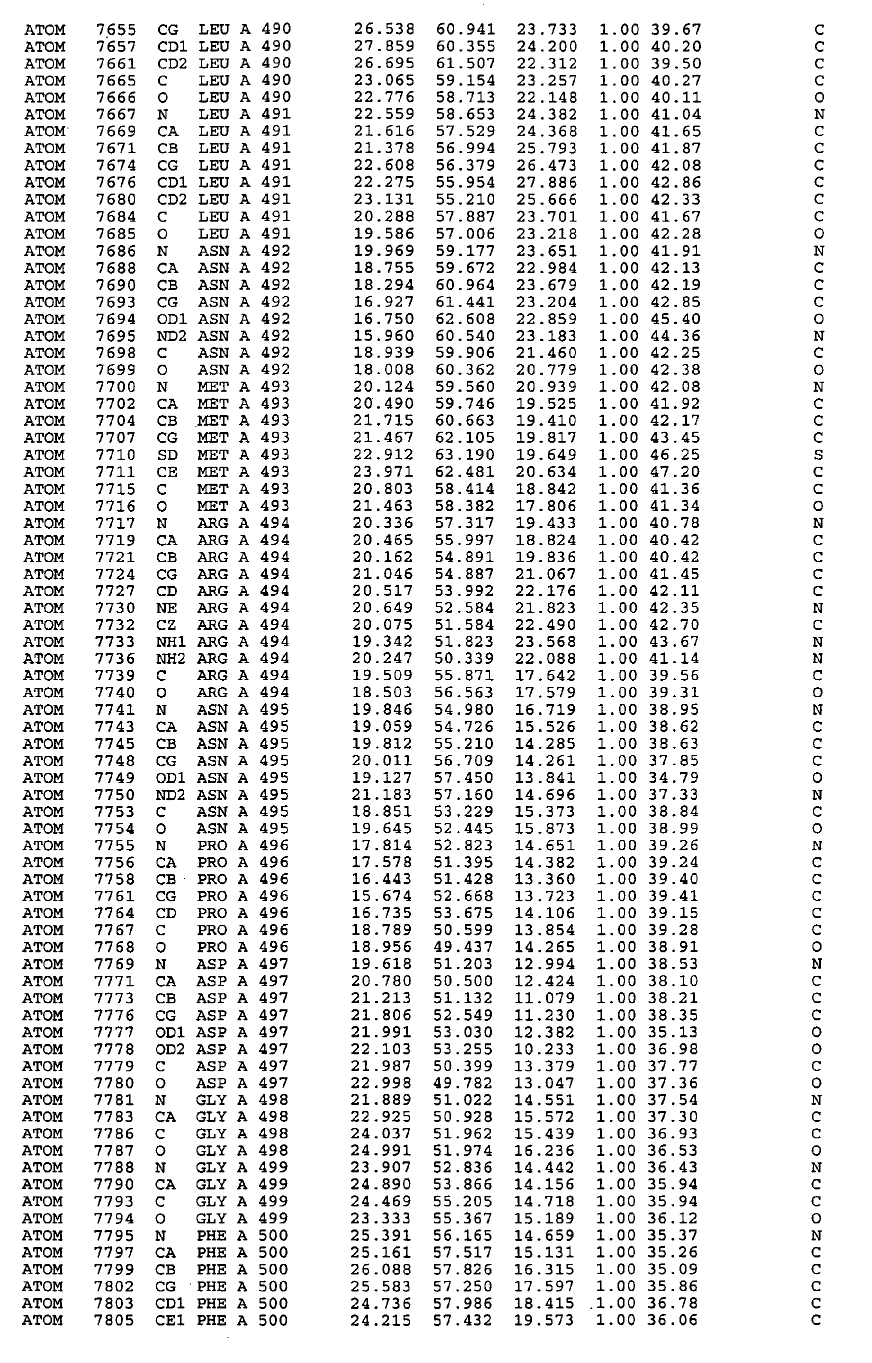

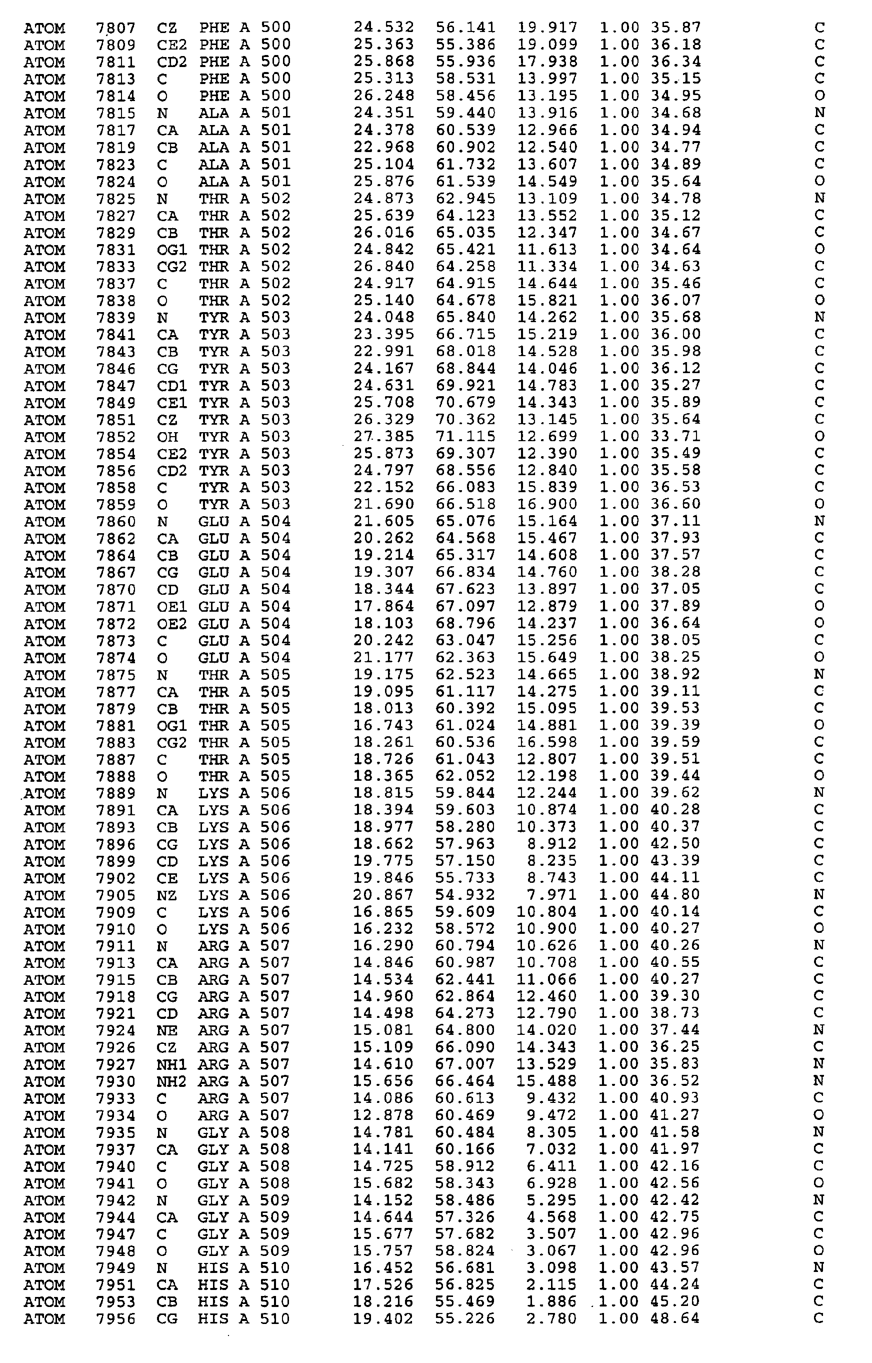

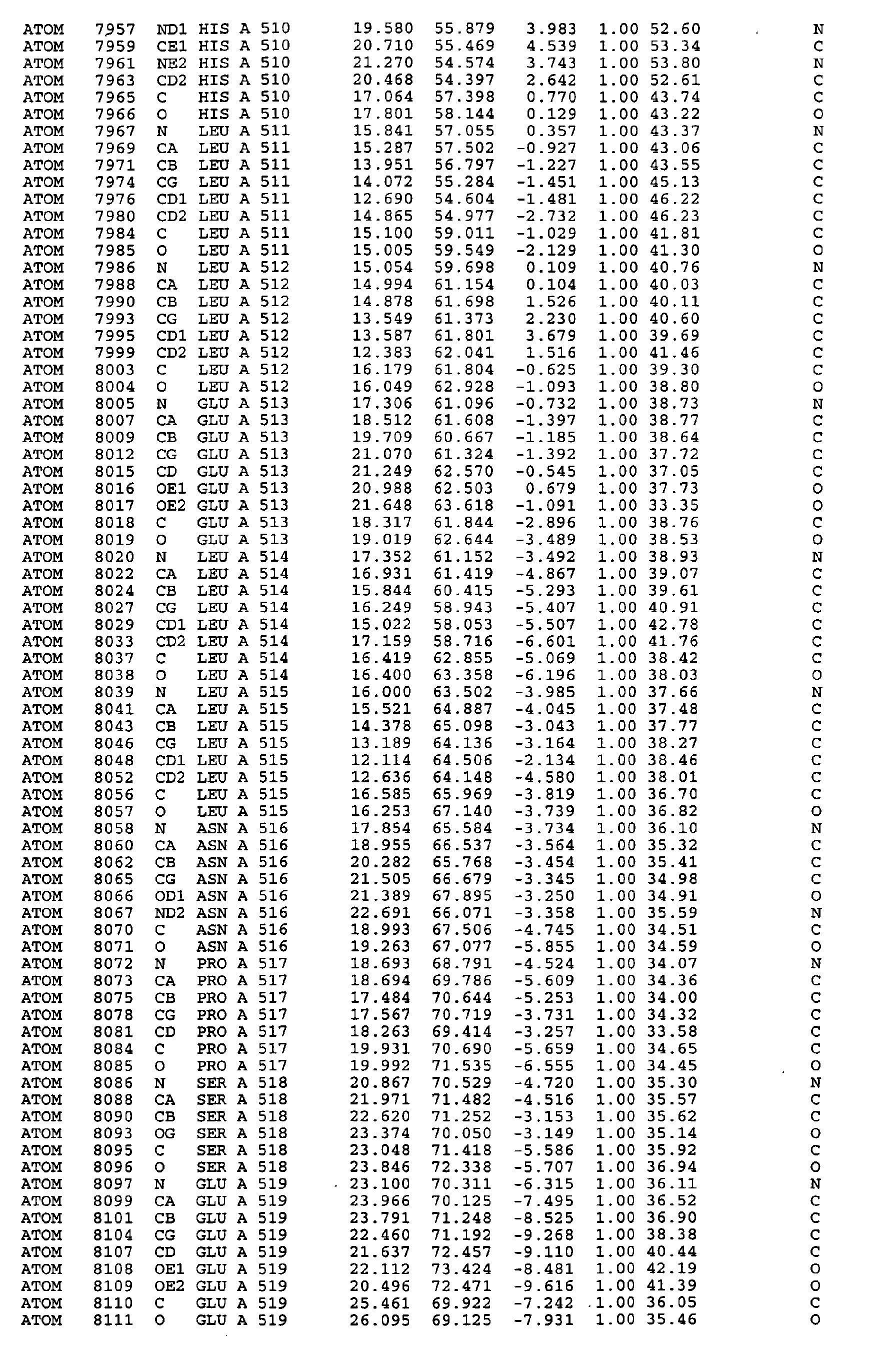

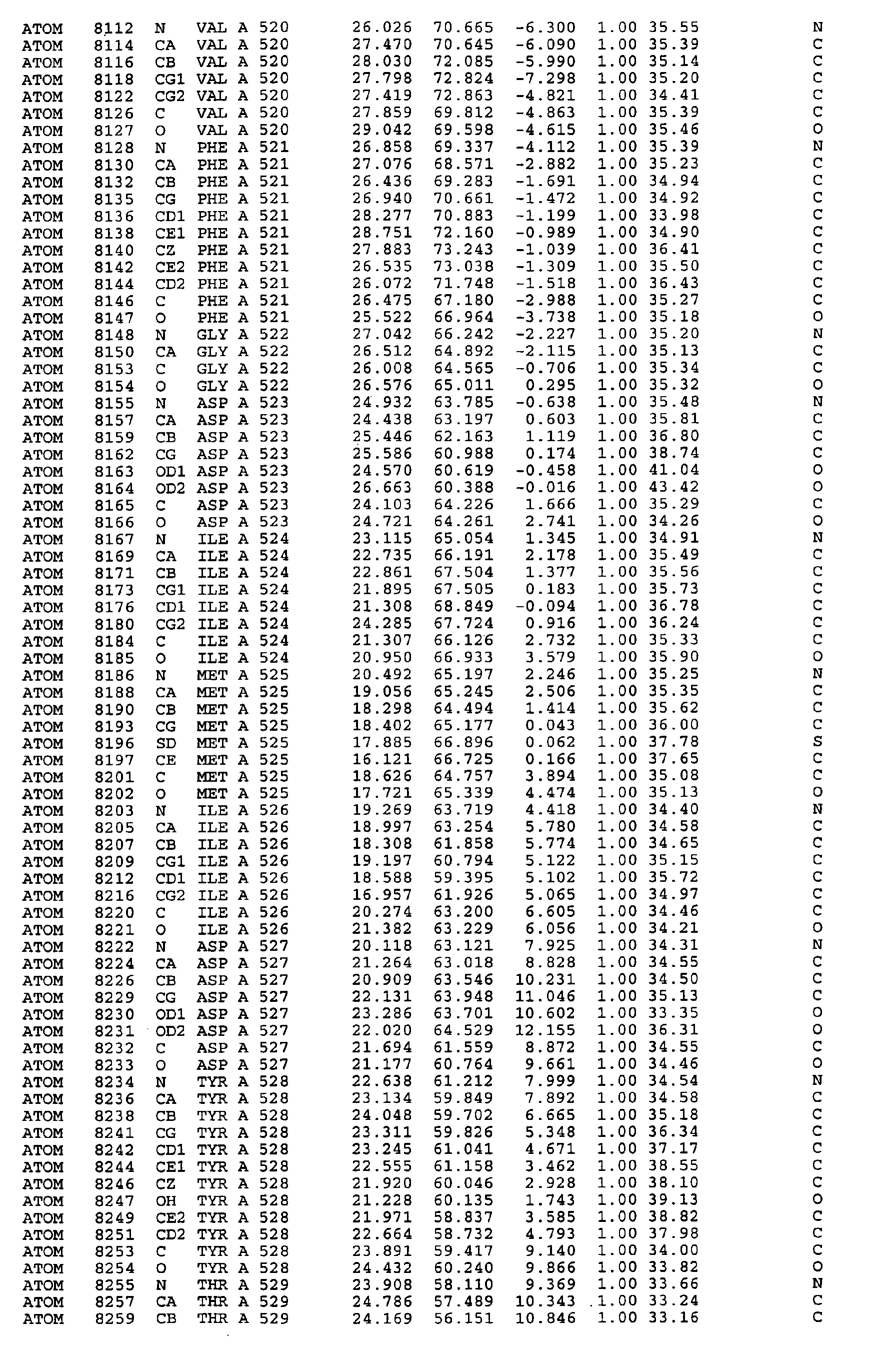

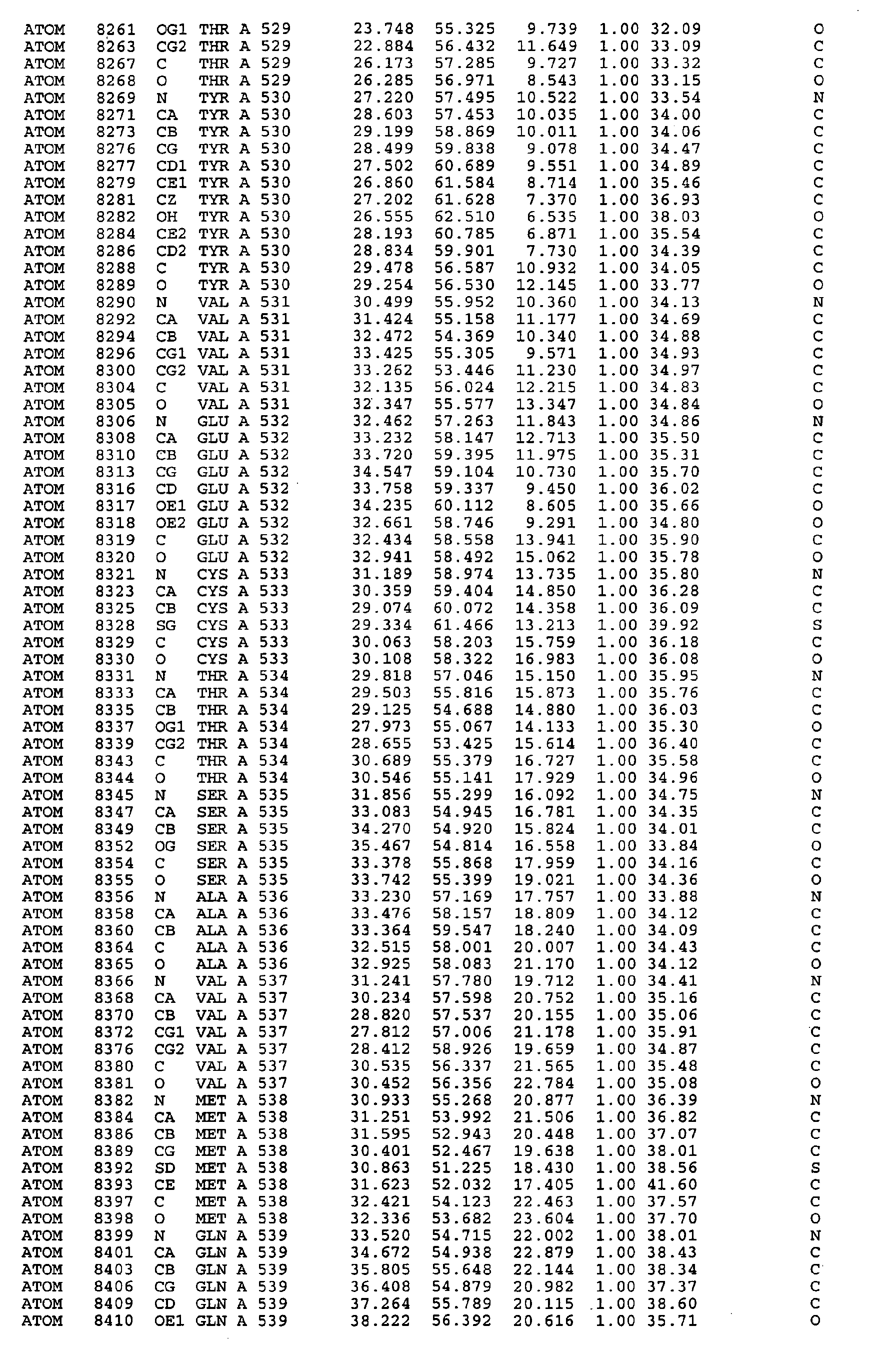

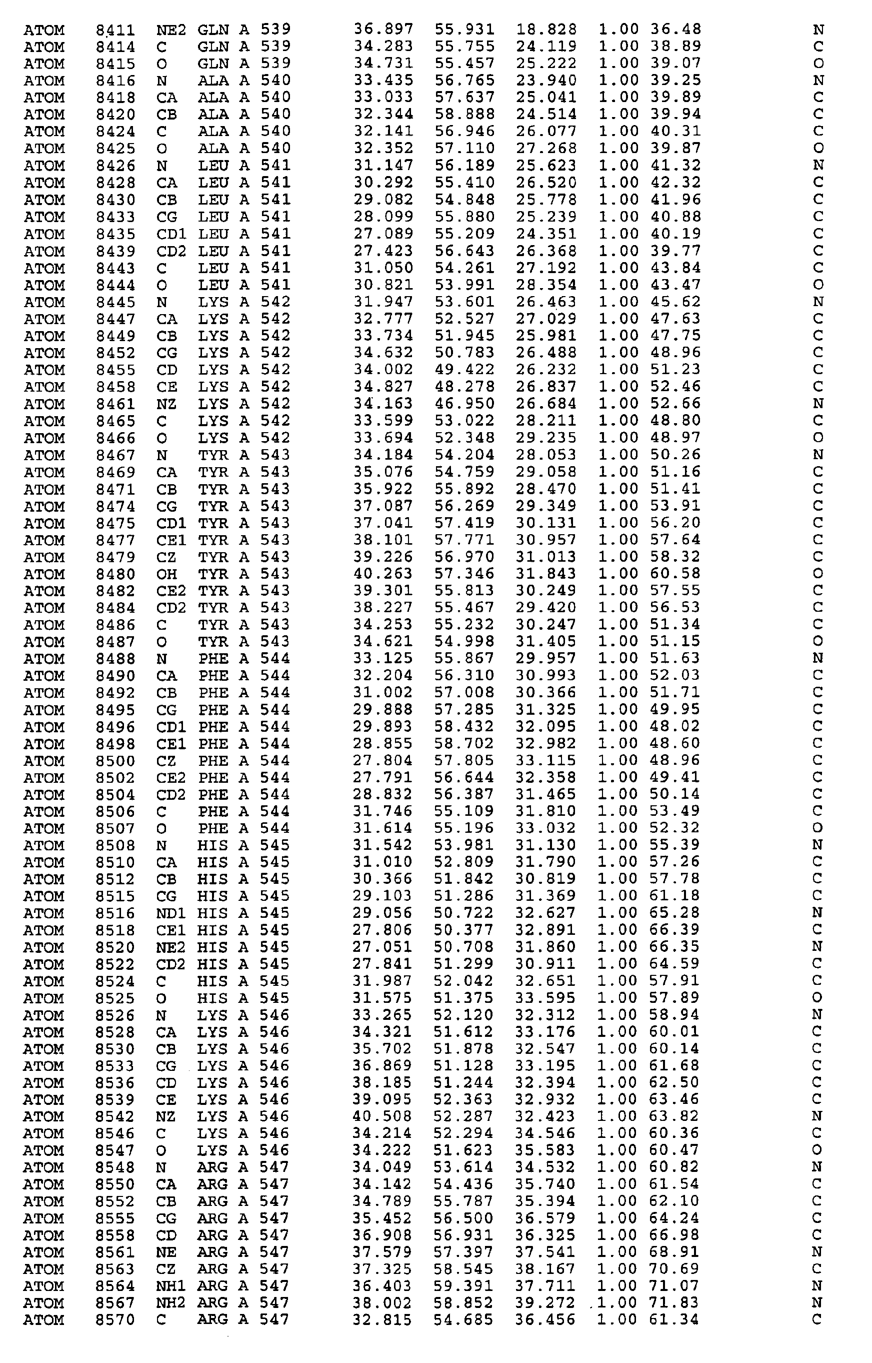

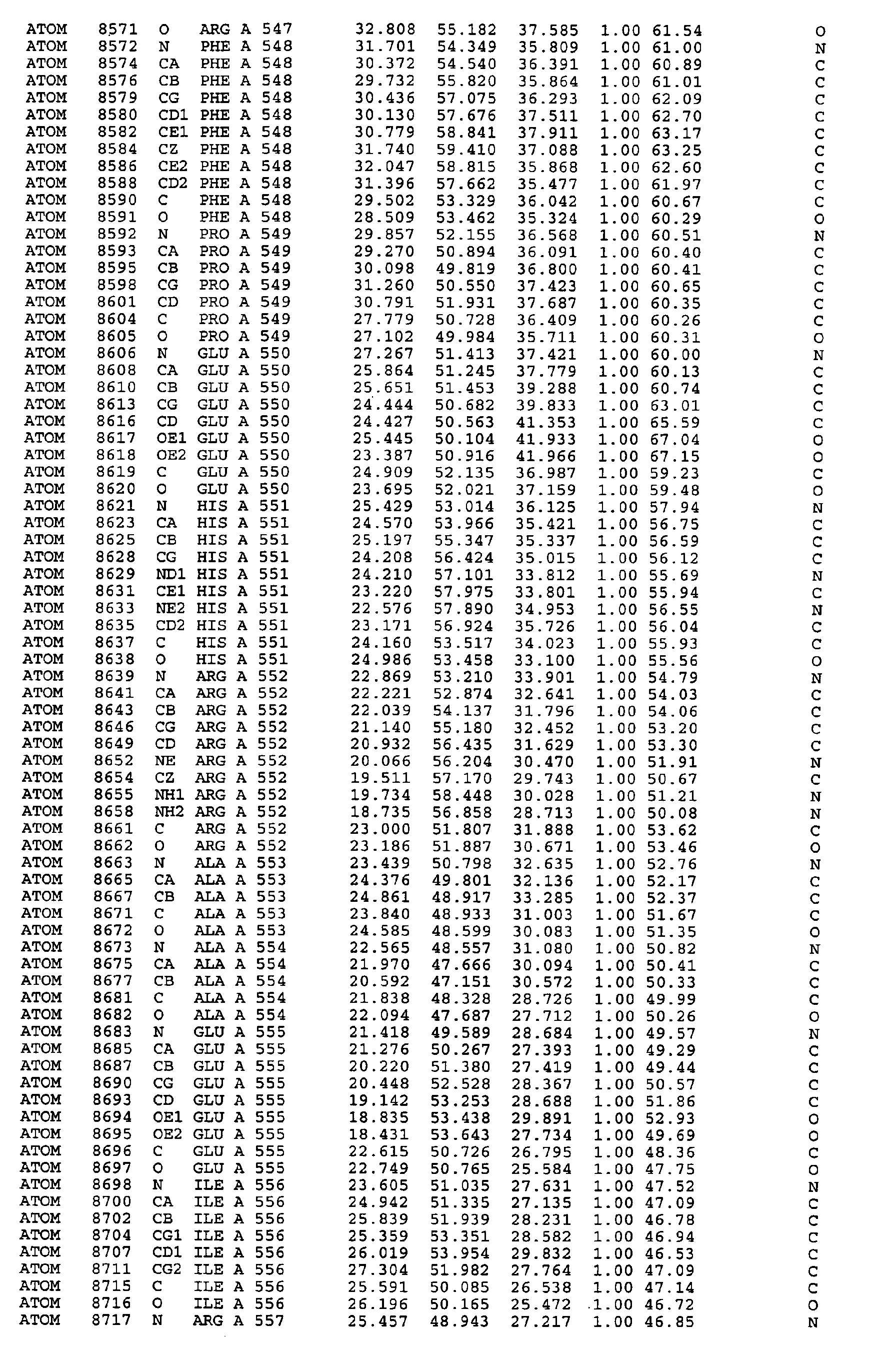

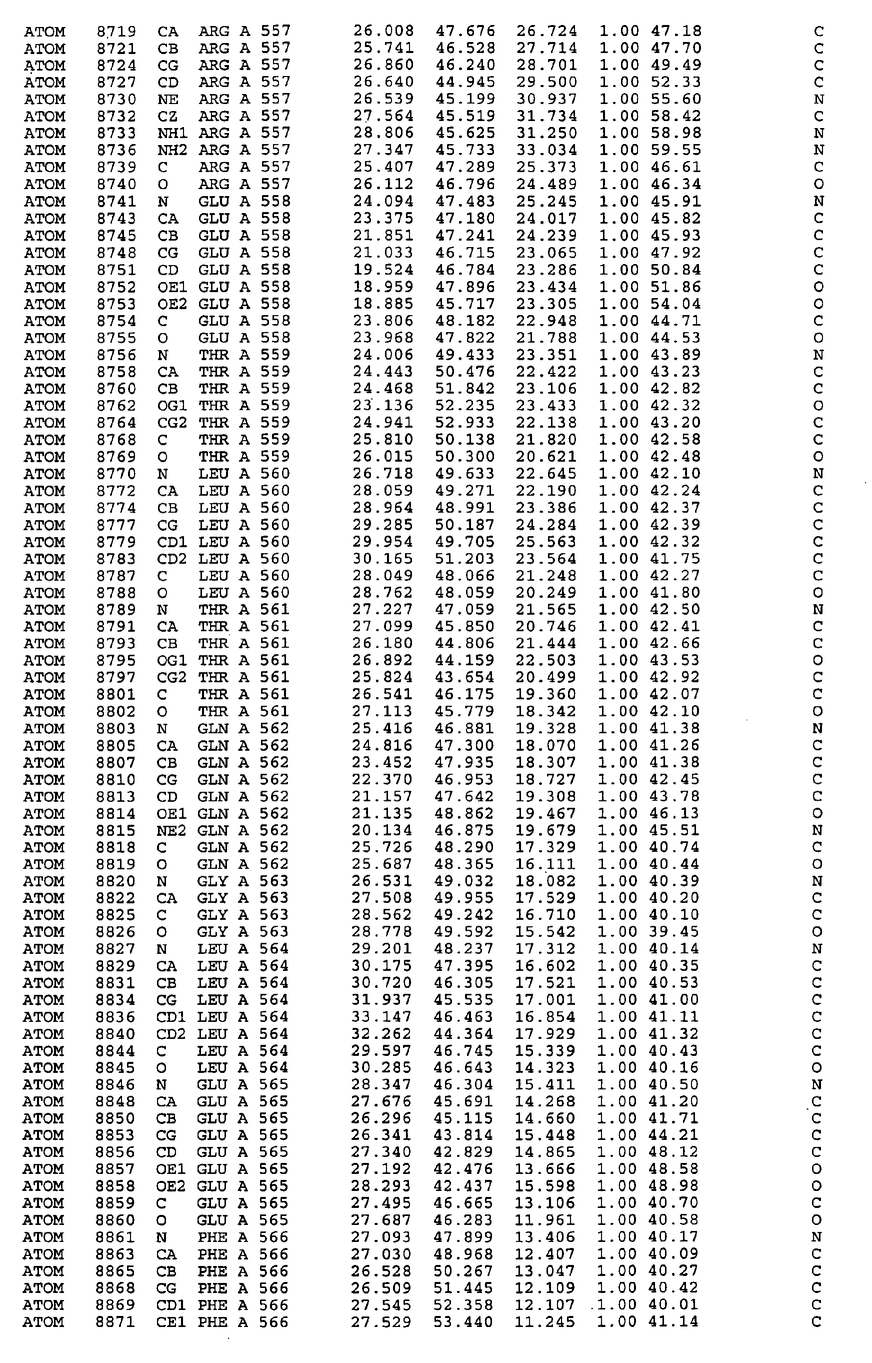

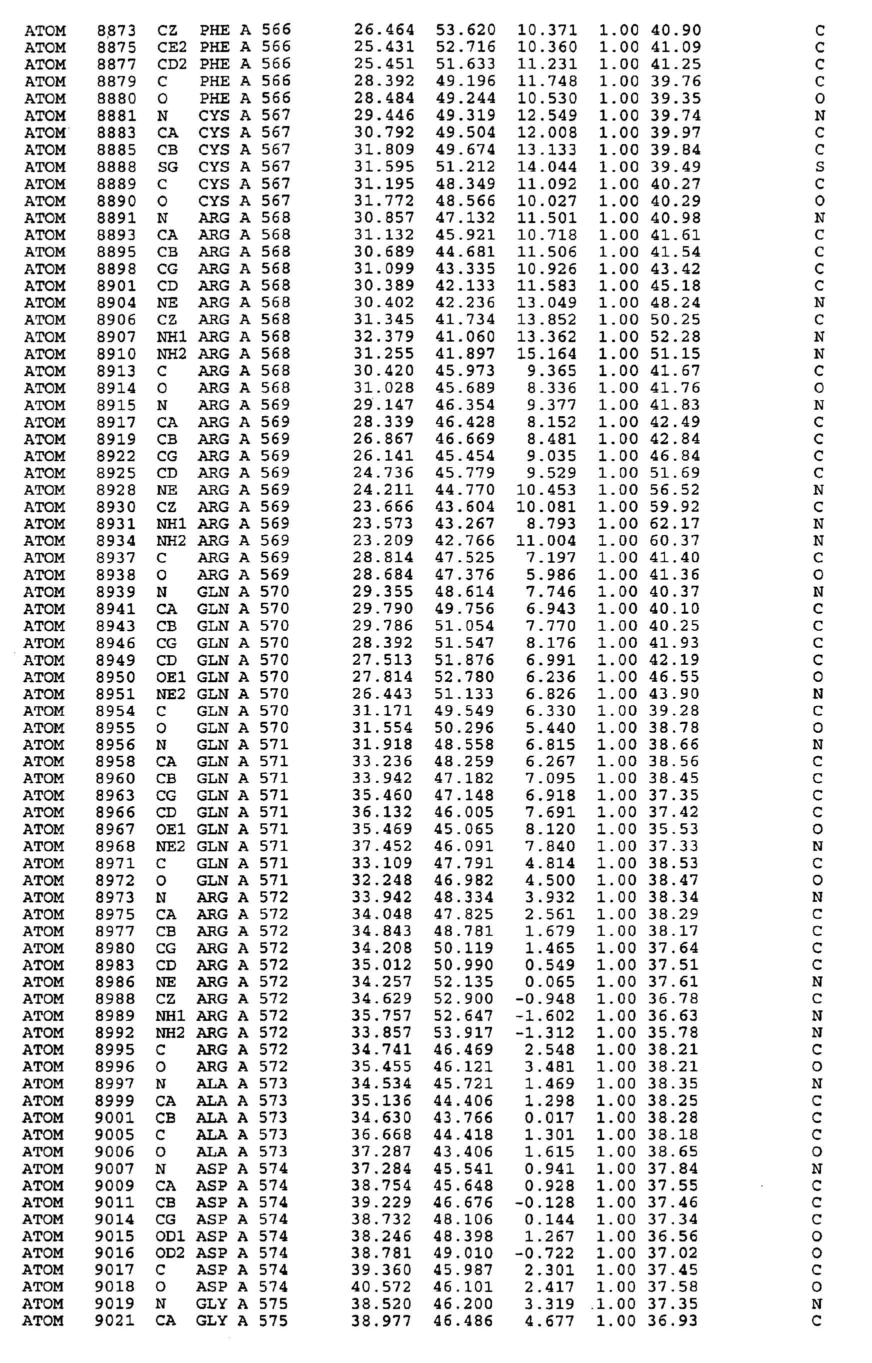

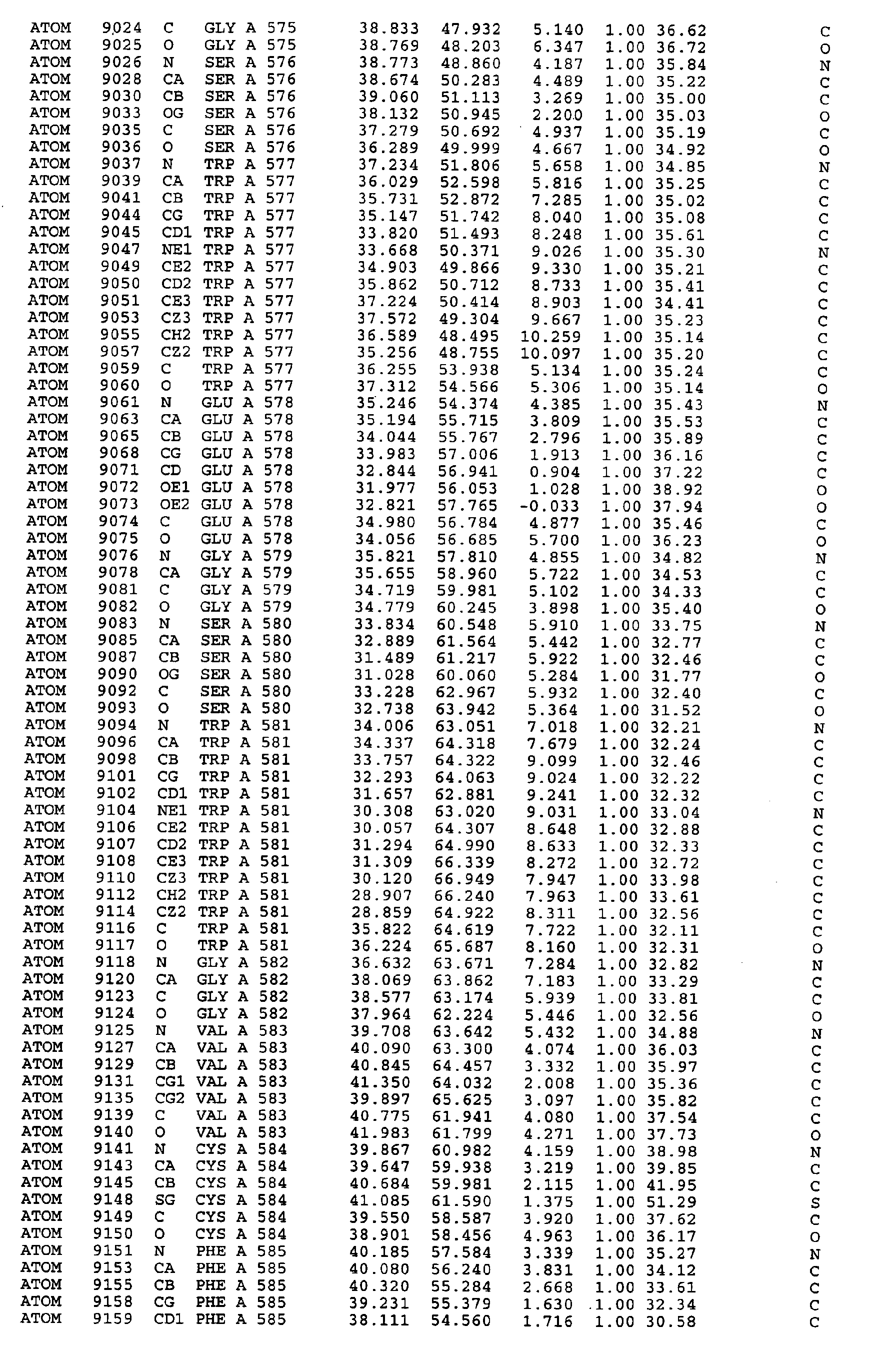

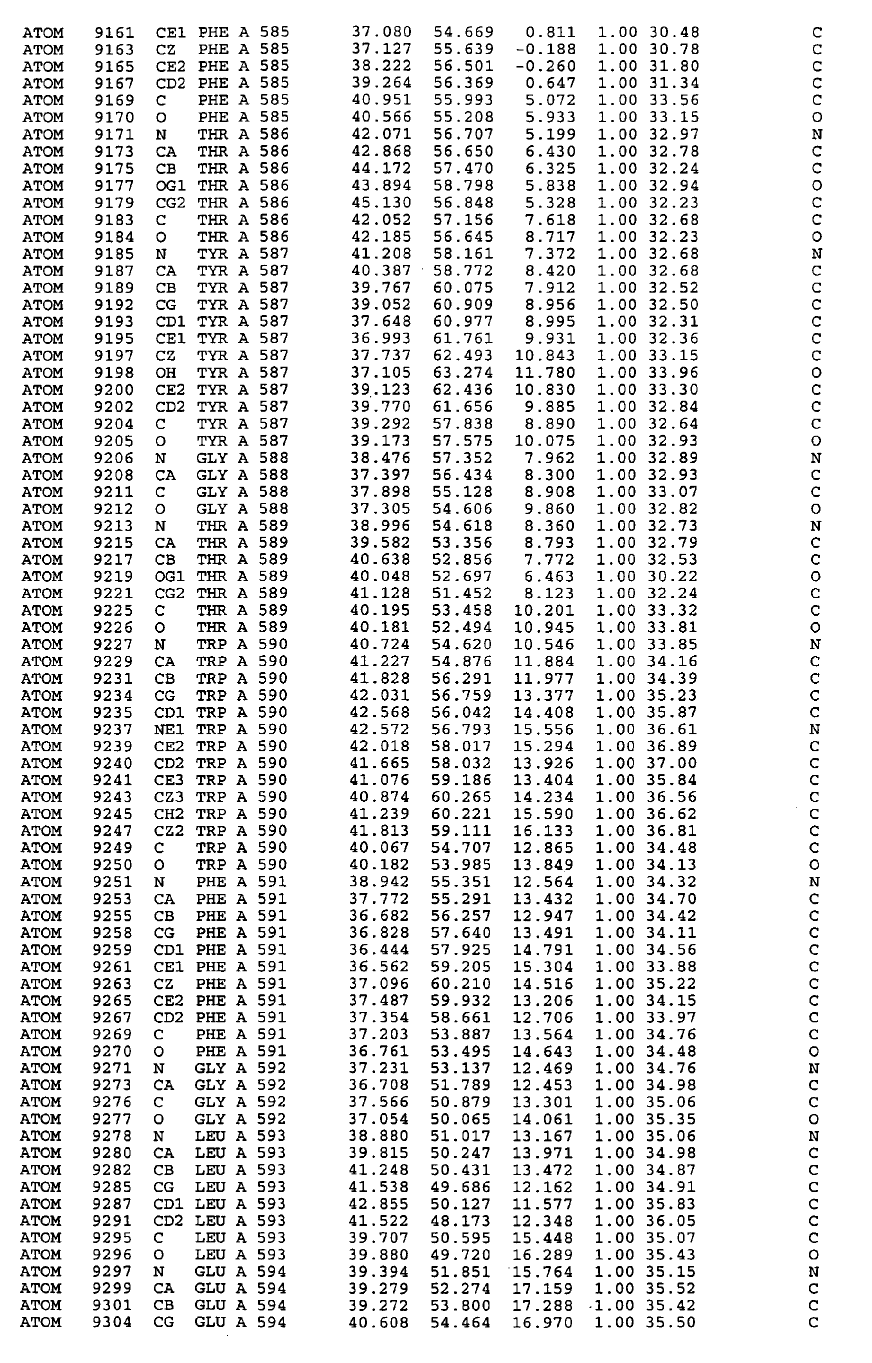

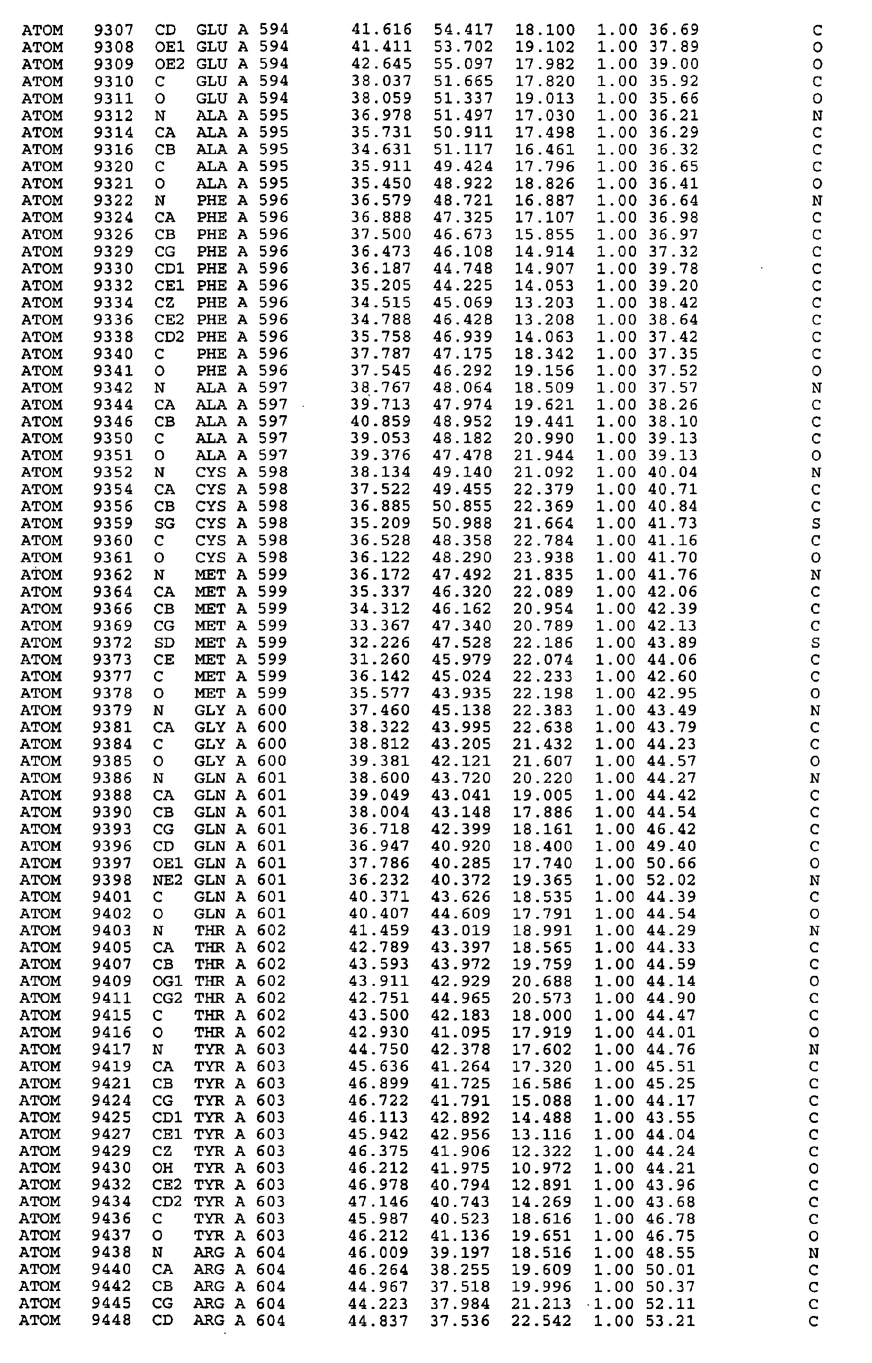

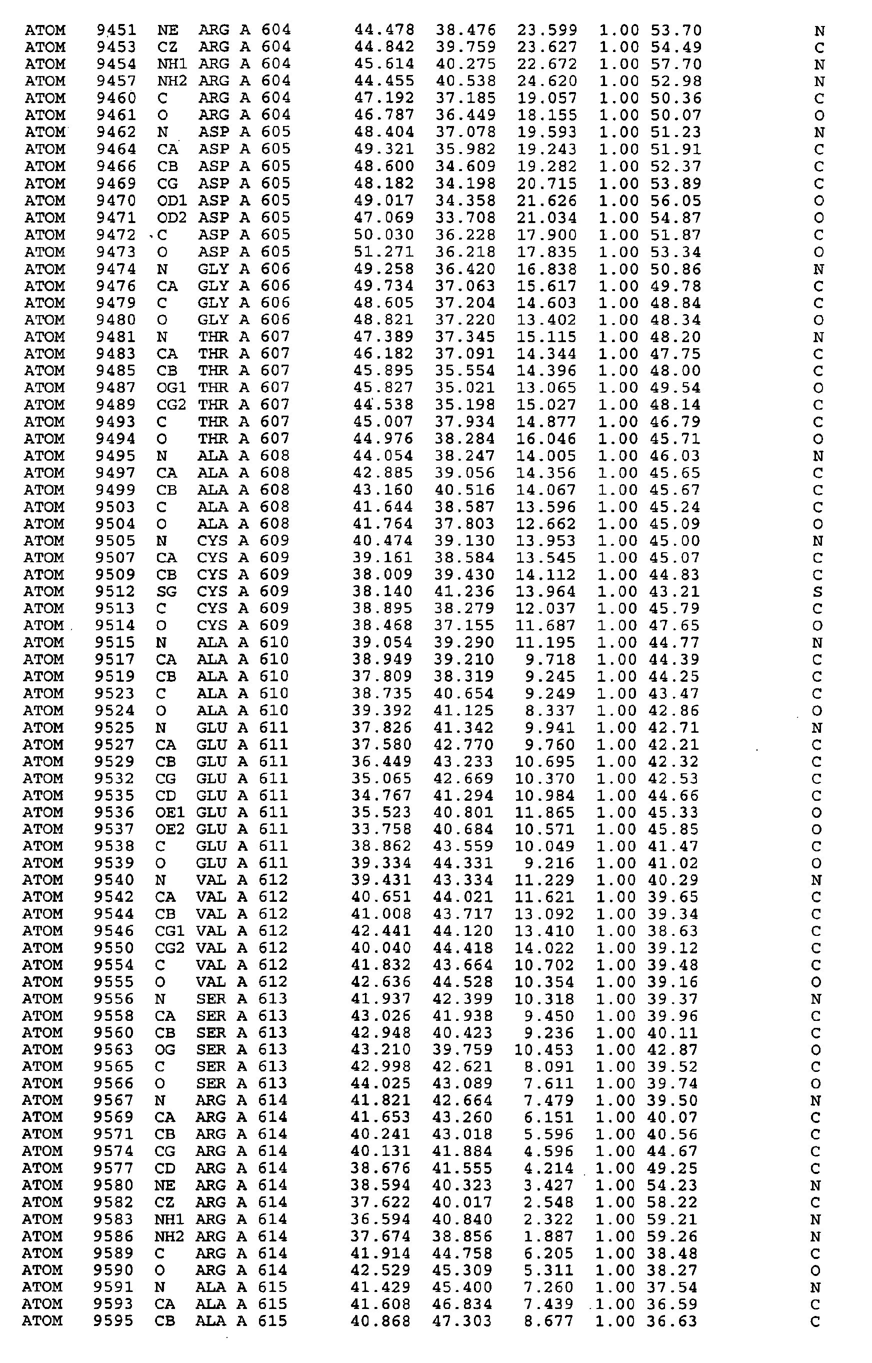

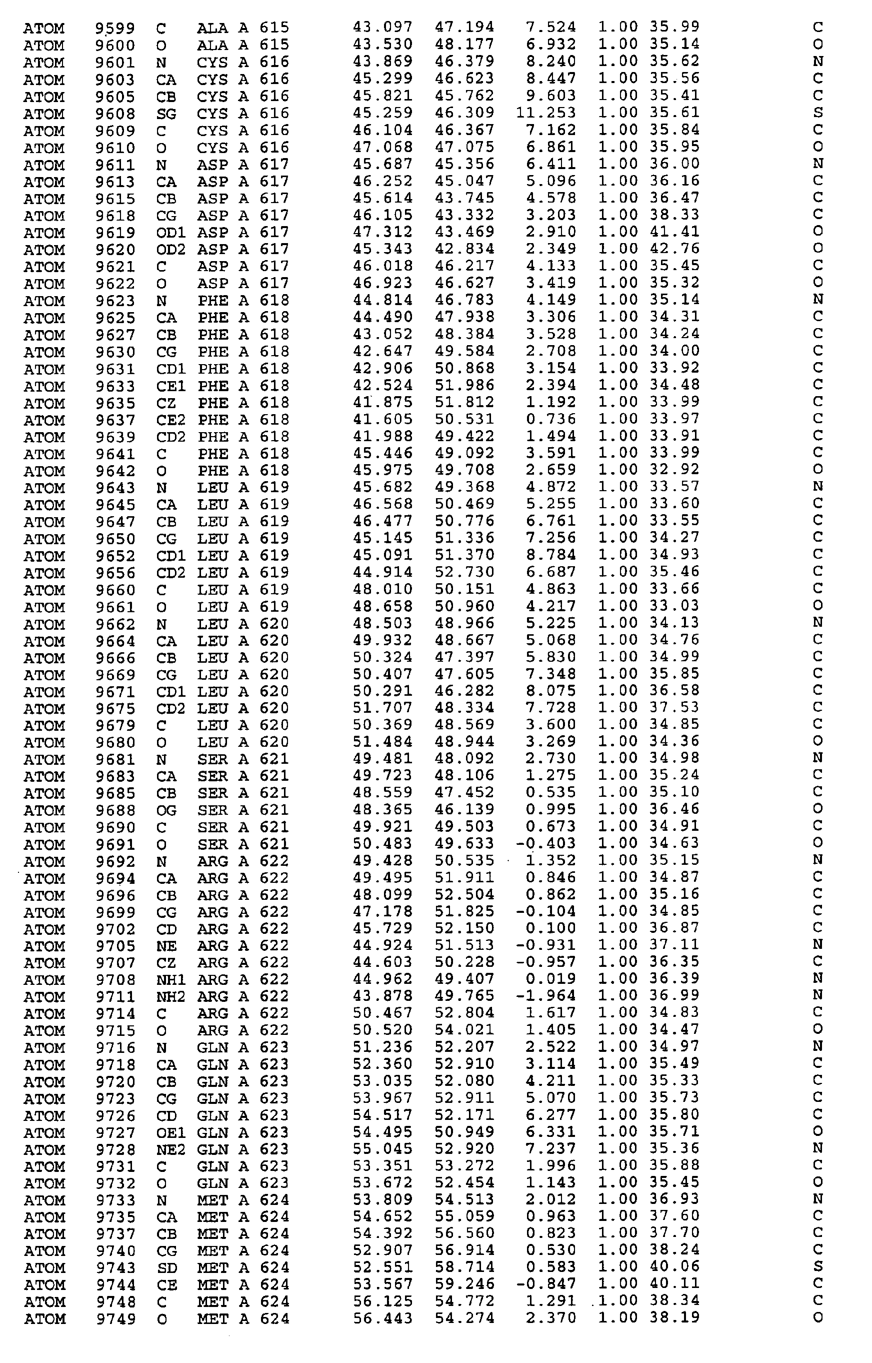

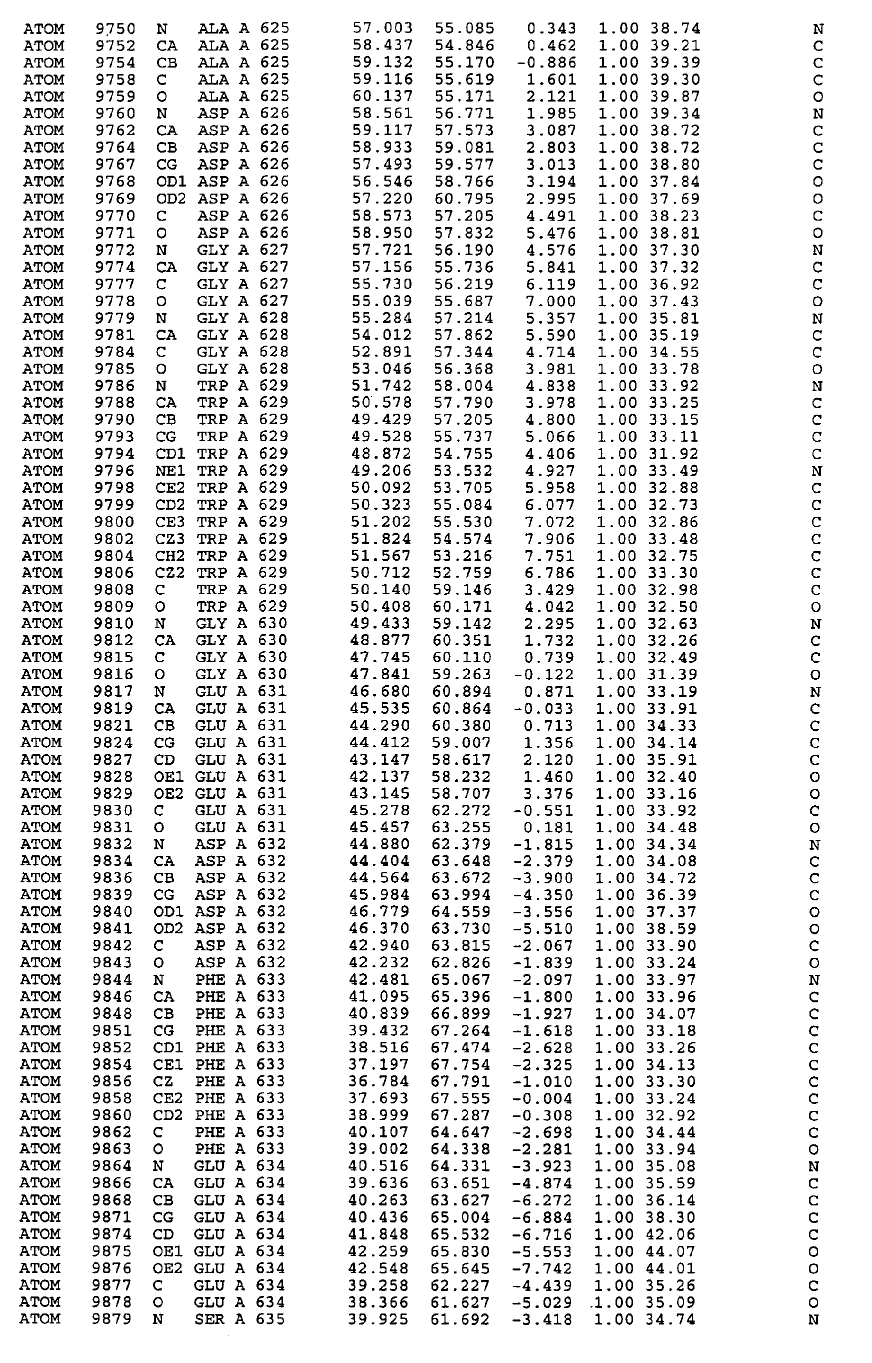

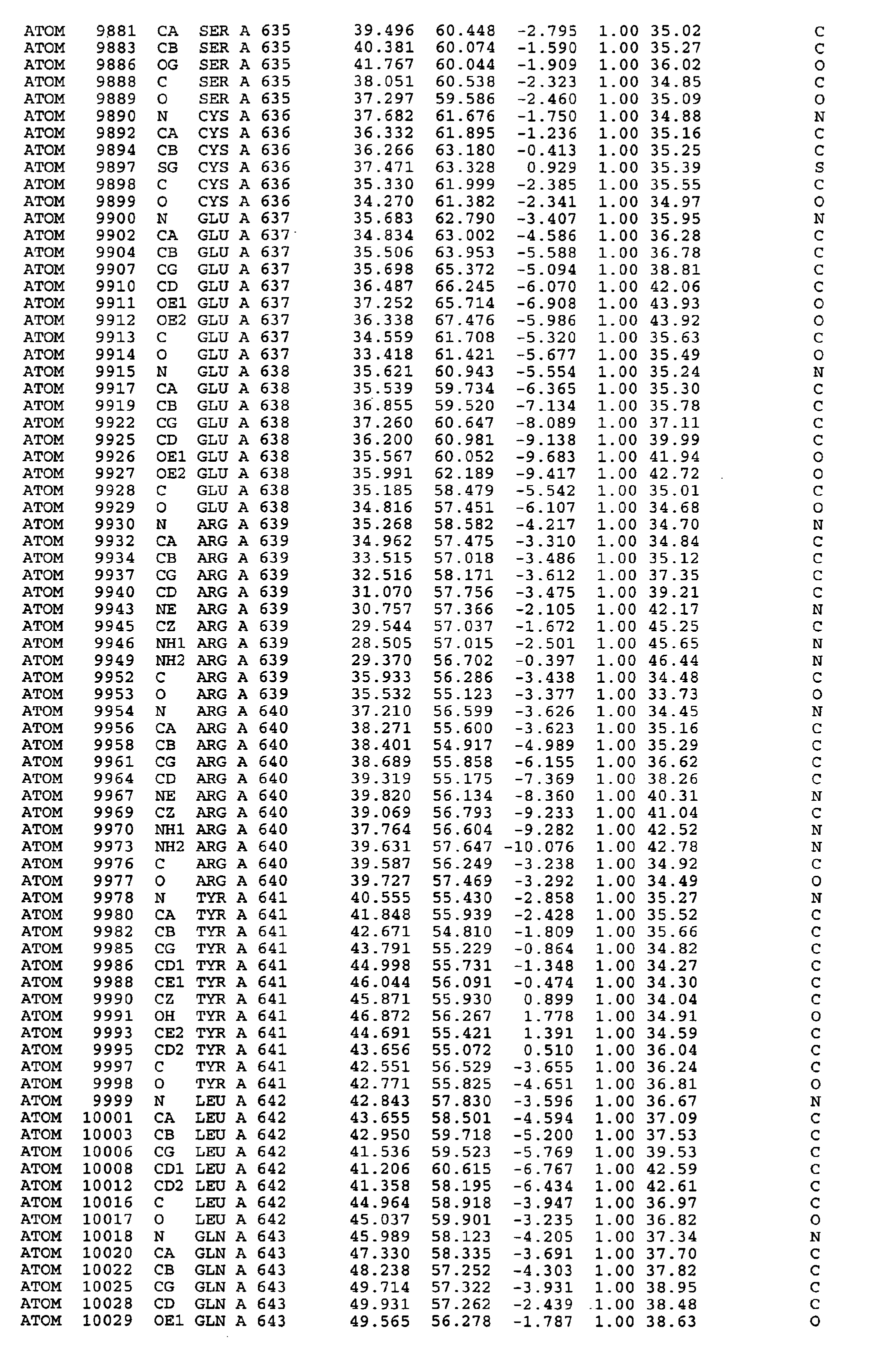

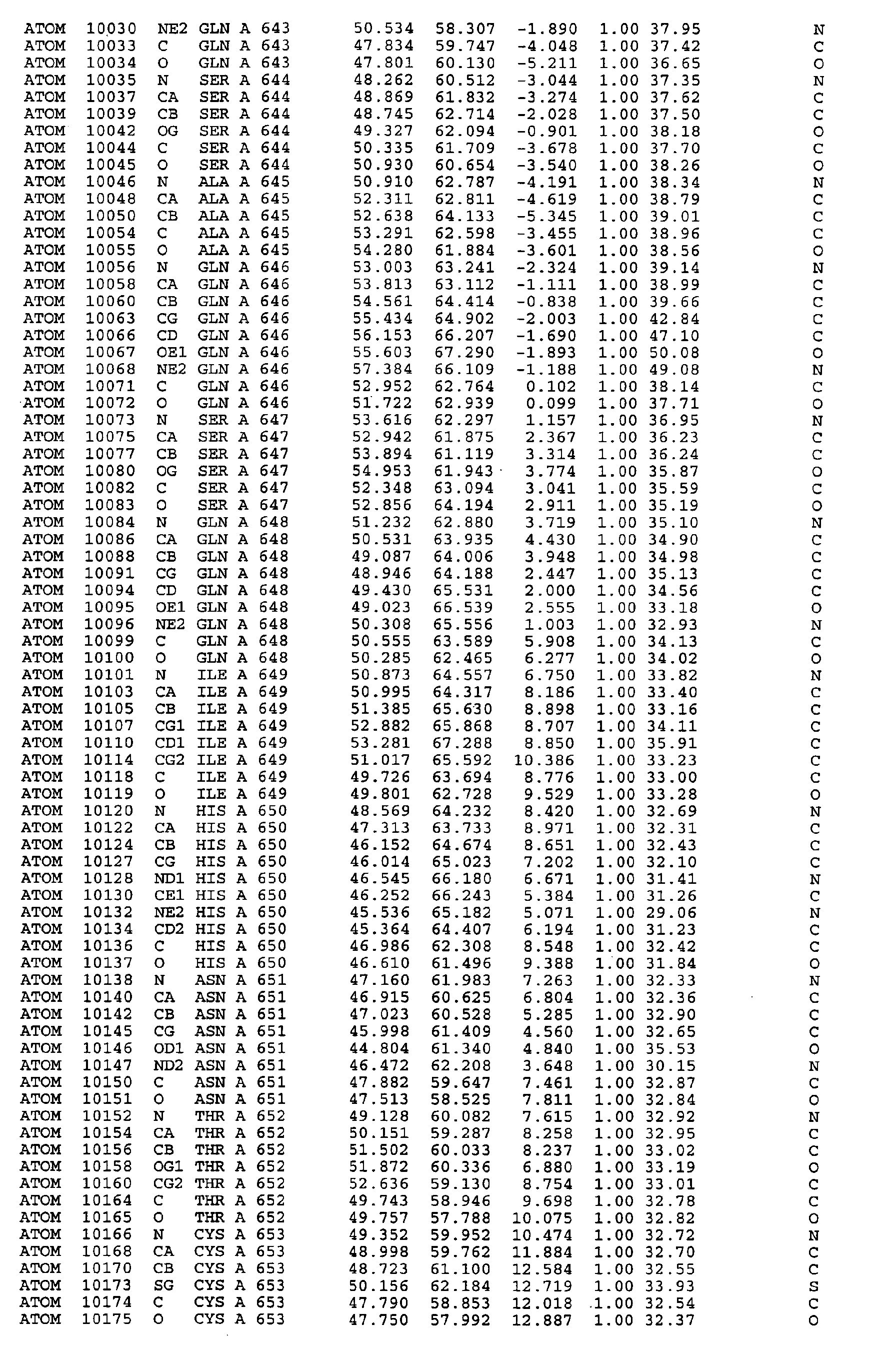

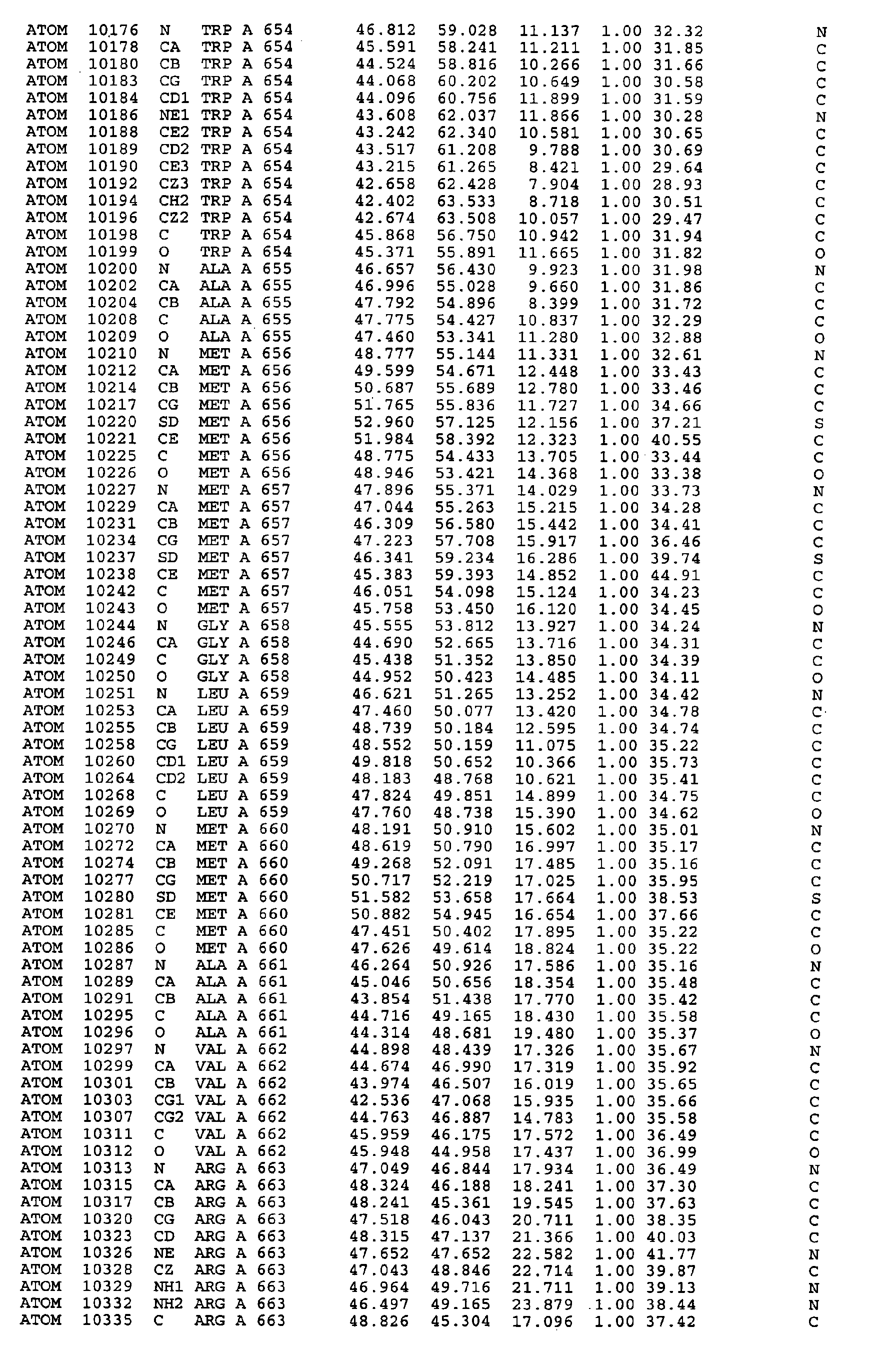

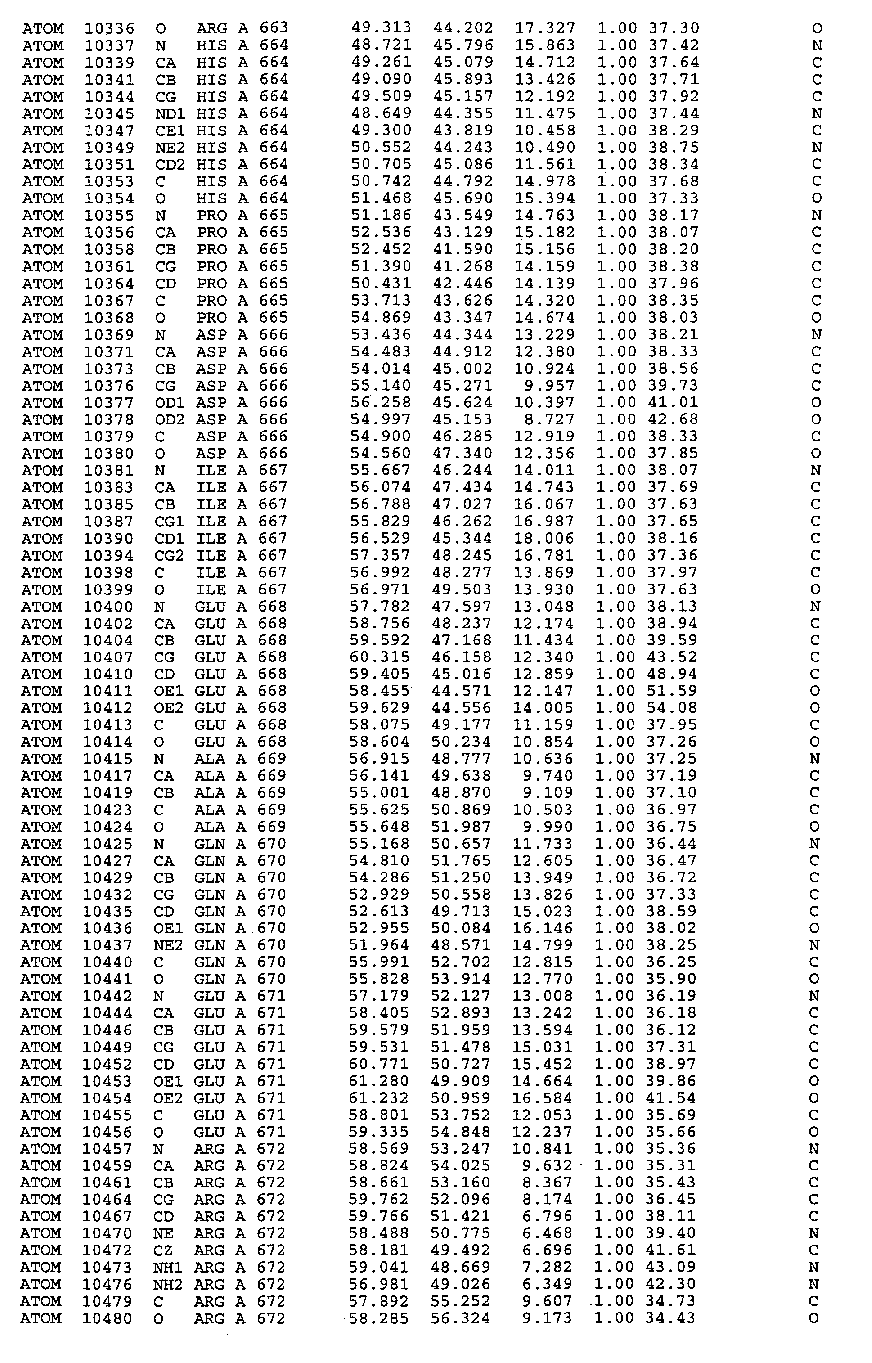

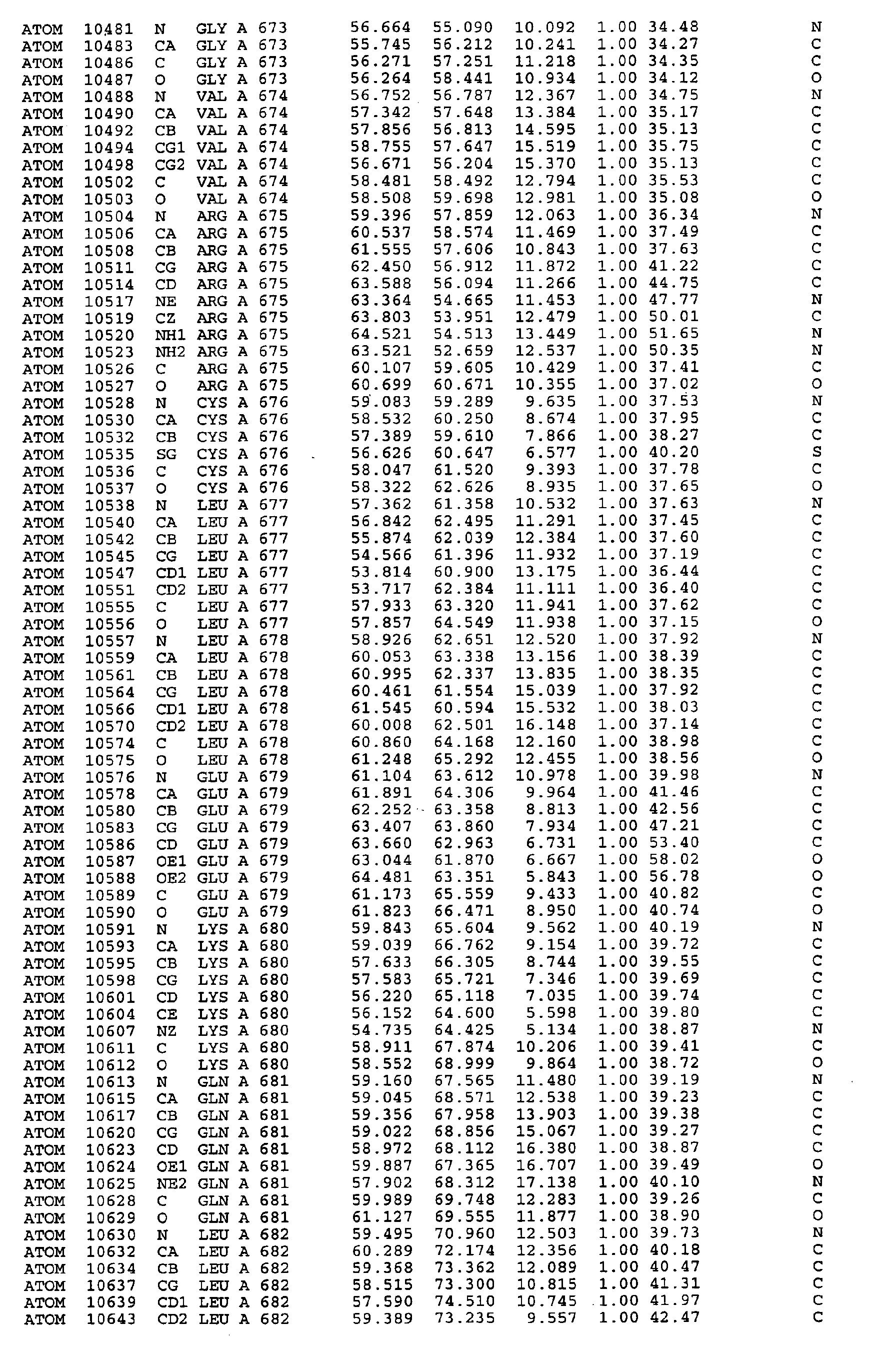

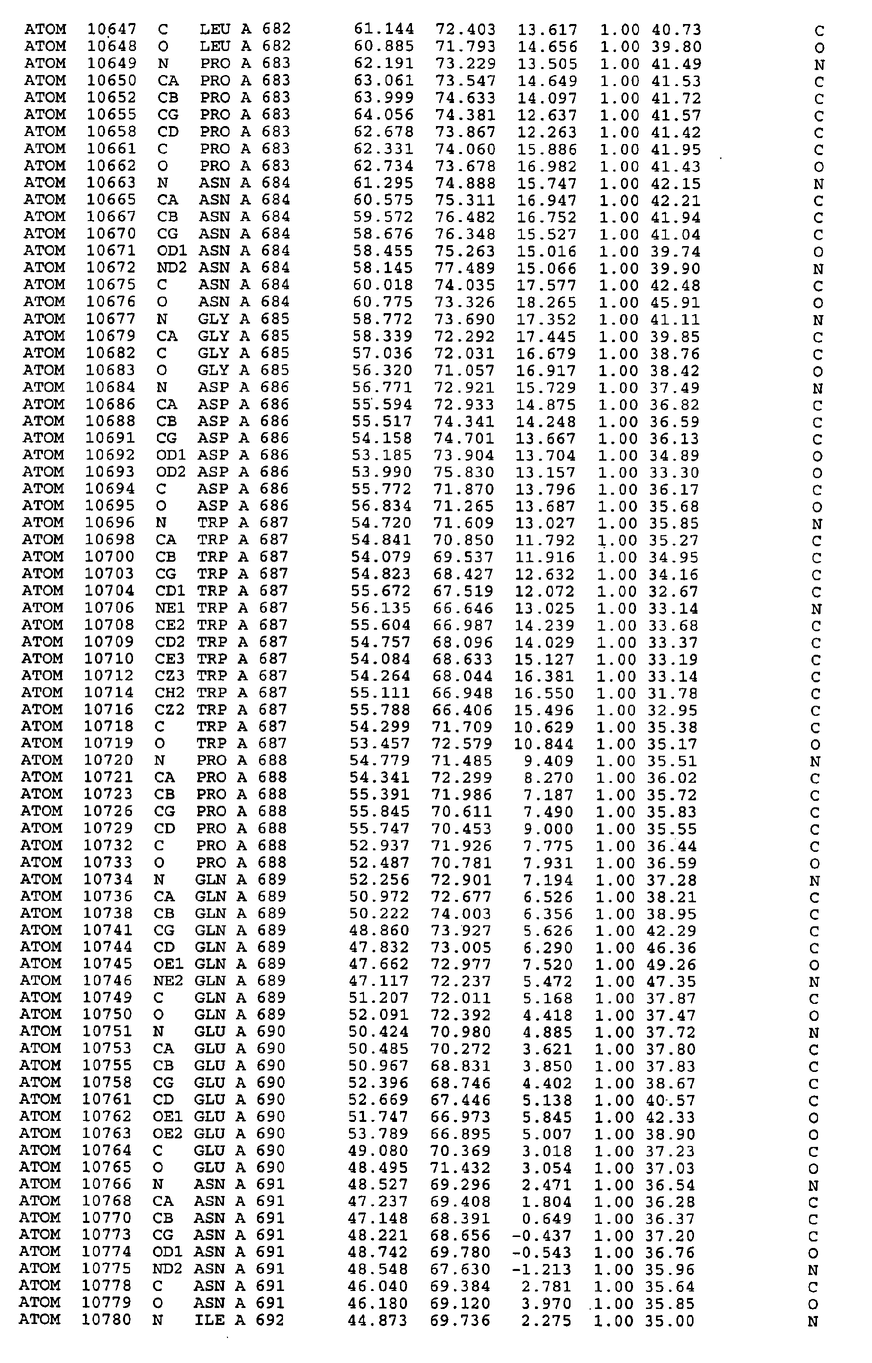

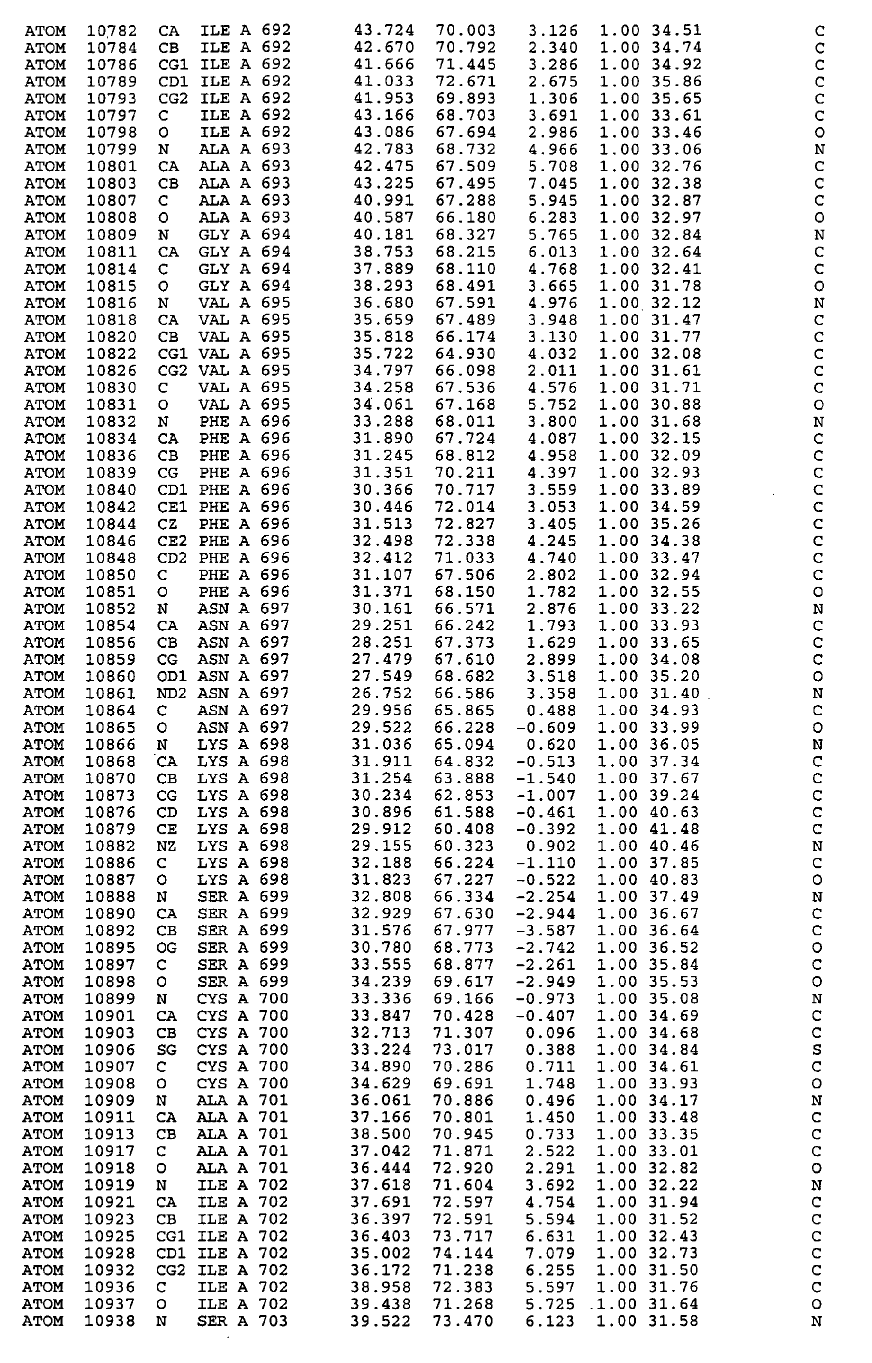

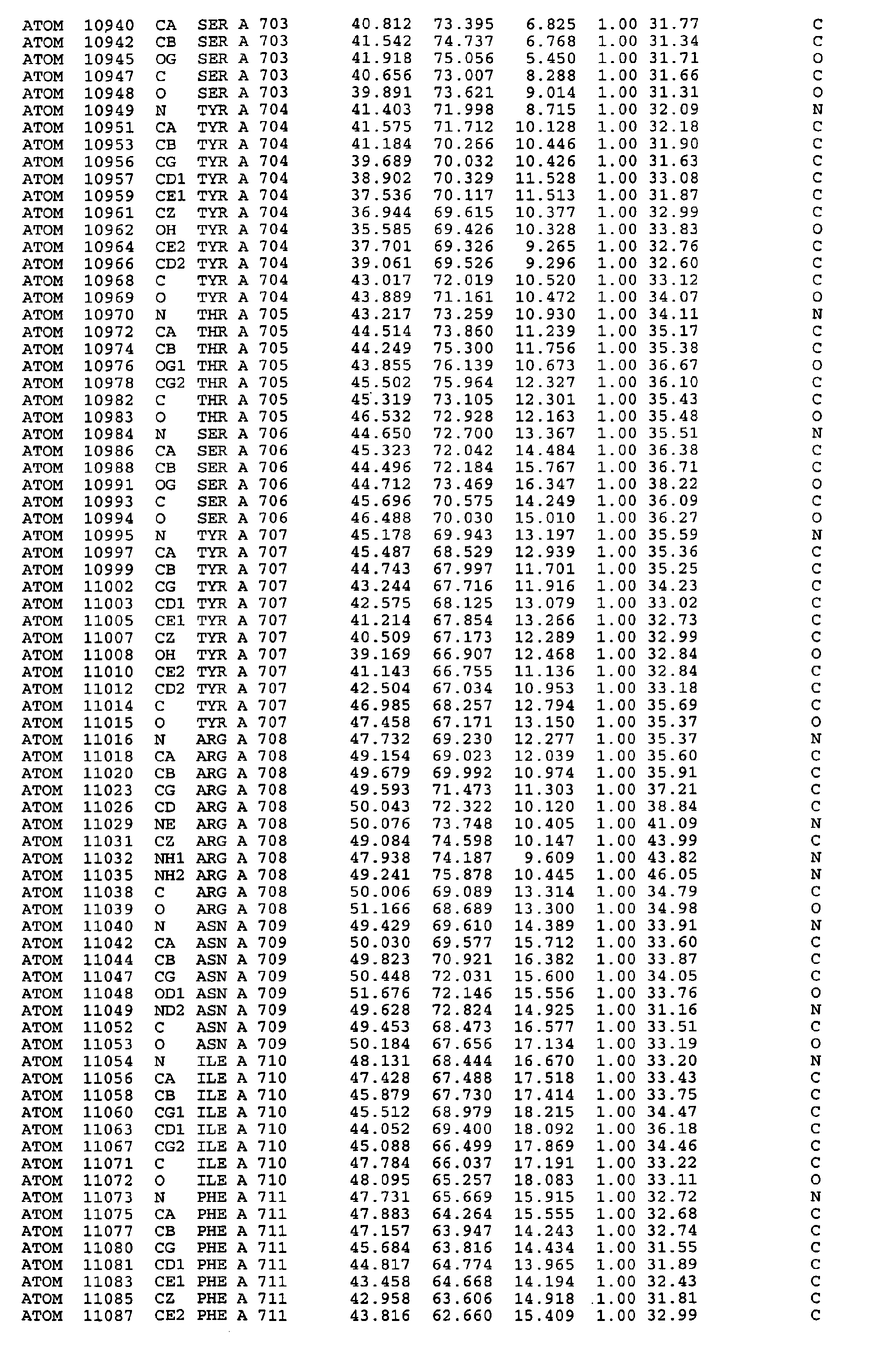

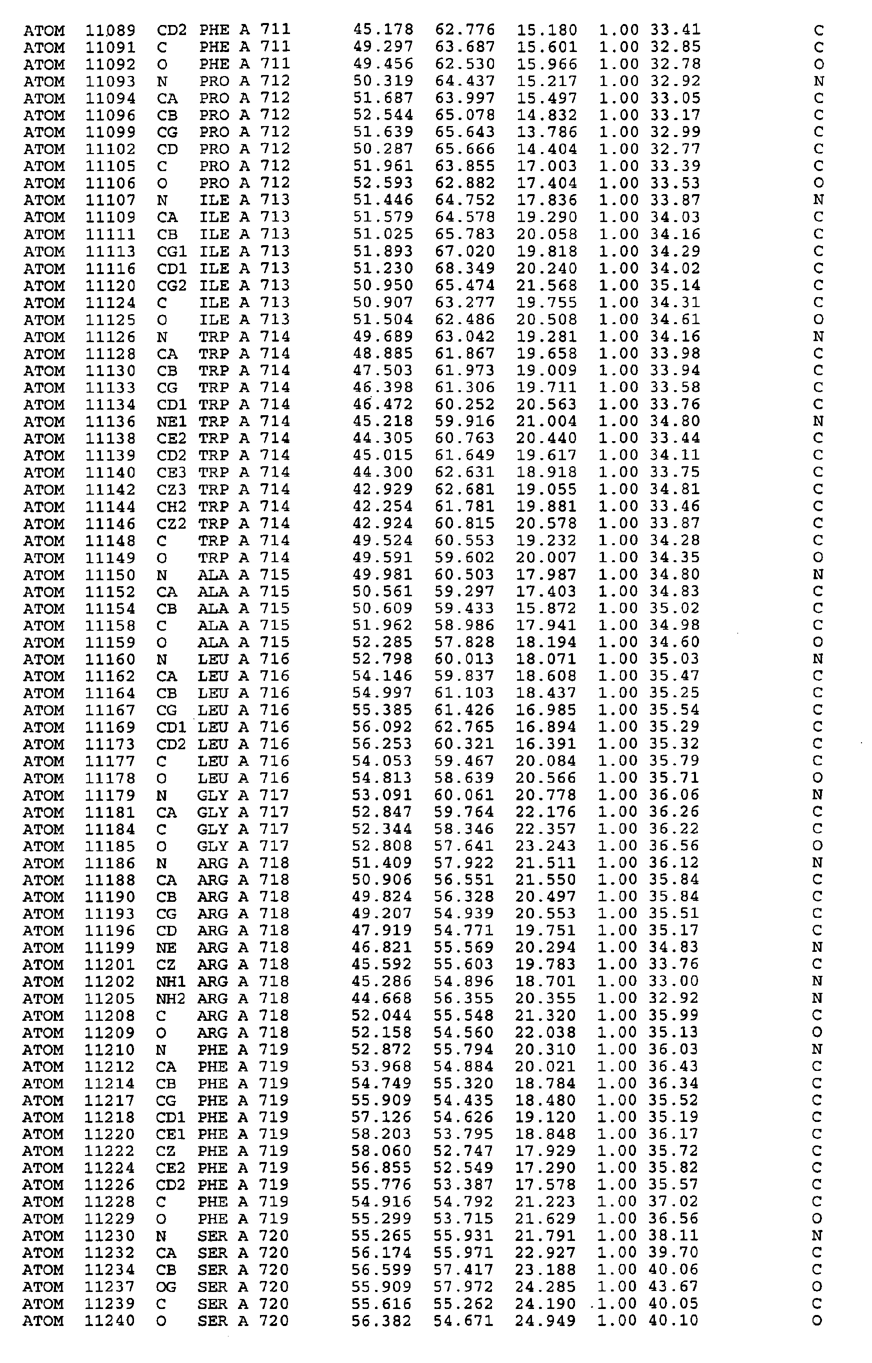

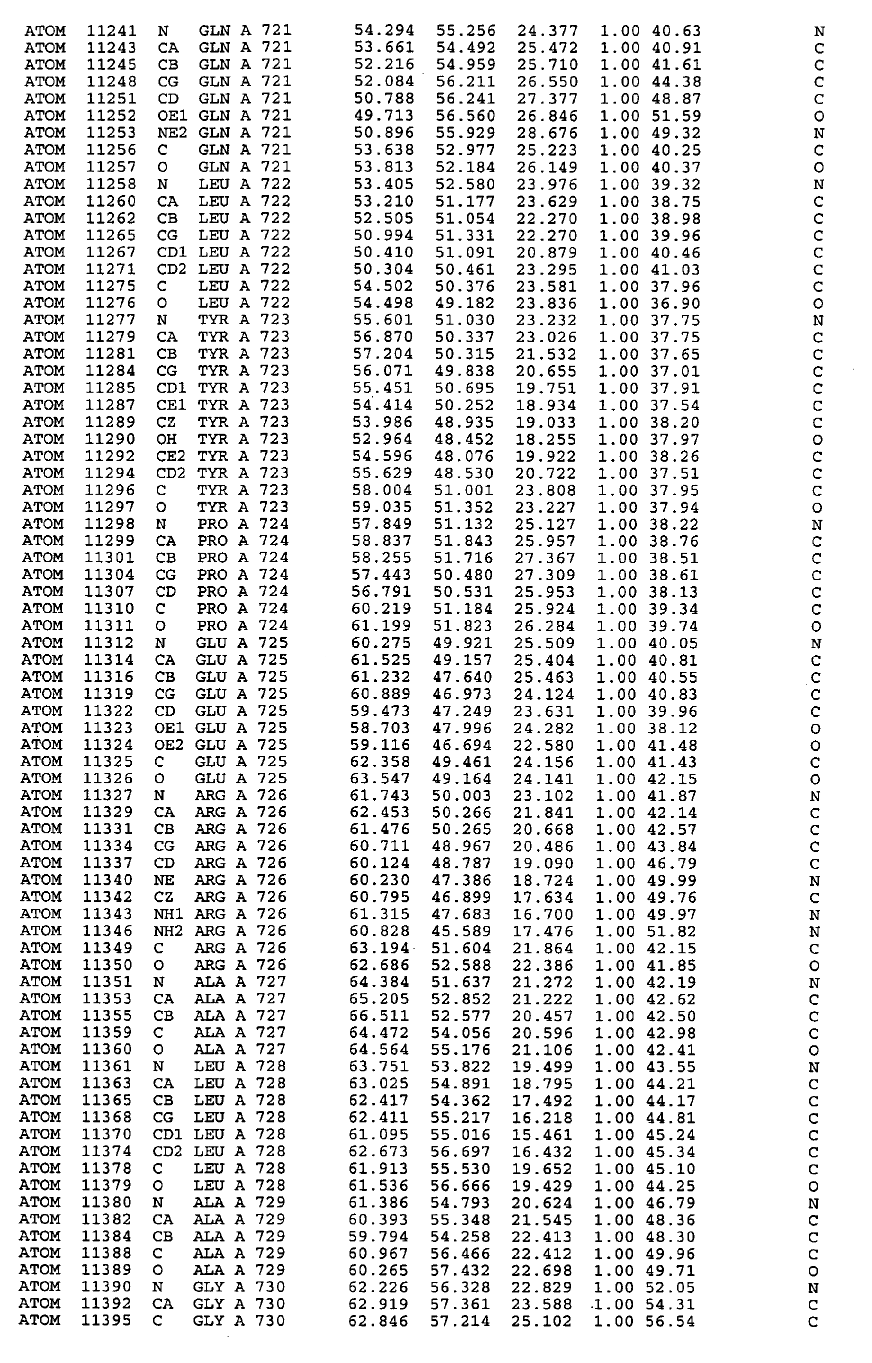

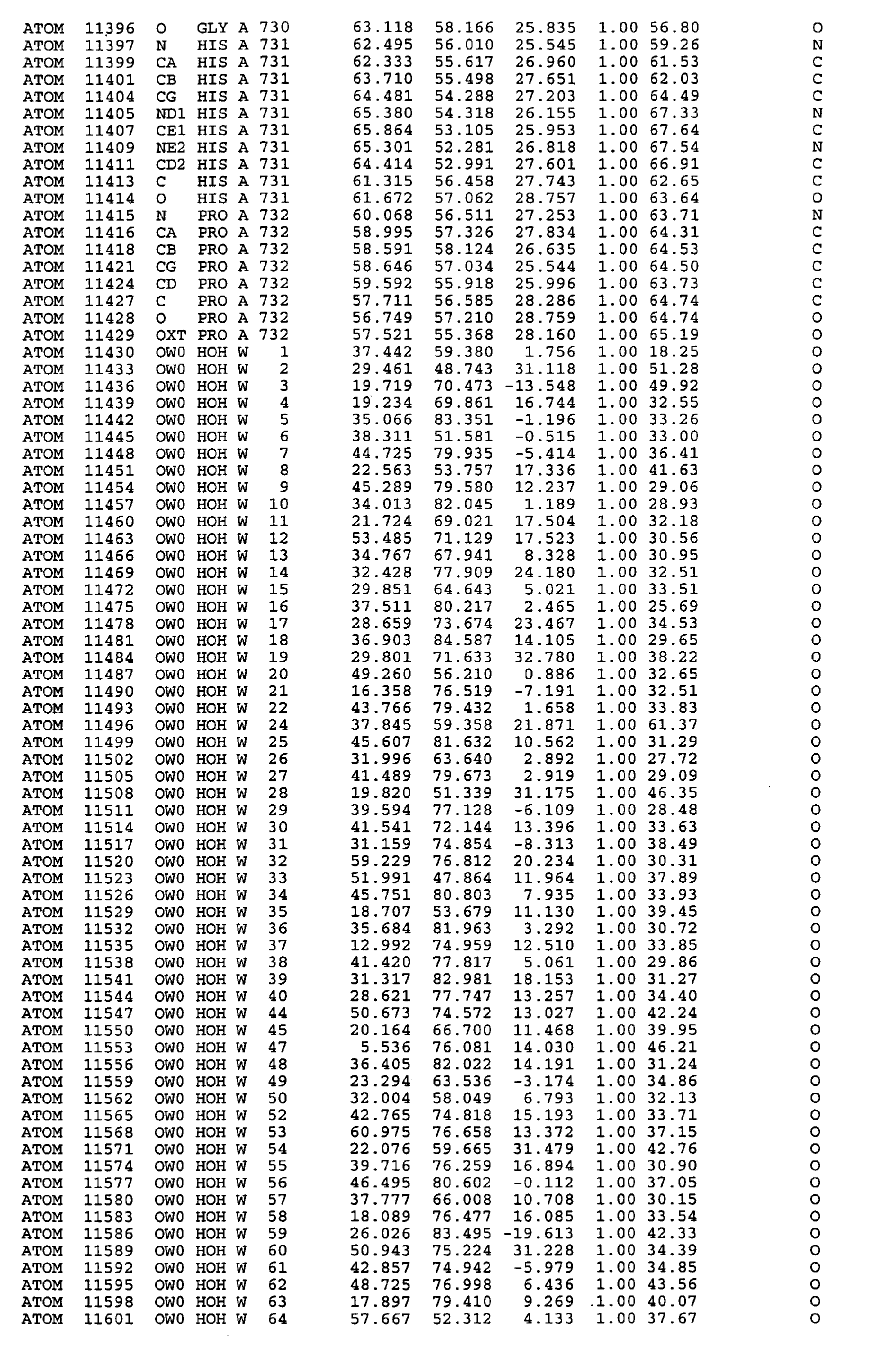

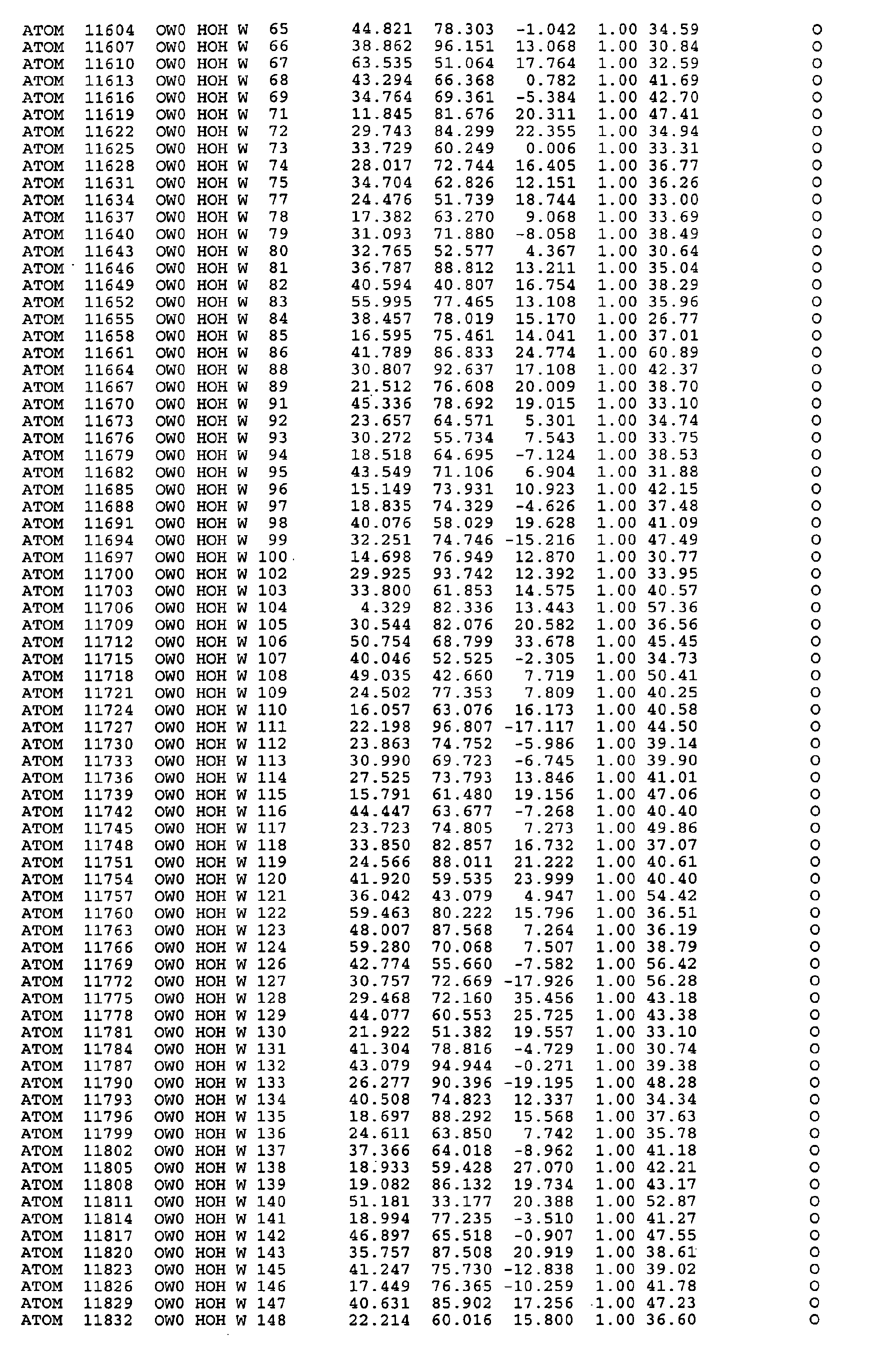

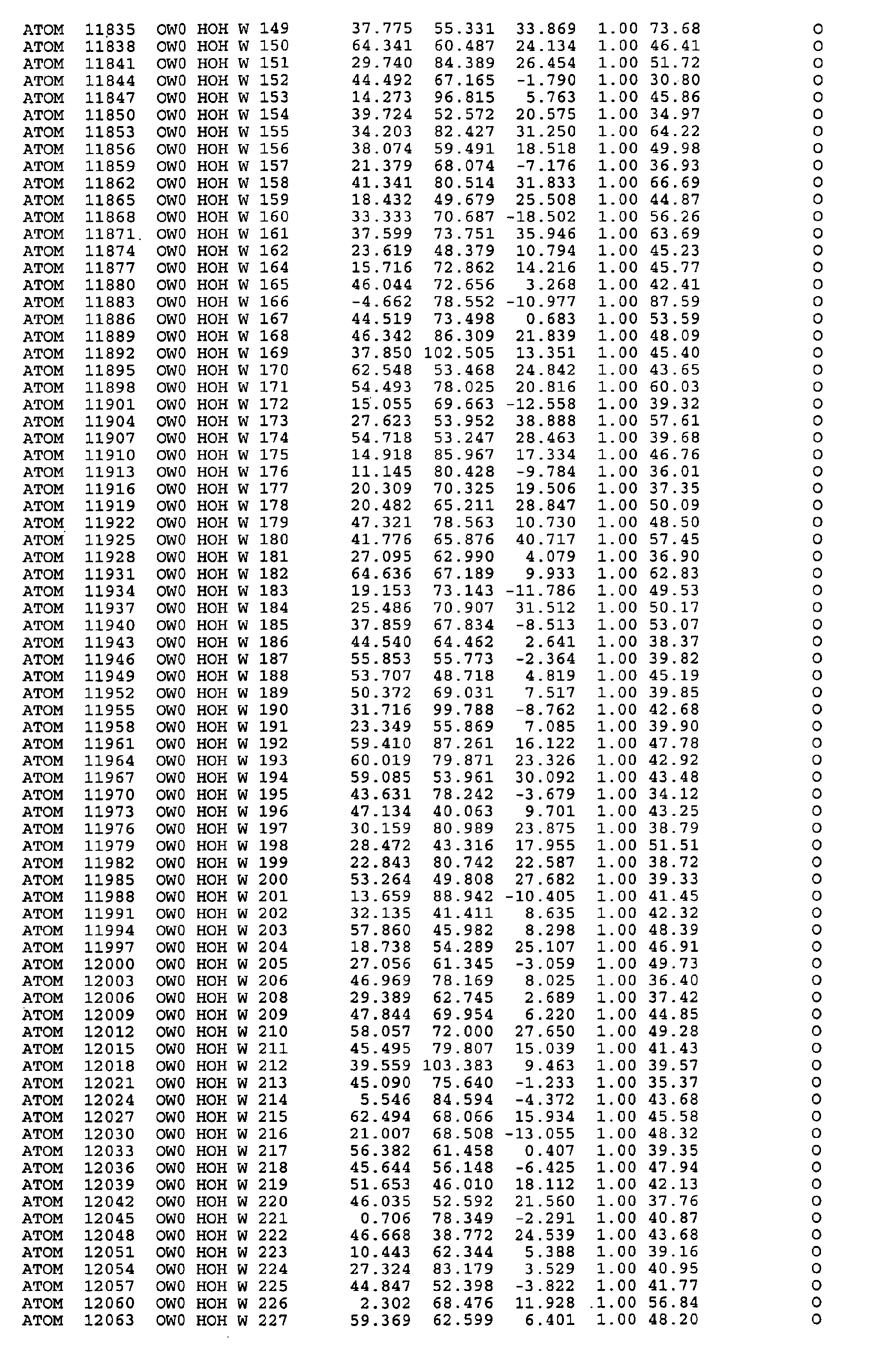

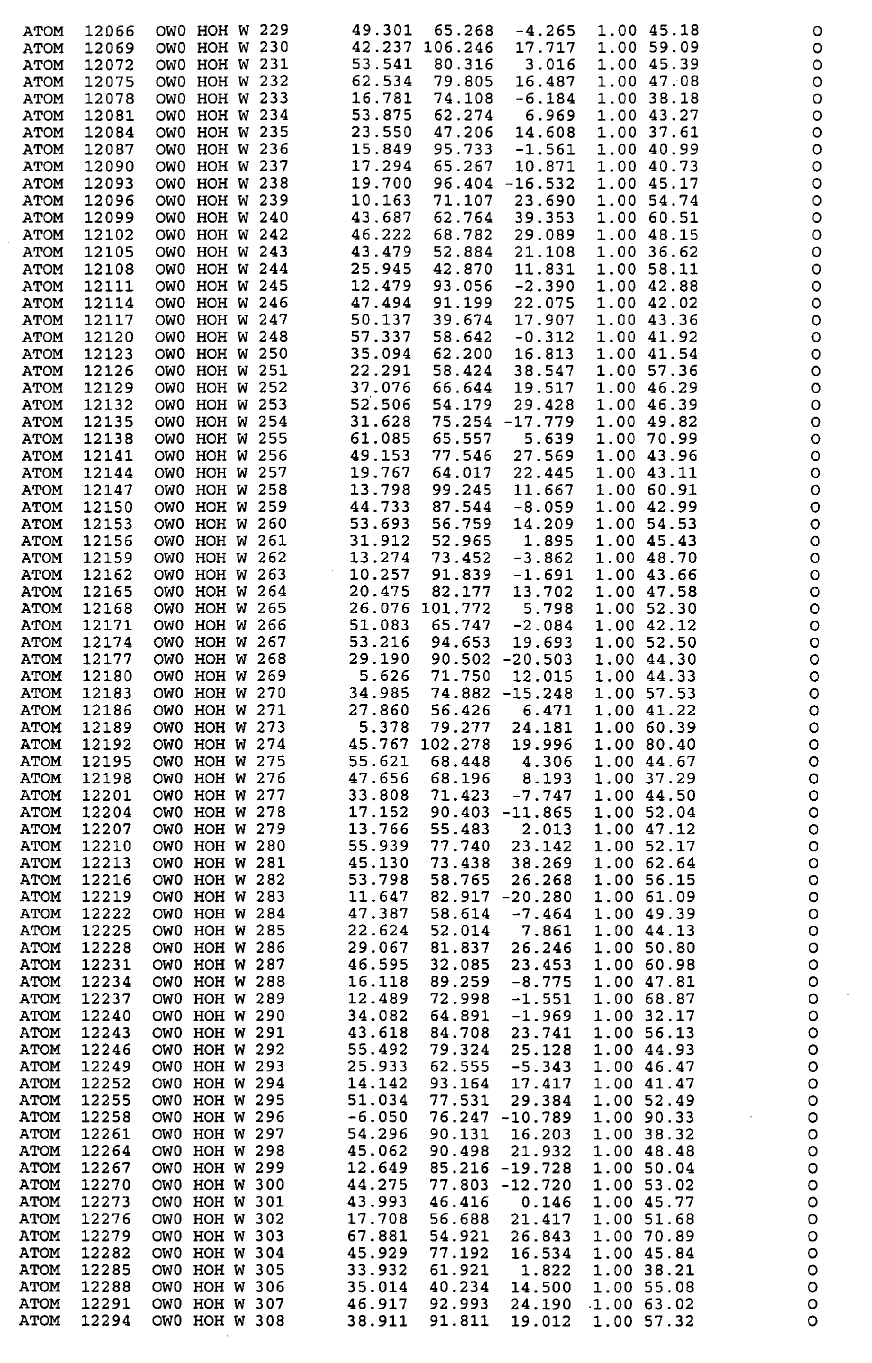

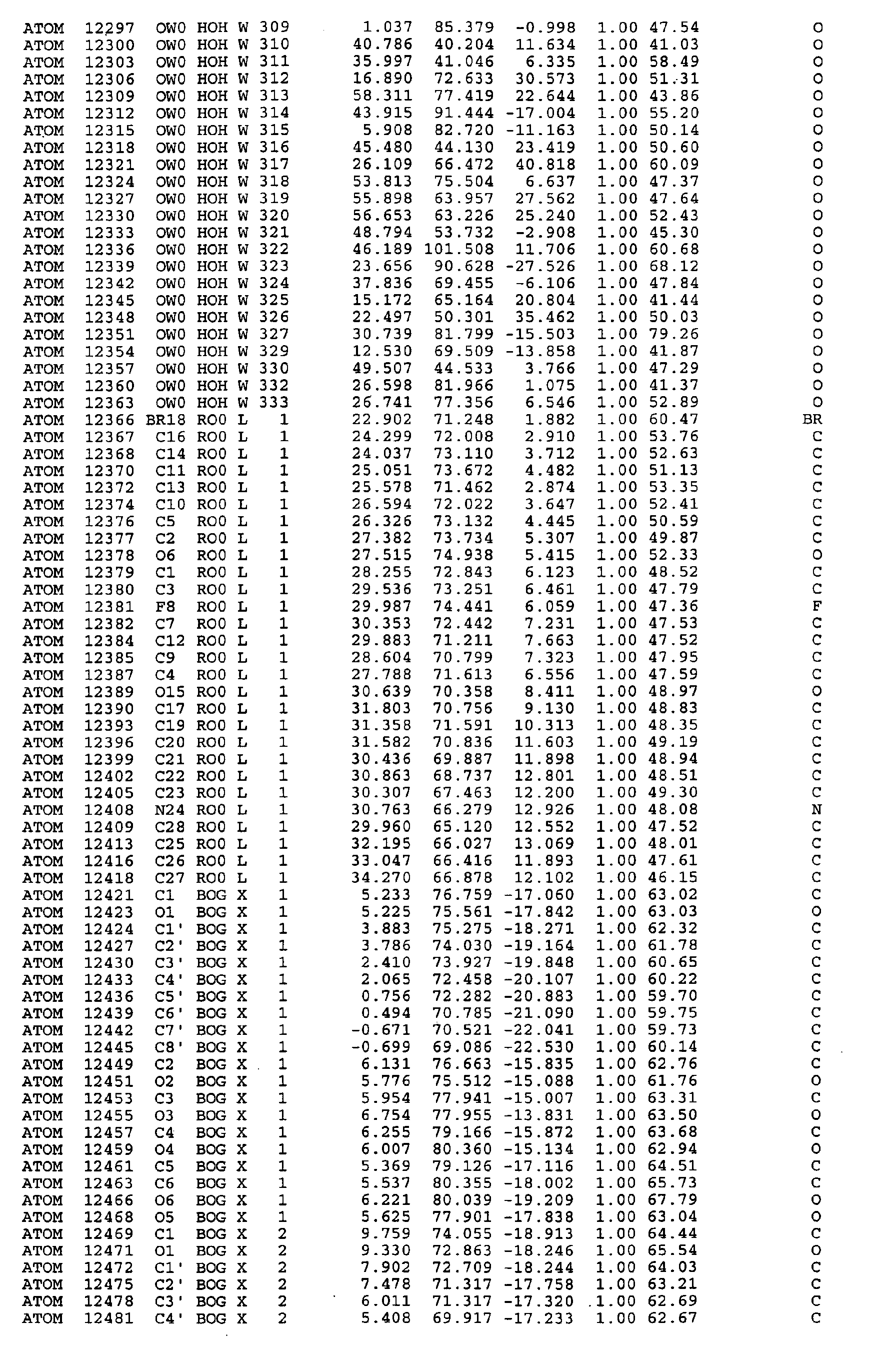

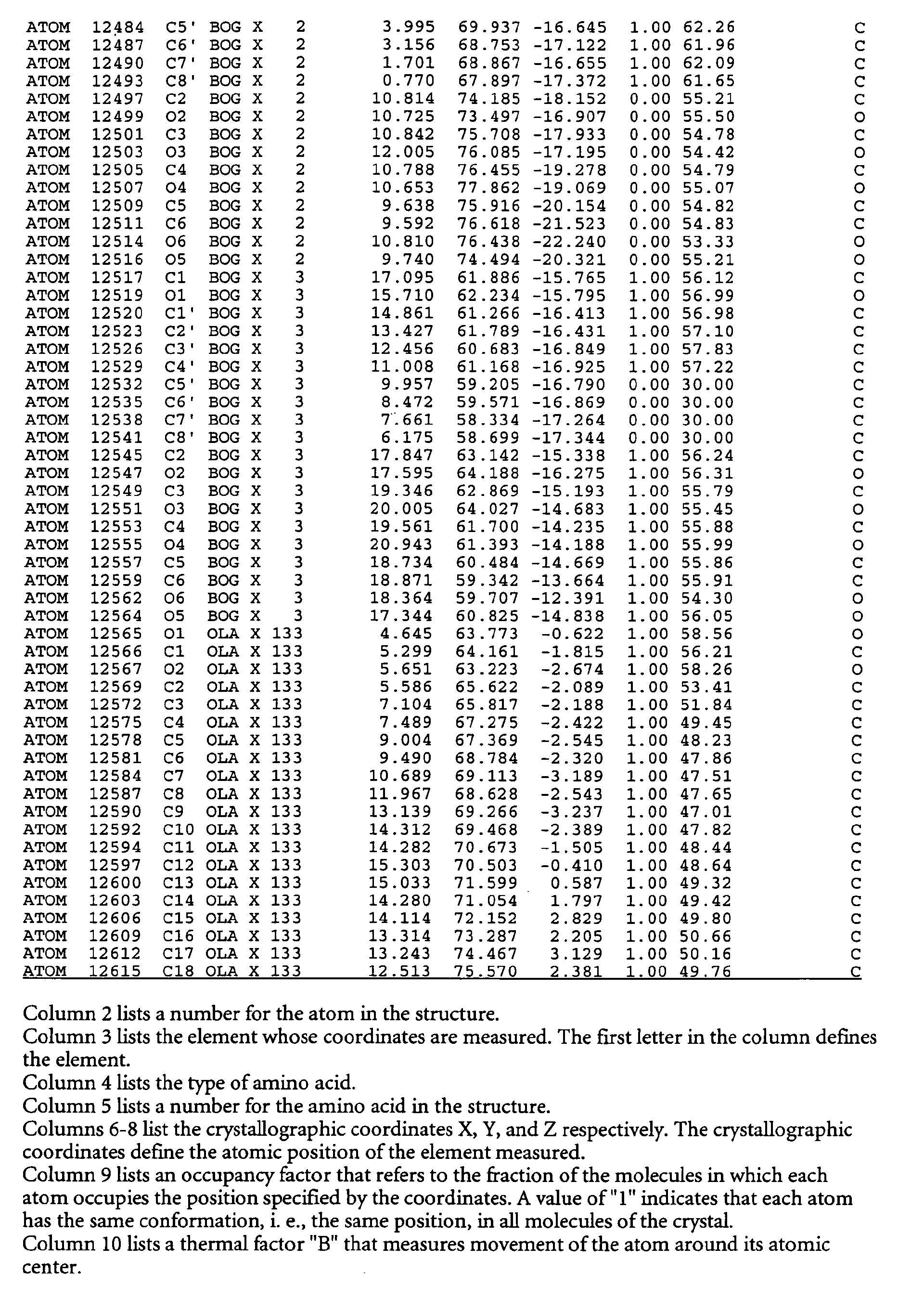

## Claims

1. A crystal form of mammalian OSC.

2. The crystal form according to claim 1, characterized as having a space group of C222₁ and one monomer of OSC in the asymmetric unit.

3. The crystal form according to claims 1 and 2, wherein the crystal has unit cell dimensions of:
a is from 186 to 194 A;
b is from 198 to 206 Å;
c is from 58 to 66 A;
and a C222₁ symmetry.

4. The crystal form according to any one of claims 1 to 3, **characterized by** the atomic structure coordinates of Table 3.

5. A co-crystal form of mammalian OSC and a ligand bound to its active site.

6. The co-crystal form according to claim 5, characterized as having a space group of C222₁ and one monomer of OSC in the asymmetric unit.

7. The co-crystal according to claim 6, wherein the co-crystal has unit cell dimensions of:
a is from 186 to 194 A;
b is from 198 to 206 A;
c is from 58 to 66 Å;
and a C222₁ symmetry.

8. A co-crystal of mammalian OSC and a ligand bound to an allosteric binding site.

9. The crystal form or co-crystal form according to anyone of claims 1 to 8 for the determination of the 3-D structure of mammalian OSC.

10. A method for crystallizing mammalian OSC, the method comprising providing a buffered, aqueous solution of pH 7.5 to 9.5 with a concentration of 2 mg/ml to 40 mg/ml of monodisperse mammalian OSC.

11. The method according to claim 10 comprising
(a) providing a buffered, aqueous solution of pH 7.5 to 9.5 with a concentration of 2 mg/ml to 40 mg/ml of mammalian OSC in β-OG micelles; and
(b) growing crystal forms using a buffered precipitant solution comprising between 2% and 25% of a small organic amphiphilic molecule, and between 10% and 35% of PEG.

12. The method according to claims 10 and 11, wherein mammalian OSC in step (a) is produced in *P. pastoris*.

13. A method for co-crystallizing mammalian OSC and an active site ligand, the method comprising
providing a buffered, aqueous solution of pH 7.5 to 9.5 with a concentration of 2 mg/ml to 40 mg/ml of monodisperse mammalian OSC and adding the active site ligand to the aqueous solution of mammalian OSC.

14. The method according to claim 13 comprising
(a) providing a buffered, aqueous solution of pH 7.5 to 9.5 with a concentration of 2 mg/ml to 40 mg/ml of mammalian OSC in β-OG micelles;
(b) adding the active site ligand to the aqueous solution of mammalian OSC; and
(c) growing co-crystal forms using a buffered precipitant solution comprising between 2% and 25% of a small organic amphiphilic molecule and between 10% and 35% of PEG.

15. The method according to claim 13 and 14, wherein mammalian OSC in step (a) is produced in P. pastoris.

16. A crystal form or a co-crystal form of mammalian OSC produced by the methods according to any one of claims 10 to 15.

17. A method for determining the three-dimensional structure of a crystal form of mammalian OSC to a resolution of 3.5 A or better, the method comprising
(a) crystallizing mammalian OSC; and
(b) analyzing the crystal form of mammalian OSC by X-ray diffraction to determine the three-dimensional structure of the crystal form of mammalian OSC, whereby the three-dimensional structure of a crystal form of mammalian OSC is determined to a resolution of 3.5 A or better.

18. A machine-readable data storage medium comprising a data storage material encoded with machine readable data which, when using a machine programmed with instructions for using said data, displays a graphical three-dimensional representation of a molecule or molecular complex comprising at least a portion of mammalian OSC comprising the amino acids of SEQ ID NO: 5, comprising the ligand binding active site being defined by a set of points having a root mean square deviation of less than about 1.5Å from points representing the alpha carbon backbone atoms of said amino acids as represented by structure coordinates listed in Table 3.

19. A method for identifying a compound that interacts with OSC, comprising the steps of
(a) generating a three-dimensional model of OSC using the structure coordinates as listed in Table 3, a root mean square deviation from the alpha carbon backbone atoms of said amino acids of less than 1.5Å; and
(b) employing said three-dimensional model to design or select a compound that interacts with OSC.

20. The method according to claim 19, further comprising the steps of
(c) obtaining the identified compound; and
(d) contacting the obtained compound with OSC in order to determine the effect the compound has on OSC activity.

21. The method according to claims 19 and 20, wherein the compound interacts with the active site of OSC.

22. The method according to claims 19 and 20, wherein the compound interacts with an allosteric binding site of OSC.

23. The method according to claims 19 to 22, wherein the compound is an inhibitor of OSC activity.

24. The method according to claims 19 to 23, wherein the method is a computer-assisted method.

25. A compound identified by the methods according to claims 19 to 24.

26. A pharmaceutical composition comprising the compound of claim 25 and a pharmaceutically acceptable carrier.

27. Use of a compound according to claim 25 for the manufacture of a medicament for the treatment and/or prevention of diseases which are associated with OSC comprising hypercholesterolemia, hyperlipemia, arteriosclerosis, vascular diseases, mycoses, parasite infections and gallstones, and/or treatment and/or prophylaxis of impaired glucose tolerance, diabetes, tumors and/or hyperproliferative disorders, preferably for the treatment and/or prophylaxis of hypercholesterolemia and/or hyperlipemia.

28. Use of a crystal form or a co-crystal form according to claims 1 to 9 and 16 for the identification and/or design of inhibitors of OSC activity.

29. The novel crystal forms, methods, compounds, compositions and uses substantially as herein before described especially with reference to the foregoing Examples.


SEQUENCE LISTING

<110>  F. Hoffmann - La Roche AG

<120>  Crystal structure of OSC

<130>  Case 21797

<160>  7

<170>  PatentIn version 3.1

<210>  1
<211>  39
<212>  DNA
<213>  Artificial

<400>  1
gtacctttcg aaacaatgac ggagggcacg tgtctgcgg                              39

<210>  2
<211>  29
<212>  DNA
<213>  Artificial

<400>  2
gcctctagat caggggtggc cagcaaggg                                         29

<210>  3
<211>  29
<212>  DNA
<213>  Artificial

<400>  3
ggctctagat caggggtggc cagcaaggg                                         29

<210>  4
<211>  2235
<212>  DNA
<213>  Homo sapiens

<400>  4

atgacggagg gcacgtgtct gcggcgccga gggggcccct acaagaccga gcccgccacc 60
gacctcggcc gctggcgact caactgcgag aggggccggc agacgtggac ctacctgcag 120
gacgagcgcg ccggccgcga gcagaccggc ctggaagcct acgccctggg gctggacacc 180
aagaattact ttaaggactt gcccaaagcc cacaccgcct ttgagggggc tctgaacggg 240
atgacatttt acgtggggct gcaggctgag gatgggcact ggacgggtga ttatggtggc 300
ccacttttcc tcctgccagg cctcctgatc acttgccacg tggcacgcat ccctctgcca 360
gccggataca gagaagagat tgtgcggtac ctgcggtcag tgcagctccc tgacggtggc 420
tggggcctgc acattgagga taagtccacc gtgtttggga ctgcgctcaa ctatgtgtct 480
ctcagaattc tgggtgttgg gcctgacgat cctgacctgg tacgagcccg gaacattctt 540
cacaagaaag gtggtgctgt ggccatcccc tcctggggga agttctggct ggctgtcctg 600
aatgtttaca gctgggaagg cctcaatacc ctgttcccag agatgtggct gtttcctgac 660
tgggcaccgg cacacccctc cacactctgg tgccactgcc ggcaggtgta cctgcccatg 720
agctactgct acgccgttcg gctgagtgcc gcggaagacc cgctggtcca gagcctccgc 780
caggagctct atgtggagga cttcgccagc attgactggc tggcgcagag gaacaacgtg 840
gcccccgacg agctgtacac gccccacagc tggctgctcc gcgtggtata tgcgctcctc 900
aacctgtatg agcaccacca cagtgcccac ctgcggcagc gggccgtgca gaagctgtat 960
gaacacattg tggccgacga ccgattcacc aagagcatca gcatcggccc gatctcgaaa 1020
accatcaaca tgcttgtgcg ctggtatgtg gacgggcccg cctccactgc cttccaggag 1080
catgtctcca gaatcccgga ctatctctgg atgggccttg acggcatgaa aatgcagggc 1140
accaacggct cacagatctg ggacaccgca ttcgccatcc aggctctgct tgaggcgggc 1200
gggcaccaca ggcccgagtt ttcgtcctgc ctgcagaagg ctcatgagtt cctgaggctc 1260
tcacaggtcc cagataaccc tcccgactac cagaagtact accgccagat gcgcaagggt 1320
ggcttctcct tcagtacgct ggactgcggc tggatcgttt ctgactgcac ggctgaggcc 1380
ttgaaggctg tgctgctcct gcaggagaag tgtccccatg tcaccgagca catccccaga 1440
gaacggctct gcgatgctgt ggctgtgctg ctgaacatga gaaatccaga tggagggttc 1500
gccacctatg agaccaagcg tggggggcac ttgctggagc tgctgaaccc ctcggaggtc 1560
ttcggggaca tcatgattga ctacacctat gtggagtgca cctcagccgt gatgcaggcg 1620
cttaagtatt tccacaagcg tttcccggag cacagggcag cggagatccg ggagaccctc 1680
acgcagggct tagagttctg tcggcggcag cagagggccg atggctcctg ggaaggctcc 1740
tggggagttt gcttcaccta cggcacctgg tttggcctgg aggccttcgc ctgtatgggg 1800
cagacctacc gagatgggac tgcctgtgca gaggtctccc gggcctgtga cttcctgctg 1860
tcccggcaga tggcagacgg aggctggggg gaggactttg agtcctgcga ggagcggcgt 1920
tatgtgcaga gtgcccagtc ccagatccat aacacatgct gggccatgat ggggctgatg 1980
gccgttcggc atcctgacat cgaggcccag gagagaggag tccggtgtct acttgagaaa 2040
cagctcccca atggcgactg gccgcaggaa aacattgctg gggtcttcaa caagtcctgt 2100
gccatctcct acacgagcta caggaacatc ttccccatct gggccctcgg ccgcttctcc 2160
cagctgtacc ctgagagagc ccttgctggc cacccccagc tgtaccctga gagagccctt 2220
gctggccacc cctga 2235

<210> 5.

<211> 732

<212> PRT

<213> Homo sapiens

<400> 5

Met Thr Glu Gly Thr Cys Leu Arg Arg Arg Gly Gly Pro Tyr Lys Thr
1               5                   10                  15

Glu Pro Ala Thr Asp Leu Gly Arg Trp Arg Leu Asn Cys Glu Arg Gly
            20                  25                  30

Arg Gln Thr Trp Thr Tyr Leu Gln Asp Glu Arg Ala Gly Arg Glu Gln
        35                  40                  45

Thr Gly Leu Glu Ala Tyr Ala Leu Gly Leu Asp Thr Lys Asn Tyr Phe
    50                  55                  60

Lys Asp Leu Pro Lys Ala His Thr Ala Phe Glu Gly Ala Leu Asn Gly
65                  70                  75                  80

Met Thr Phe Tyr Val Gly Leu Gln Ala Glu Asp Gly His Trp Thr Gly
                85                  90                  95

Asp Tyr Gly Gly Pro Leu Phe Leu Leu Pro Gly Leu Leu Ile Thr Cys
            100                 105                 110

His Val Ala Arg Ile Pro Leu Pro Ala Gly Tyr Arg Glu Glu Ile Val
        115                 120                 125

Arg Tyr Leu Arg Ser Val Gln Leu Pro Asp Gly Gly Trp Gly Leu His
    130                 135                 140

Ile Glu Asp Lys Ser Thr Val Phe Gly Thr Ala Leu Asn Tyr Val Ser
145                 150                 155                 160

Leu Arg Ile Leu Gly Val Gly Pro Asp Asp Pro Asp Leu Val Arg Ala
                165                 170                 175

Arg Asn Ile Leu His Lys Lys Gly Gly Ala Val Ala Ile Pro Ser Trp
            180                 185                 190

Gly Lys Phe Trp Leu Ala Val Leu Asn Val Tyr Ser Trp Glu Gly Leu
        195                 200                 205

Asn Thr Leu Phe Pro Glu Met Trp Leu Phe Pro Asp Trp Ala Pro Ala
    210                 215                 220

His Pro Ser Thr Leu Trp Cys His Cys Arg Gln Val Tyr Leu Pro Met
225                 230                 235                 240

Ser Tyr Cys Tyr Ala Val Arg Leu Ser Ala Ala Glu Asp Pro Leu Val
                245                 250                 255

Gln Ser Leu Arg Gln Glu Leu Tyr Val Glu Asp Phe Ala Ser Ile Asp

            260                 265                 270

Trp Leu Ala Gln Arg Asn Asn Val Ala Pro Asp Glu Leu Tyr Thr Pro
        275                 280                 285

His Ser Trp Leu Leu Arg Val Val Tyr Ala Leu Leu Asn Leu Tyr Glu
    290                 295                 300

His His His Ser Ala His Leu Arg Gln Arg Ala Val Gln Lys Leu Tyr
305                 310                 315                 320

Glu His Ile Val Ala Asp Asp Arg Phe Thr Lys Ser Ile Ser Ile Gly
                325                 330                 335

Pro Ile Ser Lys Thr Ile Asn Met Leu Val Arg Trp Tyr Val Asp Gly
            340                 345                 350

Pro Ala Ser Thr Ala Phe Gln Glu His Val Ser Arg Ile Pro Asp Tyr
        355                 360                 365

Leu Trp Met Gly Leu Asp Gly Met Lys Met Gln Gly Thr Asn Gly Ser
    370                 375                 380

Gln Ile Trp Asp Thr Ala Phe Ala Ile Gln Ala Leu Leu Glu Ala Gly
385                 390                 395                 400

Gly His His Arg Pro Glu Phe Ser Ser Cys Leu Gln Lys Ala His Glu
                405                 410                 415

Phe Leu Arg Leu Ser Gln Val Pro Asp Asn Pro Pro Asp Tyr Gln Lys
            420                 425                 430

Tyr Tyr Arg Gln Met Arg Lys Gly Gly Phe Ser Phe Ser Thr Leu Asp
        435                 440                 445

Cys Gly Trp Ile Val Ser Asp Cys Thr Ala Glu Ala Leu Lys Ala Val
    450                 455                 460

Leu Leu Leu Gln Glu Lys Cys Pro His Val Thr Glu His Ile Pro Arg
465                 470                 475                 480

Glu Arg Leu Cys Asp Ala Val Ala Val Leu Leu Asn Met Arg Asn Pro
                485                 490                 495

Asp Gly Gly Phe Ala Thr Tyr Glu Thr Lys Arg Gly Gly His Leu Leu
            500                 505                 510

Glu Leu Leu Asn Pro Ser Glu Val Phe Gly Asp Ile Met Ile Asp Tyr
        515                 520                 525

Thr Tyr Val Glu Cys Thr Ser Ala Val Met Gln Ala Leu Lys Tyr Phe
    530                 535                 540

His Lys Arg Phe Pro Glu His Arg Ala Ala Glu Ile Arg Glu Thr Leu
545                 550                 555                 560

Thr Gln Gly Leu Glu Phe Cys Arg Arg Gln Gln Arg Ala Asp Gly Ser
                565                 570                 575

Trp Glu Gly Ser Trp Gly Val Cys Phe Thr Tyr Gly Thr Trp Phe Gly
            580                 585                 590

Leu Glu Ala Phe Ala Cys Met Gly Gln Thr Tyr Arg Asp Gly Thr Ala
        595                 600                 605

Cys Ala Glu Val Ser Arg Ala Cys Asp Phe Leu Leu Ser Arg Gln Met
    610                 615                 620

Ala Asp Gly Gly Trp Gly Glu Asp Phe Glu Ser Cys Glu Glu Arg Arg
625                 630                 635                 640

Tyr Val Gln Ser Ala Gln Ser Gln Ile His Asn Thr Cys Trp Ala Met
                645                 650                 655

Met Gly Leu Met Ala Val Arg His Pro Asp Ile Glu Ala Gln Glu Arg
            660                 665                 670

Gly Val Arg Cys Leu Leu Glu Lys Gln Leu Pro Asn Gly Asp Trp Pro
        675                 680                 685

Gln Glu Asn Ile Ala Gly Val Phe Asn Lys Ser Cys Ala Ile Ser Tyr
    690                 695                 700

Thr Ser Tyr Arg Asn Ile Phe Pro Ile Trp Ala Leu Gly Arg Phe Ser
705                 710                 715                 720

Gln Leu Tyr Pro Glu Arg Ala Leu Ala Gly His Pro
                725                 730

<210>  6

<211>  753

<212>  PRT

<213>  Homo sapiens

<400>  6

Met Thr Glu Gly Thr Cys Leu Arg Arg Arg Gly Gly Pro Tyr Lys Thr
1               5                   10                  15

Glu Pro Ala Thr Asp Leu Gly Arg Trp Arg Leu Asn Cys Glu Arg Gly
            20                  25                  30

Arg Gln Thr Trp Thr Tyr Leu Gln Asp Glu Arg Ala Gly Arg Glu Gln
        35                  40                  45

Thr Gly Leu Glu Ala Tyr Ala Leu Gly Leu Asp Thr Lys Asn Tyr Phe
    50                  55                  60

Lys Asp Leu Pro Lys Ala His Thr Ala Phe Glu Gly Ala Leu Asn Gly
65                  70                  75                  80

Met Thr Phe Tyr Val Gly Leu Gln Ala Glu Asp Gly His Trp Thr Gly
                85                  90                  95

Asp Tyr Gly Gly Pro Leu Phe Leu Leu Pro Gly Leu Leu Ile Thr Cys
            100                 105                 110

His Val Ala Arg Ile Pro Leu Pro Ala Gly Tyr Arg Glu Glu Ile Val
        115                 120                 125

Arg Tyr Leu Arg Ser Val Gln Leu Pro Asp Gly Gly Trp Gly Leu His
    130                 135                 140

Ile Glu Asp Lys Ser Thr Val Phe Gly Thr Ala Leu Asn Tyr Val Ser
145                 150                 155                 160

Leu Arg Ile Leu Gly Val Gly Pro Asp Asp Pro Asp Leu Val Arg Ala
                165                 170                 175

Arg Asn Ile Leu His Lys Lys Gly Gly Ala Val Ala Ile Pro Ser Trp
            180                 185                 190

Gly Lys Phe Trp Leu Ala Val Leu Asn Val Tyr Ser Trp Glu Gly Leu
        195                 200                 205

Asn Thr Leu Phe Pro Glu Met Trp Leu Phe Pro Asp Trp Ala Pro Ala
    210                 215                 220

His Pro Ser Thr Leu Trp Cys His Cys Arg Gln Val Tyr Leu Pro Met
225                 230                 235                 240

Ser Tyr Cys Tyr Ala Val Arg Leu Ser Ala Ala Glu Asp Pro Leu Val
                245                 250                 255

Gln Ser Leu Arg Gln Glu Leu Tyr Val Glu Asp Phe Ala Ser Ile Asp
            260                 265                 270

Trp Leu Ala Gln Arg Asn Asn Val Ala Pro Asp Glu Leu Tyr Thr Pro
        275                 280                 285

His Ser Trp Leu Leu Arg Val Val Tyr Ala Leu Leu Asn Leu Tyr Glu
    290                 295                 300

His His His Ser Ala His Leu Arg Gln Arg Ala Val Gln Lys Leu Tyr
305                 310                 315                 320

Glu His Ile Val Ala Asp Asp Arg Phe Thr Lys Ser Ile Ser Ile Gly
                325                 330                 335

Pro Ile Ser Lys Thr Ile Asn Met Leu Val Arg Trp Tyr Val Asp Gly
            340                 345                 350

Pro Ala Ser Thr Ala Phe Gln Glu His Val Ser Arg Ile Pro Asp Tyr
        355                 360                 365

Leu Trp Met Gly Leu Asp Gly Met Lys Met Gln Gly Thr Asn Gly Ser
    370                 375                 380

Gln Ile Trp Asp Thr Ala Phe Ala Ile Gln Ala Leu Leu Glu Ala Gly
385                 390                 395                 400

Gly His His Arg Pro Glu Phe Ser Ser Cys Leu Gln Lys Ala His Glu
                405                 410                 415

Phe Leu Arg Leu Ser Gln Val Pro Asp Asn Pro Pro Asp Tyr Gln Lys
            420                 425                 430

Tyr Tyr Arg Gln Met Arg Lys Gly Gly Phe Ser Phe Ser Thr Leu Asp
        435                 440                 445

Cys Gly Trp Ile Val Ser Asp Cys Thr Ala Glu Ala Leu Lys Ala Val
    450                 455                 460

Leu Leu Leu Gln Glu Lys Cys Pro His Val Thr Glu His Ile Pro Arg
465                 470                 475                 480

Glu Arg Leu Cys Asp Ala Val Ala Val Leu Leu Asn Met Arg Asn Pro
                485                 490                 495

Asp Gly Gly Phe Ala Thr Tyr Glu Thr Lys Arg Gly Gly His Leu Leu
            500                 505                 510

Glu Leu Leu Asn Pro Ser Glu Val Phe Gly Asp Ile Met Ile Asp Tyr
        515                 520                 525

Thr Tyr Val Glu Cys Thr Ser Ala Val Met Gln Ala Leu Lys Tyr Phe
    530                 535                 540

His Lys Arg Phe Pro Glu His Arg Ala Ala Glu Ile Arg Glu Thr Leu
545                 550                 555                 560

Thr Gln Gly Leu Glu Phe Cys Arg Arg Gln Gln Arg Ala Asp Gly Ser
                565                 570                 575

Trp Glu Gly Ser Trp Gly Val Cys Phe Thr Tyr Gly Thr Trp Phe Gly
            580                 585                 590

Leu Glu Ala Phe Ala Cys Met Gly Gln Thr Tyr Arg Asp Gly Thr Ala
        595                 600                 605

Cys Ala Glu Val Ser Arg Ala Cys Asp Phe Leu Leu Ser Arg Gln Met
    610                 615                 620

Ala Asp Gly Gly Trp Gly Glu Asp Phe Glu Ser Cys Glu Glu Arg Arg
625                 630                 635                 640

Tyr Val Gln Ser Ala Gln Ser Gln Ile His Asn Thr Cys Trp Ala Met
                645                 650                 655

Met Gly Leu Met Ala Val Arg His Pro Asp Ile Glu Ala Gln Glu Arg
            660                 665                 670

Gly Val Arg Cys Leu Leu Glu Lys Gln Leu Pro Asn Gly Asp Trp Pro
        675                 680                 685

Gln Glu Asn Ile Ala Gly Val Phe Asn Lys Ser Cys Ala Ile Ser Tyr
    690                 695                 700

Thr Ser Tyr Arg Asn Ile Phe Pro Ile Trp Ala Leu Gly Arg Phe Ser

705                 710                 715                 720

Gln Leu Tyr Pro Glu Arg Ala Leu Ala Gly His Leu Glu Gln Lys Leu
                725                 730                 735

Ile Ser Glu Glu Asp Leu Asn Ser Ala Val Asp His His His His His
            740                 745                 750

His

<210>  7

<211>  10

<212>  PRT

<213>  Homo sapiens

<400>  7

Cys Pro His Val Thr Glu His Ile Pro Arg
1               5                   10